# EUROPEAN PATENT APPLICATION

(11) **EP 4 488 363 A2**
(43) Date of publication of application: **08.01.2025**
(21) Application number: 24203168.0
(22) Date of filing: 20.07.2018
(51) Int. Cl.: C12N 5/071

(54) **RE-AGGREGATION OF STEM CELL-DERIVED PANCREATIC BETA CELLS**

(30) Priority: 21.07.2017 US 201762535659 P
(62) Divisional of application: 18836082.0
(71) Applicant: Vertex Pharmaceuticals Incorporated, Boston, MA 02210 (US)
(72) Inventor: POH, Yeh-Chuin, Belmont, 02478 (US); HARB, George, Boston, 02125 (US); PAGLIUCA, Felicia J., Boston, 02116 (US)
(74) Representative: J A Kemp LLP

(57) **Abstract**

The present application discloses cell clusters resembling the function and characteristics of endogenous pancreatic islets, and methods for making and using such cell clusters. The present disclosure provides an in vitro cell cluster comprising at least one non-native pancreatic β cell that exhibits an in vitro glucose-stimulated insulin secretion response when exposed to a glucose challenge, wherein the cell cluster is an unsorted cell cluster, and wherein the cell cluster comprises: at least 35% of cells that express NKX6.1 and C-peptide; at least 70% of cells that express chromogranin A; at most about 2% of cells that express SOX2; or at most about 10% of cells that express SOX9. In some cases, when transplanted into a subject, the in vitro cell cluster exhibits an in vivo glucose-stimulated insulin secretion response to a glucose challenge in the subject.

## Description

### CROSS-REFERENCE

This application claims the benefit of U.S. Provisional Application No. 62/535,659, filed on July 21, 2017, which application is incorporated herein by reference in its entirety.

### BACKGROUND

Transplantation of pancreas or pancreatic islets has been used for treating diabetes, such as type I diabetes. Pancreatic islet transplantation does not need major surgery and the function of the islet grafts can be maintained for years in a recipient. However, a shortage of pancreatic islets donors prevents this therapy from being effectively implemented. Artificial pancreas or pancreatic islets provide an alternative source of transplantable islets. Thus, there is a need for methods of *in vitro* restitution of pancreatic islets whose function and characteristics resemble endogenous pancreatic islets.

### SUMMARY

In one aspect, the present disclosure provides an *in vitro* cell cluster comprising at least one non-native pancreatic β cell that exhibits an *in vitro* glucose-stimulated insulin secretion response when exposed to a glucose challenge, wherein the cell cluster is an unsorted cell cluster, and wherein the cell cluster comprises: (a) at least about 35% cells that express NKX6.1 and C-peptide; (b) at least about 70% cells that express chromogranin A; (c) at most about 2% cells that express SOX2; or (d) at most about 10% cells that express SOX9.

In another aspect, the present disclosure provides an *in vitro* cell cluster comprising at least one NKX6.1+ and C-peptide+ cell that comprises no fluorescent protein, wherein the cell cluster exhibits an *in vitro* glucose-stimulated insulin secretion response when exposed to a glucose challenge, and wherein the cell cluster comprises: (a) at least about 35% cells that express NKX6.1 and C-peptide; (b) at least about 70% cells that express chromogranin A; (c) at most about 2% cells that express SOX2; or (d) at most about 10% cells that express SOX9.

In some cases, the at least one NKX6.1+ and C-peptide+ cell is a non-native pancreatic β cell exhibiting an *in vitro* glucose-stimulated insulin secretion response when exposed to a glucose challenge. In some cases, the cell cluster comprises at least about 35% cells that express NKX6.1 and C-peptide. In some cases, the cell cluster comprises at least about 40% or at least about 60% cells that express both NKX6.1 and C-peptide. In some cases, the cell cluster comprises at least about 70% cells that express chromogranin A. In some cases, the cell cluster comprises at least about 80%, at least about 90%, at least about 95%, or 100% cells that express chromogranin A. In some cases, the cell cluster comprises at most about 2% cells in the cell cluster that express SOX2. In some cases, the cell cluster comprises at most about 1.5%, at most about 1%, or at most about 0.5% cells that express SOX2. In some cases, the cell cluster comprises at most about 10% cells that express SOX9. In some cases, the cell cluster comprises at most about 5%, at most about 3%, or at most about 2 % cells that express SOX9. In some cases, the cell cluster comprises at most about 2%, at most about 1%, at most about 0.5%, or at most about 0.1% cells that express Ki67.

In some cases, the *in vitro* cell cluster has a diameter that is about 150 µm. In some cases, the *in vitro* cell cluster has a diameter that is from about 50 µm to about 250 µm. In some cases, the *in vitro* cell cluster has a diameter that is from about 100 µm to about 200 µm. In some cases, the *in vitro* cell cluster exhibits a higher insulin secretion in response to a first glucose concentration as compared to a second glucose concentration, wherein the first glucose concentration is higher than the second glucose concentration. In some cases, the *in vitro* cell cluster further comprises at least one cell expressing glucagon or at least one cell expressing somatostatin. In some cases, the *in vitro* cell cluster exhibits insulin secretion in response to a first concentration of K+. In some cases, when transplanted into a subject, the *in vitro* cell cluster exhibits an *in vivo* glucose-stimulated insulin secretion response to a glucose challenge in the subject within 28 days after transplantation. In some cases, when transplanted into a subject, the second *in vitro* cell cluster exhibits an *in vivo* glucose-stimulated insulin secretion response to a glucose challenge in the subject within 14 days after transplantation.

In some cases, (a) the at least one non-native pancreatic β cell comprises one or more crystalline insulin granules; (b) the at least one non-native pancreatic β cell expresses the following genes: INS, PDX1, NKX6-1, and ZNT8; or (c) the at least one non-native pancreatic β cell does not express at least one of somatostatin and glucagon. In some cases, the at least one non-native pancreatic β cell exhibits an *in vitro* glucose-stimulated insulin secretion response to a first glucose challenge, a second glucose challenge, and a third glucose challenge, when the first glucose challenge, the second glucose challenge, and the third glucose challenge are applied sequentially. In some cases, secretion of insulin by the at least one non-native pancreatic β cell in response to a glucose challenge is proportional to glucose concentration in the glucose challenge. In some cases, the at least one non-native pancreatic β cell is genetically modified.

In some cases, the at least one non-native pancreatic β cell does not comprise an exogenous nucleic acid sequence coding for a signal polypeptide indicative of insulin expression in the at least one non-native pancreatic β cell. In some cases, expression of the signal polypeptide in the non-native pancreatic β cell is driven by an insulin promoter. In some cases, the signal polypeptide comprises a fluorescent protein.

In yet another aspect, the present disclosure provides a composition comprising at least one cell cluster as provided herein in a culture medium.

In some cases, the culture medium is serum-free. In some cases, the reaggregation culture medium does not comprise triiodothyronine. In some cases, the reaggregation culture medium does not comprise an exogenous thyroid hormone signaling pathway activator. In some cases, the reaggregation culture medium does not comprise an activing receptor-like kinse-5 (Alk5) inhibitor. In some cases, the culture medium does not comprise an exogenous inhibitor of Rho-associated, coiled-coil containing protein kinase (ROCK). In some cases, the culture medium does not comprise an exogenous extracellular matrix molecule. In some cases, the cell cluster is in suspension in the culture medium. In some cases, the culture medium is stirred at a predetermined speed that is at least 30 rpm. In some cases, the culture medium is stirred at a predetermined speed that is at least 60 rpm. In some cases, the culture medium comprises MCDB131 basal medium. In some cases, the MCDB131 basal medium is supplemented with 2% bovine serum albumin (BSA). In some cases, the culture medium comprises CMRLs medium. In some cases, the composition further comprises a bioreactor containing the at least one cell cluster and the culture medium. In some cases, the bioreactor comprises a spinner flask.

In yet another aspect, the present disclosure provides a method, comprising: (a) differentiating a population of cells comprising at least one NKX6.1+ cell into a first cell cluster comprising at least one NKX6.1+ and C-peptide+ cell in a culture medium comprising a factor selected from the group consisting of: a transforming growth factor β (TGF-β) signaling pathway inhibitor, a thyroid hormone (TH) signaling pathway activator, at least one sonic-hedgehog (SHH) pathway inhibitor, a retinoic acid (RA) signaling pathway activator, a γ-secretase inhibitor, a bone morphogenic protein (BMP) signaling pathway inhibitor, at least one growth factor from epidermal growth factor (EGF) family, and any combination thereof; (b) dissociating a plurality of cells from the first cell cluster; and (c) culturing the plurality of cells from (b) in a reaggregation culture medium and allowing at least a portion of the plurality of cells to form a second *in vitro* cell cluster, wherein: (i) at least about 35% cells that express NKX6.1 and C-peptide; (ii) at least about 70% cells that express chromogranin A; (iii) at most about 2% cells that express SOX2; or (iv) at most about 10% cells that express SOX9. In some cases, the at least one growth factor from epidermal growth factor (EGF) family comprises betacellulin.

In yet another aspect, the present disclosure provides a method, comprising: (a) obtaining a first cell cluster comprising at least one NKX6. 1+ and C-peptide+ cell; (b) dissociating a plurality of cells from the first cell cluster; and (c) culturing the plurality of cells from (b) in a reaggregation culture medium and allowing at least a portion of the plurality of cells to form a second *in vitro* cell cluster, wherein: (i) at least about 35% cells that express NKX6.1 and C-peptide; (ii) at least about 70% cells that express chromogranin A; (iii) at most about 2% cells that express SOX2; or (iv) at most about 10% cells that express SOX9.

In yet another aspect, the present disclosure provides a method, comprising: (a) obtaining a first cell cluster comprising at least one NKX6. 1+ and C-peptide+ cell; (b) dissociating a plurality of cells from the first cell cluster, wherein the dissociating does not comprise subjecting the plurality of cells to flow cytometry; and (c) culturing the plurality of cells from (b) in a serum-free reaggregation culture medium and allowing at least a portion of the plurality of cells to form a second *in vitro* cell cluster, wherein: (i) the second *in vitro* cell cluster comprises a higher percentage cells that express chromogranin A as compared the first cell cluster; (ii) the second *in vitro* cell cluster comprises a higher percentage cells that express NKX6.1 and C-peptide as compared the first cell cluster; (iii) the second *in vitro* cell cluster comprises a lower percentage cells that express SOX2 as compared the first cell cluster; or (iv) the second *in vitro* cell cluster comprises a lower percentage of cells that express SOX9 as compared the first cell cluster.

In yet another aspect, the present disclosure provides a method, comprising: (a) obtaining a first cell cluster comprising at least one NKX6. 1+ and C-peptide+ cell; (b) dissociating a plurality of cells from the first cell cluster, wherein the dissociating does not comprise subjecting the plurality of cells to flow cytometry; and (c) culturing the plurality of cells from (b) in a reaggregation culture medium and allowing at least a portion of the plurality of cells to form a second *in vitro* cell cluster, wherein the second *in vitro* cell cluster comprises at least one non-native pancreatic β cell that exhibits an *in vitro* glucose-stimulated insulin secretion response when exposed to a glucose challenge; and wherein: (i) at least about 35% cells that express NKX6.1 and C-peptide; (ii) at least about 70% cells that express chromogranin A; (iii) at most about 2% cells that express SOX2; or (iv) at most about 10% cells that express SOX9.

In some cases, the second *in vitro* cell cluster comprises at least one non-native pancreatic β cell that exhibits an *in vitro* glucose-stimulated insulin secretion response when exposed to a glucose challenge. In some cases, the dissociating comprises enzymatic dissociation of the first cell cluster. In some cases, the dissociating comprises contacting the first cell cluster with trypsin. In some cases, the reaggregation culture medium is serum-free. In some cases, the reaggregation culture medium is stirred at a predetermined speed. In some cases, the predetermined speed is that is at least 30 rpm or at least 60 rpm. In some cases, the reaggregation culture medium comprises MCDB131 basal medium. In some cases, the MCDB131 basal medium is supplemented with 2% bovine serum albumin (BSA). In some cases, the reaggregation culture medium comprises CMRLs medium. In some cases, the reaggregation culture medium comprises MCDB131 basal medium.

In some cases, (a) the at least one non-native pancreatic β cell comprises one or more crystalline insulin granules; (b) the at least one non-native pancreatic β cell expresses the following genes: INS, PDX1, NKX6-1, and ZNT8; or (c) the at least one non-native pancreatic β cell does not express at least one of somatostatin and glucagon. In some cases, the at least one non-native pancreatic β cell exhibits an *in vitro* glucose-stimulated insulin secretion response to a first glucose challenge, a second glucose challenge, and a third glucose challenge, when the first glucose challenge, the second glucose challenge, and the third glucose challenge are applied sequentially. In some cases, the at least one non-native pancreatic β cell is genetically modified. In some cases, secretion of insulin by the at least one non-native pancreatic β cell in response to a glucose challenge is proportional to glucose concentration in the glucose challenge.

In some cases, the reaggregation culture medium does not comprise triiodothyronine. In some cases, the reaggregation culture medium does not comprise an exogenous thyroid hormone signaling pathway activator. In some cases, the reaggregation culture medium does not comprise an activing receptor-like kinse-5 (Alk5) inhibitor. In some cases, the reaggregation culture medium does not comprise an exogenous transforming growth factor β signaling pathway inhibitor. In some cases, the reaggregation culture medium does not comprise an exogenous inhibitor of Rho-associated, coiled-coil containing protein kinase (ROCK). In some cases, the reaggregation culture medium does not comprise an exogenous extracellular matrix molecule.

In some cases, the first cell cluster is recovered from cryopreservation. In some cases, the method further comprises recovering the first cell cluster from cryopreservation. In some cases, the method further comprises cryopreserving the plurality of cells for a period of time before the culturing. In some cases, the method further comprises recovering the plurality of cells from the cryopreservation before the culturing.

In some cases, the second *in vitro* cell cluster has a diameter that is at most 50% of the first cell cluster. In some cases, the second *in vitro* cell cluster has a diameter that is at most 30% of the first cell cluster. In some cases, the second *in vitro* cell cluster has a diameter that is about 150 µm. In some cases, the second *in vitro* cell cluster has a diameter that is from about 50 µm to about 250 µm. In some cases, the second *in vitro* cell cluster has a diameter that is from about 100 µm to about 200 µm. In some cases, a percentage of cells in the second *in vitro* cell cluster that express chromogranin A is at least 1.2, at least 1.3, at least 1.4, or at least 1.5 times more than a percentage of cells in the first cell cluster that express chromogranin A. In some cases, the second *in vitro* cell cluster comprises at least about 70%, at least about 80%, at least about 90%, at least about 95%, or 100% cells that express chromogranin A. In some cases, a percentage of cells in the second *in vitro* cell cluster that express both NKX6.1 and C-peptide is at least 1.5, at least 1.75, or at least 2 times more than a percentage of cells in the first cell cluster that express both NKX6.1 and C-peptide. In some cases, the second *in vitro* cell cluster comprises at least about 35%, at least about 40%, or at least about 60% cells that express both NKX6.1 and C-peptide. In some cases, a percentage of cells in the second *in vitro* cell cluster that express SOX2 is at least 2, at least 3, at least 5, or at least 10 times lower than a percentage of cells in the first cell cluster that express SOX2. In some cases, the second *in vitro* cell cluster comprises at most about 2%, at most about 1.5%, at most about 1%, or at most about 0.5% cells that express SOX2. In some cases, a percentage of cells in the second *in vitro* cell cluster that express SOX9 is at least 2, at least 3, at least 5, or at least 10 times lower than a percentage of cells in the first cell cluster that express SOX9. In some cases, the second *in vitro* cell cluster comprises at most about 10%, at most about 5%, at most about 3%, or at most about 2 % cells that express SOX9. In some cases, the second *in vitro* cell cluster has a lower percentage cells expressing Ki67 as compared to the first cell cluster. In some cases, the second *in vitro* cell cluster comprises at most about 2%, at most about 1%, at most about 0.5%, or at most about 0.1% cells that express Ki67. In some cases, the second *in vitro* cell cluster has a higher insulin content as compared to the first cell cluster. In some cases, the second *in vitro* cell cluster has at least 1.1, at least 1.25 or at least 1.5 times higher insulin content as compared to the first cell cluster. In some cases, the second *in vitro* cell cluster exhibits a higher stimulation index compared to the first cell cluster, wherein the stimulation index equals to a ratio of insulin secreted in response to a first glucose concentration as compared to a second glucose concentration, wherein the first glucose concentration is higher than the second glucose concentration.

In some cases, the second *in vitro* cell cluster exhibits insulin secretion in response to a first concentration of K+. In some cases, the second *in vitro* cell cluster exhibits biphasic insulin secretion in response to a high glucose concentration that is larger than 10 mM. In some cases, the second *in vitro* cell cluster further comprises at least one cell expressing glucagon or at least one cell expressing somatostatin. In some cases, at least about 95%, at least about 98%, or at least about 99% of cells that express both NKX6.1 and C-peptide in the first cell cluster are retained in the second *in vitro* cell cluster. In some cases, the second *in vitro* cell cluster has a higher oxygen consumption rate as compared to the first cell cluster. In some cases, the second *in vitro* cell cluster has at least 1.1, at least 1.25 or at least 1.5 times higher oxygen consumption rate as compared to the first cell cluster. In some cases, when transplanted into a subject, the second *in vitro* cell cluster exhibits an *in vivo* glucose-stimulated insulin secretion response to a glucose challenge in the subject within 28 days after transplantation. In some cases, when transplanted into a subject, the second *in vitro* cell cluster exhibits an *in vivo* glucose-stimulated insulin secretion response to a glucose challenge in the subject within 14 days after transplantation.

In yet another aspect, the present disclosure provides an *in vitro* cell cluster that is generated by the method provided herein.

In yet another aspect, the present disclosure provides a device comprising the *in vitro* cell cluster as disclosed herein, wherein the device is configured to produce and release insulin when implanted into a subject. In some cases, the device further comprises a semipermeable membrane, wherein the semipermeable membrane is configured to retain the cell cluster in the device and permit passage of insulin secreted by the cell cluster. In some cases, the cell cluster is encapsulated by the semipermeable membrane. In some cases, the semipermeable membrane is made of polysaccharide or polycation. In some cases, the semipermeable membrane is made of a material selected from the group consisting of: poly(lactide) (PLA), poly(glycolic acid) (PGA), poly(lactide-co-glycolide) (PLGA), and other polyhydroxyacids, poly(caprolactone), polycarbonates, polyamides, polyanhydrides, polyphosphazene, polyamino acids, polyortho esters, polyacetals, polycyanoacrylates, polytetrafluoroethylene (PTFE), biodegradable polyurethanes, albumin, collagen, fibrin, polyamino acids, prolamines, alginate, agarose, agarose with gelatin, dextran, polyacrylates, ethylene- vinyl acetate polymers and other acyl-substituted cellulose acetates and derivatives thereof, polyurethanes, polystyrenes, polyvinyl chloride, polyvinyl fluoride, poly(vinyl imidazole), chlorosulphonated polyolefins, polyethylene oxide, and any combinations thereof. In some cases, the semipermeable membrane comprises alginate. In some cases, the cell cluster is encapsulated in a microcapsule that comprises an alginate core surrounded by the semipermeable membrane.

In yet another aspect, the present disclosure provides a method of treating or preventing a disease in a subject in need thereof, the method comprising administering the cell cluster of any one of claims as provided herein or a portion thereof to the subject.

In yet another aspect, the present disclosure provides a method of treating or preventing a disease in a subject in need thereof, the method comprising implanting the device as provided herein to the subject. In some cases, the subject has, or has an increased risk of developing a metabolic disorder. In some cases, the subject has diabetes selected from the group consisting of: Type I diabetes, Type II diabetes, and Type 1.5 diabetes.

In yet another aspect, the present disclosure provides composition, methods, and kits. The compositions can include a cell cluster comprising at least one non-native pancreatic β cell. The methods can include obtaining at least one non-native pancreatic β cell. The kits can include a cell cluster comprising at least one non-native pancreatic β cell. In some aspects, this disclosure provides a cell cluster comprising at least one non-native pancreatic β cell. In some embodiments, at least about 70% of cells in the cell cluster express chromogranin A as measured by flow cytometry. In some embodiments, the cell cluster exhibits an *in vitro* glucose-stimulated insulin secretion response when exposed to a glucose challenge. In some aspects, this disclosure provides a cell cluster comprising at least one non-native pancreatic β cell. In some embodiments, at least about 25% of cells in the cell cluster express NKX6.1 and C-peptide as measured by flow cytometry. In some embodiments, at least about 30% of cells in the cell cluster express NKX6.1 and C-peptide as measured by flow cytometry. In some embodiments, at least about 35% of cells in the cell cluster express NKX6.1 and C-peptide as measured by flow cytometry. In some embodiments, at least about 40% of cells in the cell cluster express NKX6.1 and C-peptide as measured by flow cytometry. In some embodiments, at least about 45% of cells in the cell cluster express NKX6.1 and C-peptide as measured by flow cytometry. In some embodiments, at least about 50% of cells in the cell cluster express NKX6.1 and C-peptide as measured by flow cytometry. In some embodiments, the cell cluster exhibits an *in vitro* glucose-stimulated insulin secretion response when exposed to a glucose challenge. In some aspects, this disclosure provides a cell cluster comprising at least one non-native pancreatic β cell. In some embodiments, at least about 70% of cells in the cell cluster express chromogranin A as measured by flow cytometry. In some embodiments, the cell cluster exhibits an *in vivo* glucose-stimulated insulin secretion response to a glucose challenge in a subject when transplanted into the subject. In some embodiments, the cell cluster exhibits an *in vivo* glucose-stimulated insulin secretion response to a glucose challenge in a subject within 28 days after the transplantation. In some aspects, this disclosure provides a cell cluster comprising at least one non-native pancreatic β cell, wherein at least about 25% of cells in the cell cluster express NKX6.1 and C-peptide as measured by flow cytometry. In some embodiments, at least about 30% of cells in the cell cluster express NKX6.1 and C-peptide as measured by flow cytometry. In some embodiments, at least about 35% of cells in the cell cluster express NKX6.1 and C-peptide as measured by flow cytometry. In some embodiments, at least about 40% of cells in the cell cluster express NKX6.1 and C-peptide as measured by flow cytometry. In some embodiments, at least about 45% of cells in the cell cluster express NKX6.1 and C-peptide as measured by flow cytometry. In some embodiments, at least about 50% of cells in the cell cluster express NKX6.1 and C-peptide as measured by flow cytometry. In some embodiments, the cell cluster exhibits an *in vivo* glucose-stimulated insulin secretion response to a glucose challenge in a subject when transplanted in the subject. In some embodiments, the cell cluster exhibits an *in vivo* glucose-stimulated insulin secretion response to a glucose challenge in a subject within about 28 days after the transplantation. In some embodiments, at least about 90% of cells in the cell cluster express chromogranin A as measured by flow cytometry. In some embodiments, at least about 70% of cells in the cell cluster express NKX6.1 and C-peptide as measured by flow cytometry. In some embodiments, at least about 25% of cells in the cell cluster express NKX6.1 and C-peptide as measured by flow cytometry. In some embodiments, at least about 30% of cells in the cell cluster express NKX6.1 and C-peptide as measured by flow cytometry. In some embodiments, at least about 35% of cells in the cell cluster express NKX6.1 and C-peptide as measured by flow cytometry. In some embodiments, at least about 40% of cells in the cell cluster express NKX6.1 and C-peptide as measured by flow cytometry. In some embodiments, at least about 45% of cells in the cell cluster express NKX6.1 and C-peptide as measured by flow cytometry. In some embodiments, at least about 50% of cells in the cell cluster express NKX6.1 and C-peptide as measured by flow cytometry. In some embodiments, the cell cluster exhibits an *in vitro* glucose-stimulated insulin secretion response to a first glucose challenge and a second glucose challenge when the first and second glucose challenges are applied sequentially. In some embodiments, when transplanted into the subject, the cell cluster exhibits an *in vivo* glucose-stimulated insulin secretion response to a glucose challenge in the subject within 14 days after the transplantation. In some embodiments, the cell cluster has a diameter less than about 200 µm. In some embodiments, the cell cluster has a diameter less than about 300 µm. In some embodiments, the cell cluster has a diameter less than about 400 µm. In some embodiments, the diameter is less than about 100 µm. In some embodiments, less than about 10% of cells in the cell cluster are not viable. In some embodiments, the cell cluster exhibits a glucose-stimulated calcium flux response when exposed to a glucose challenge. In some aspects, this disclosure provides a cell cluster comprising at least one non-native pancreatic β cell, wherein at least about 70% of cells in the cell cluster express chromogranin A, and at least about 25% of cells in the cell cluster express NKX6.1 and C-peptide, as measured by flow cytometry. In some aspects, this disclosure provides a cell cluster comprising at least one non-native pancreatic β cell, wherein at least about 70% of cells in the cell cluster express chromogranin A, and at least about 30% of cells in the cell cluster express NKX6.1 and C-peptide, as measured by flow cytometry. In some aspects, this disclosure provides a cell cluster comprising at least one non-native pancreatic β cell, wherein at least about 70% of cells in the cell cluster express chromogranin A, and at least about 35% of cells in the cell cluster express NKX6.1 and C-peptide, as measured by flow cytometry. In some aspects, this disclosure provides a cell cluster comprising at least one non-native pancreatic β cell, wherein at least about 70% of cells in the cell cluster express chromogranin A, and at least about 40% of cells in the cell cluster express NKX6.1 and C-peptide, as measured by flow cytometry. In some aspects, this disclosure provides a cell cluster comprising at least one non-native pancreatic β cell, wherein at least about 70% of cells in the cell cluster express chromogranin A, and at least about 45% of cells in the cell cluster express NKX6.1 and C-peptide, as measured by flow cytometry. In some aspects, this disclosure provides a cell cluster comprising at least one non-native pancreatic β cell, wherein at least about 70% of cells in the cell cluster express chromogranin A, and at least about 50% of cells in the cell cluster express NKX6.1 and C-peptide, as measured by flow cytometry. In some embodiments, the cell cluster exhibits an *in vitro* glucose-stimulated insulin secretion response when exposed to a glucose challenge. In some embodiments, when transplanted into a subject, the cell cluster exhibits an *in vivo* glucose-stimulated insulin secretion response to a glucose challenge in the subject within about 28 days after the transplantation. In some embodiments, this disclosure provides compositions comprising a scaffold. In some embodiments, the scaffold is a biodegradable scaffold.

In some aspects, this disclosure provides a method comprising obtaining a first cell cluster comprising at least one non-native pancreatic β cell, dissociating a plurality of cells from the first cell cluster, culturing the plurality of cells in a medium comprising a thyroid hormone signaling pathway activator and a transforming growth factor β signaling pathway inhibitor, thereby forming a second cell cluster *in vitro* using at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry. In some aspects, this disclosure provides a method comprising obtaining a first cell cluster comprising at least one non-native pancreatic β cell, dissociating a plurality of cells from the first cell cluster, culturing the plurality of cells from (a) in a medium comprising serum, and one of a thyroid hormone signaling pathway activator and a transforming growth factor β signaling pathway inhibitor, thereby forming a second cell cluster *in vitro* using at least a portion of the plurality of cells, wherein the second cell cluster comprises a non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry. In some embodiments, the medium further comprises a thyroid hormone signaling pathway activator. In some embodiments, the medium further comprises a transforming growth factor β signaling pathway inhibitor. In some embodiments, the thyroid hormone signaling pathway activator is triiodothyronine. In some embodiments, a concentration of the triiodothyronine in the medium is from about 0.1 µM to about 10 µM. In some embodiments, the transforming growth factor β signaling pathway inhibitor is an activin receptor-like kinase-5 inhibitor. In some embodiments, a concentration of the activin receptor-like kinase-5 inhibitor in the medium is from about 1 µM to about 50 µM. In some embodiments, the medium further comprises a serum selected from the group consisting of human serum, human platelet lysate, fetal bovine serum, and serum replacement. In some embodiments, the medium comprises from about 5% to about 15% serum. In some embodiments, the medium comprises about 10% serum. In some embodiments, the medium further comprises Connought Medical Research Laboratories 1066 supplemented islet media (CMRLS) or MCDB131 basal medium. In some embodiments, the medium further comprises an extracellular matrix molecule. In some embodiments, the extracellular matrix molecule is heparin. In some embodiments, the extracellular matrix molecule is laminin. In some embodiments, the second cell cluster comprises a higher percentage of cells expressing chromogranin A as compared to the first cell cluster as measured by flow cytometry. In some embodiments, the second cell cluster comprises a higher percentage of cells expressing NKX6.1 and C-peptide compared to the first cell cluster as measured by flow cytometry. In some embodiments, the second cell cluster comprises less inviable cells compared to the first cell cluster. In some embodiments, a portion of the plurality of cells fail to form the second cell cluster, and wherein less than about 10% of the portion of the plurality of cells that fail to form the second cell cluster express NKX6.1 and C-peptide as measured by flow cytometry. In some embodiments, a diameter of the second cell cluster is less than a diameter of the first cell cluster. In some embodiments, when exposed to a glucose challenge *in vitro,* the second cell cluster exhibits a higher stimulation index compared to the first cell cluster, wherein the stimulation index equals a ratio of insulin secreted in response to a first glucose concentration as compared to a second glucose concentration, wherein the first glucose concentration is higher than the second glucose concentration. In some embodiments, when exposed to a glucose challenge *in vivo,* the second cell cluster exhibits a higher stimulation index compared to the first cell cluster, wherein the stimulation index equals a ratio of insulin secreted in response to a first glucose concentration compared to a second glucose concentration, wherein the first glucose concentration is higher than the second glucose concentration. In some embodiments, the plurality of cells are cultured in a spinner flask. In some embodiments, the plurality of cells are generated from one or more stem cells *in vitro.* In some embodiments, the plurality of cells are generated from one or more induced pluripotent stem cells. In some embodiments, the plurality of cells are cultured in the medium for at least 1 day. In some embodiments, the plurality of cells are cultured in the medium for at least 4 days.

In some aspects, this disclosure provides a method for treating or preventing a disease in a subject in need thereof, the method comprising administering to the subject any of the compositions of the present disclosure. In some embodiments, the subject is a human. In some embodiments, the subject has, or has an increased risk of developing, diabetes. In some embodiments, the diabetes is Type I diabetes, Type II diabetes, Type 1.5 diabetes, or pre-diabetes. In some embodiments, the administering comprises administering the composition under a kidney capsule, in a liver, or in a pancreas. In some embodiments, the method further comprises cryopreserving the second cell cluster, thereby producing a cryopreserved second cell cluster. In some embodiments, the method further comprises thawing the cryopreserved second cell cluster, thereby producing a thawed second cell cluster comprising at least one non-native pancreatic β cell. In some embodiments, at least about 70% of cells in the thawed second cell cluster express chromogranin A as measured by flow cytometry. In some embodiments, at least about 25% of cells in the thawed second cell cluster express NKX6.1 and C-peptide as measured by flow cytometry. In some embodiments, at least about 30% of cells in the thawed second cell cluster express NKX6.1 and C-peptide as measured by flow cytometry. In some embodiments, at least about 35% of cells in the thawed second cell cluster express NKX6.1 and C-peptide as measured by flow cytometry. In some embodiments, at least about 40% of cells in the thawed second cell cluster express NKX6.1 and C-peptide as measured by flow cytometry. In some embodiments, at least about 45% of cells in the thawed second cell cluster express NKX6.1 and C-peptide as measured by flow cytometry. In some embodiments, at least about 50% of cells in the thawed second cell cluster express NKX6.1 and C-peptide as measured by flow cytometry.

In some aspects, the present disclosure provides a system for use in any of the methods of the present disclosure. In some embodiments, the system can comprise a magnetic stir plate, a spinner flask comprising a plurality of cells from the first cell cluster, wherein the magnetic stir plate and the spinner flask are configured to stir the plurality of cells from the first cluster thereby forming the second cell cluster *in vitro* using at least a portion of the plurality of cells.

In some aspects, the present disclosure provides a method for treating or preventing a disease in a subject in need thereof, the method comprising obtaining a first cell cluster comprising at least one non-native pancreatic β cell, dissociating a plurality of cells from the first cell cluster, culturing the plurality of cells from (a) in a medium comprising a thyroid hormone signaling pathway activator and a transforming growth factor β signaling pathway inhibitor, thereby forming a second cell cluster *in vitro* using at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 25% of cells in the second cell cluster express NKX6.1 and C-peptide as measured by flow cytometry, and administering a composition comprising the second cell cluster or a portion thereof to the subject.

In some aspects, the present disclosure provides a method for treating or preventing a disease in a subject in need thereof, the method comprising obtaining a first cell cluster comprising at least one non-native pancreatic β cell, dissociating a plurality of cells from the first cell cluster, culturing the plurality of cells in a medium comprising serum, and a thyroid hormone signaling pathway activator or a transforming growth factor β signaling pathway inhibitor, thereby forming a second cell cluster *in vitro* from at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 25% of cells in the second cell cluster express NKX6.1 and C-peptide as measured by flow cytometry, and administering a composition comprising the second cell cluster or a portion thereof to the subject.

In some aspects, the present disclosure provides a pharmaceutical composition comprising a cell cluster of the present disclosure and at least one pharmaceutically acceptable excipient. In some embodiments, the pharmaceutical composition is a liquid composition. In some embodiments, the pharmaceutical composition is a capsule. In some embodiments, the capsule is a gel capsule. In some embodiments, the capsule is a liposome.

In some aspects, the present disclosure provides a cell cluster comprising at least one non-native pancreatic β cell, and the cell cluster is prepared by a process comprising obtaining a first cell cluster comprising at least one non-native pancreatic β cell, dissociating a plurality of cells from the first cell cluster, culturing the plurality of cells in a medium comprising a thyroid hormone signaling pathway activator and a transforming growth factor β signaling pathway inhibitor, thereby forming a second cell cluster *in vitro* from at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry.

In some aspects, the present disclosure provides a kit comprising (i) a cell cluster comprising at least one non-native pancreatic β cell, wherein at least about 70% of cells in the cell cluster express chromogranin A as measured by flow cytometry, and wherein the cell cluster exhibits an *in vitro* glucose-stimulated insulin secretion response when exposed to a glucose challenge, and (ii) a buffer. In some aspects, the present disclosure provides a kit comprising (i) a cell cluster comprising at least one non-native pancreatic β cell, wherein at least about 25% of cells in the cell cluster express NKX6.1 and C-peptide as measured by flow cytometry, and wherein the cell cluster exhibits an *in vitro* glucose-stimulated insulin secretion response when exposed to a glucose challenge, and (ii) a buffer.

Provided herein in certain embodiments are improvements to methods, compositions, and systems to U.S. Application No. 14/684,101 filed April 10, 2015 and U.S. Application No. 14/684,129 filed April 10, 2015, which claim priority to U.S. Provisional Application No. 61/833,898 filed June 11, 2013 and U.S. Provisional Application No. 61/972,212 filed March 28, 2014, each of which is entirely incorporated herein by reference.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings (also "Figure" and "FIG." herein), of which:
**FIG.** 1 shows the effects of using various Stage 6 cell culture media on *in vitro* glucose-stimulated insulin secretion responses of re-aggregated cell clusters.
**FIG.** 2 shows fluorescence images (top) and quantification (bottom) of cells expressing LIN28.
**FIG.** 3 shows an overview of an exemplary protocol for re-aggregating cell clusters and the morphology of the clusters during the re-aggregation.
**FIG.** 4 shows immunofluorescence images of native cell clusters (top) and re-aggregated cell clusters (bottom) stained for DAPI (e.g., nuclei), Chromogranin A (CHGA), or Insulin (INS).
**FIGS. 5A** and **5B** show the enrichment of cells expressing markers of endogenous mature pancreatic β cells in re-aggregated cell clusters. **FIG. 5A** shows the flow cytometry characterization of cells expressing different markers in native clusters, re-aggregated cell clusters, and re-aggregated cell clusters after cryopreservation. **FIG. 5B** quantifies the percentage of cells expressing different markers in native clusters, re-aggregated cell clusters, and re-aggregated cell clusters after cryopreservation.
**FIG. 6** shows the enrichment of cells expressing markers of endogenous mature pancreatic β cells in re-aggregated cell clusters, as compared to human donor islets (flow cytometry characterization of human donor islets shown at the top).
**FIG. 7** shows the distribution of cells expressing markers of endogenous mature pancreatic β cells in re-aggregated cell clusters and cells that failed to re-aggregate.
**FIG. 8** shows the depletion of cells expressing markers of off-target cell populations (SOX2 expressing cells and SOX9 expressing cells) in re-aggregated cell clusters with and without cryopreservation.
**FIG. 9** shows immunofluorescence images of native cell clusters (top) and re-aggregated cell clusters (bottom) stained for (left) DAPI (e.g., nuclei) or (right) Ki67.
**FIG. 10** shows exemplary *in vitro* glucose-stimulated insulin secretion responses of the native clusters, re-aggregated cell clusters, and re-aggregated cell clusters after cryopreservation. SI stands for stimulation index as calculated as a ratio of insulin secretion in response to 20 mM glucose challenge versus 2.8 mM glucose challenge.
**FIG. 11** shows *in vitro* glucose-stimulated insulin secretion responses of re-aggregated cell clusters after cryopreservation following sequential changes in glucose concentration and *in vitro* insulin secretion in response to KCl challenge.
**FIG. 12** shows insulin content in native clusters, re-aggregated cell clusters, and re-aggregated cell clusters after cryopreservation.
**FIG. 13** shows *in vivo* glucose-stimulated insulin secretion responses of native clusters and re-aggregated cell clusters at 2 weeks.
**FIG. 14** shows *in vivo* glucose-stimulated insulin secretion responses of native clusters and re-aggregated cell clusters at 4 weeks.

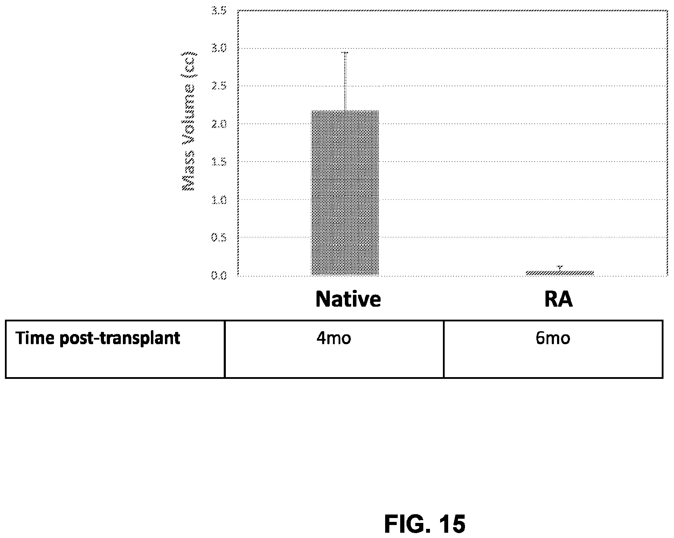

**FIG. 15** shows off-target growth in explanted grafts of native cell cluster and re-aggregated cell clusters following transplant.
**FIG. 16** shows (left) a sample graph of oxygen consumption rate (OCR) over time of cells with the sequential addition of various chemicals to measure parameters of mitochondrial function, and (right) a diagram of the mitochondrial membrane showing how the various chemicals affect mitochondrial function.
**FIG. 17** shows oxygen consumption rate before and after glucose stimulation, for native cell clusters, re-aggregated cell clusters, and human islet cells.
**FIG. 18** shows OCR levels in a mitochondrial stress test, over time for human islets and islets derived using the methods of the present disclosure.
**FIG. 19** shows histological staining of a tissue cross section of the kidney with a graft of re-aggregated cell clusters (SC-Islet Graft) for CHGA and a human marker (HuMit).
**FIG. 20** shows immunofluorescence staining of a tissue cross section of the kidney with a graft of re-aggregated cell clusters (SC-Islet Graft) for C-peptide, glucagon, and DAPI (e.g., nuclei).

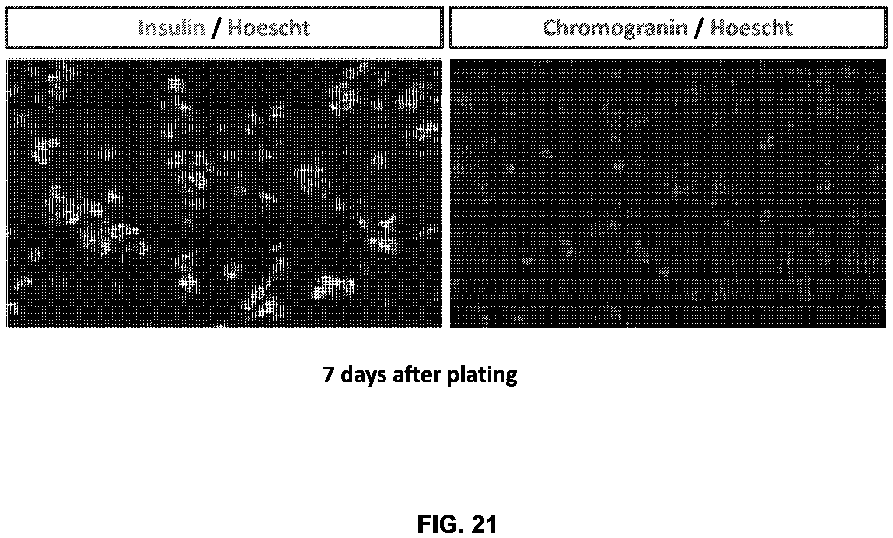

**FIG. 21** shows immunofluorescence images of re-aggregated cell clusters for (left) insulin and Hoechst or (right) chromogranin A and Hoechst showing the effects of using Laminin-332 (LN-332) as an extracellular matrix protein on cell growth.

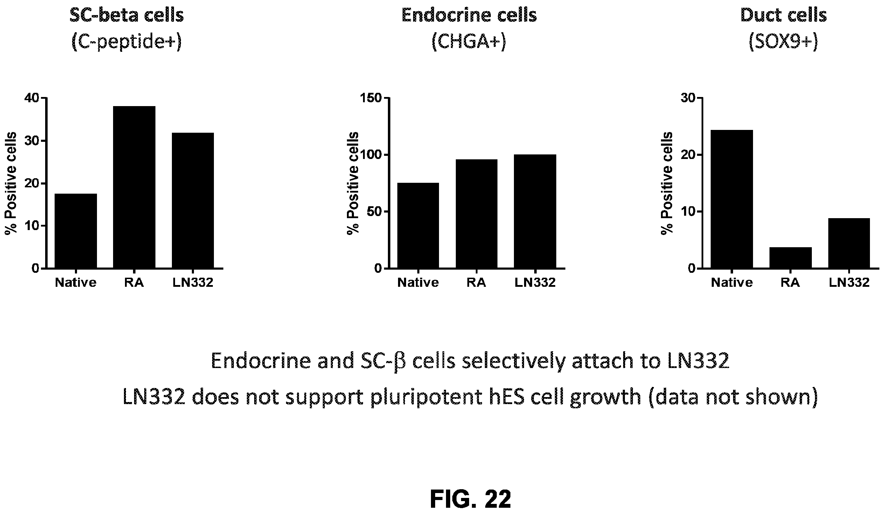

**FIG. 22** shows bar graphs of the percentage of cells positive for C-peptide (left panel), Chromogranin A (middle panel), and SOX9 (right panel) in native clusters, re-aggregated clusters (RA) and cells grown using an LN-332 matrix protein.

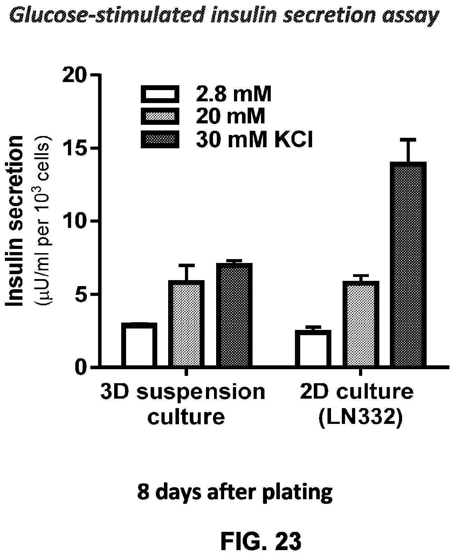

**FIG. 23** shows *in vitro* glucose-stimulated insulin secretion of cells grown using 3D suspension culture (left) and 2D culture using LN-332 (right) 8 days post-plating.
**FIG. 24** shows effects of different stirring speeds on the size of re-aggregated clusters over the days post re-aggregation.
**FIG. 25** shows heterogeneous composition of the re-aggregated cell cluster as indicated by expression of different cell markers.
**FIG. 26A** shows quantification of percentages of endocrine cells and β (beta) cells in native and re-aggregated cell clusters, respectively, as measured by flow cytometry (FACS).
**FIG. 26B** shows quantification of insulin stimulation indices of native and re-aggregated cell clusters.
**FIG. 27** shows, in another example, *in vitro* glucose-stimulated insulin secretion responses of the native clusters, re-aggregated cell clusters, and re-aggregated cell clusters after cryopreservation. SI stands for stimulation index as a ratio of insulin secretion in response to 20 mM glucose challenge versus 2.8 mM glucose challenge.
**FIG. 28** shows dynamic glucose stimulated insulin secretion over time of human donor islets, native and re-aggregated cell clusters in response to a series of dynamic glucose challenges and KCl challenge (left) and quantification of the responses (right).
**FIG. 29** shows long-term *in vivo* glycemic control by re-aggregated cell clusters in diabetic mice.

### DETAILED DESCRIPTION OF THE DISCLOSURE

While various embodiments of the disclosure have been shown and described herein, it will be obvious to those skilled in the art that such embodiments are provided by way of example only. Numerous variations, changes, and substitutions may occur to those skilled in the art without departing from the disclosure. It should be understood that various alternatives to the embodiments of the disclosure described herein may be employed.

### I. Overview

Provided herein are cell clusters that resemble the function and characteristics of endogenous pancreatic islets. Also provided herein are methods of making and using such cell clusters.

Cell clusters provided herein can resemble the characteristics of endogenous pancreatic islets. For example, the cell clusters can have a diameter similar to an endogenous pancreatic islet, e.g., between about 50 µm and about 250 µm, between about 75 µm and about 250 µm, or between about 100 µm and about 200 µm. The cell clusters can comprise a plurality of cells expressing marker genes of an endogenous mature pancreatic β cell. In some cases, at least about 70%, at least about 80%, at least about 90%, at least about 95%, or 100% cells in the cell cluster express chromogranin A (CHGA). In some cases, at least about 35%, at least about 40%, at least about 50%, or at least about 60% cells in the cell cluster express both NKX6.1 and insulin or C-peptide.

The cell clusters can also resemble the function of endogenous pancreatic islets. For example, the cell clusters can exhibit an *in vitro* glucose-stimulated insulin secretion response to a glucose challenge. When transplanted to a subject, the cell clusters can also exhibit an *in vivo* glucose-stimulated insulin secretion response to a glucose challenge in the subject, e.g., within 14 days after the transplantation.

This disclosure also provides methods of treating diabetes (e.g., type I or type II diabetes) by administering, e.g., transplanting, the cell clusters resembling the function and characteristics of endogenous pancreatic islets to a subject in need thereof. The cell clusters can be transplanted under a kidney capsule and with a biodegradable scaffold.

Also provided herein are methods for making cell clusters that resemble the functions and characteristics of endogenous pancreatic islets. The methods can comprise dissociating a first cell cluster. The cells from the first cell cluster can then be seeded in a spinner flask and cultured in a medium comprising triiodothyronine (T3) and an activin receptor-like kinase-5 (Alk5) inhibitor. In some cases, the cells from the first cell cluster are cultured in a medium that does not comprise serum, e.g., does not comprise fetal bovine serum (FBS). In some cases, the medium does not comprise T3. In some cases, the medium does not comprise an exogenous thyroid hormone signaling pathway activator. In some cases, the medium does not comprise Alk5 inhibitor. In some cases, the medium does not comprise an exogenous small molecule compound. In some cases, the medium does not comprise an exogenous inhibitor of Rho-associated, coiled-coil containing protein kinase (ROCK). In the spinner flask, the cells can re-aggregate into a second cell cluster resembling the functions and characteristics of endogenous pancreatic islets. The second cell cluster can comprise at least one pancreatic β cell (e.g., a non-native β cell differentiated from a stem cell).

Further provided herein are methods for enriching pancreatic β cells (e.g., a non-native β cell differentiated form a stem cell) in a cell cluster. The terms "enriching" and its grammatical equivalences can mean that the yield (fraction) of cells of one type is increased by at least about 5% over the fraction of cells of that type in the starting cluster or culture. The methods can comprise dissociating a first cell cluster comprising at least a pancreatic β cell (e.g., a non-native β cell differentiated from a stem cell). The cells from the first cell can then be seeded in a spinner flask and cultured in a medium comprising T3 and an Alk5 inhibitor. In the spinner flask, the cells can re-aggregate into a second cell cluster. The second cell cluster can comprise more cells expressing markers of an endogenous mature pancreatic β cell and exhibiting *in vitro* and *in vivo* glucose-stimulated insulin secretion responses to glucose challenges, compared to the first cell cluster.

### II. Cell clusters resembling the functions and characteristics of endogenous pancreatic islets

Provided herein are cell clusters that resemble the functions and characteristics of endogenous pancreatic islets. Such cell clusters can mimic the function of endogenous pancreatic islets in regulating metabolism, e.g., glucose metabolism in a subject. Thus, the cell clusters can be transplanted to a subject for treating disease resulting from insufficient pancreatic islet function, e.g., diabetes. The terms "cluster" and "aggregate" can be used interchangeably, and refer to a group of cells that have close cell-to-cell contact, and in some cases, the cells in a cluster can be adhered to one another.

A cell cluster herein can comprise at least one non-native cell, e.g., a non-native pancreatic β cell. A non-native cell (e.g., a non-native pancreatic β cell) can share characteristics of an endogenous cell (e.g., an endogenous mature pancreatic β cell), but is different in certain aspects (e.g., gene expression profiles). A non-native cell can be a genetically modified cell. A non-native cell can be a cell differentiated from a progenitor cell, e.g., a stem cell. The stem cell can be an embryonic stem cell (ESC) or induced pluripotent stem cell (iPSC). In some cases, the non-native cell can be a cell differentiated from a progenitor cell *in vitro.* In some cases, the non-native cell can be a cell differentiated from a progenitor cell in *in vivo.* For example, a cell cluster can comprise at least one non-native pancreatic β cell. The non-native pancreatic β cells can be those described in U.S. Patent Application Nos. 14/684,129 and 14/684,101, which are incorporated herein in their entireties. A cell cluster can comprise a plurality of non-native pancreatic β cells. In some cases, at least about 20%, 30%, 40%, 50%, 60%, 70%, 80%, 90%, 99% cells in a cell cluster are non-native pancreatic β cells. A cell cluster can comprise one or more native cells. For example, a cell cluster can comprise one or more primary cells, e.g., primary cells from an endogenous pancreatic islet.

A cell cluster can comprise one or more cells expressing at least one marker of an endogenous cell, e.g., an endogenous mature pancreatic β cell. The term "marker" can refer to a molecule that can be observed or detected. For example, a marker can include, but is not limited to, a nucleic acid, such as a transcript of a specific gene, a polypeptide product of a gene, a non-gene product polypeptide, a glycoprotein, a carbohydrate, a glycolipid, a lipid, a lipoprotein, or a small molecule. In many cases, a marker can refer to a molecule that can be characteristic of a particular type of cell, so that the marker can be called as a marker of the type of cell. For instance, Insulin gene can be referred to as a marker of β cells. In some cases, a marker is a gene. Non-limiting of markers of an endogenous mature pancreatic β cell include insulin, C-peptide, PDX1, NKX6.1, CHGA, MAFA, ZNT8, PAX6, NEUROD1, glucokinase (GCK), SLC2A, PCSK1, KCNJ11, ABCC8, SLC30A8, SNAP25, RAB3A, GAD2, and PTPRN.

A cell cluster can comprise one more cells expressing one or multiple markers of an endogenous cell, e.g., an endogenous mature pancreatic β cell. For example, cell cluster can comprise one or more cells co-expressing at least 2, 3, 4, 5, 6, 7, 8, 9, 10, 15, or 20 marker(s) of an endogenous cell, e.g., an endogenous mature pancreatic β cell. In some cases, a cell cluster comprises cells that express NKX6.1 and C-peptide, both of which can be markers of a β cell.

A cell cluster can comprise a plurality of cells expressing at least one marker of an endogenous cell. For example, at least about 1%, 5%, 10%, 20%, 30%, 40%, 50%, 60%, 70%, 80%, 90%, 95%, 99% cells in a cell cluster can express at least one marker of an endogenous cell. In some cases, all cells in a cell cluster can express a marker of an endogenous cell. In some cases, the endogenous cell can be a pancreatic cell, e.g., a pancreatic β cell, pancreatic α cells, pancreatic β cells, pancreatic Δ cells, or pancreatic γ cells. A cell cluster as provided herein can comprise a heterogeneous group of cells, e.g., cells of different types. For example, the cell cluster can comprises a cell expressing insulin/C-peptide, which can be a marker of a pancreatic β cell, a cell expressing glucagon, which can be a marker of a pancreatic α cell, a cell expressing somatostatin, which can be a marker of a pancreatic Δ cell, a cell expressing pancreatic polypeptides, or any combination thereof.

For example, the cell cluster herein can comprise a plurality of cells expressing one or more markers of an endogenous mature pancreatic β cell. For example, at least about 10%, 20%, 30%, 40%, 50%, 60%, 70%, 80%, 90%, 95%, or 99% cells in the cell cluster can express one or more markers of an endogenous mature pancreatic β cell.

The cell cluster can comprise a plurality of cells expressing CHGA. In some cases, at least about 30%, 40%, 50%, 60%, 70%, 80%, 90%, 95%, or 99% cells in the cell cluster express CHGA. In some cases, at least about 85% cells in a cell cluster can express CHGA. In some cases, a cell cluster can comprise about 90% cell expressing CHGA. In some cases, a cell cluster can comprise about 95% cells expressing CHGA. In certain cases, all cells in a cell cluster can express CHGA.

The cell cluster can comprise a plurality of cells expressing NKX6.1. For example, at least about 30%, 40%, 50%, 60%, 70%, 80%, 90%, 95%, 99% cells in a cell cluster can express NKX6.1. In some cases, at least about 50% cells in a cell cluster can express NKX6.1. In some cases, all cells in a cell cluster can express NKX6.1.

The cell cluster can comprise a plurality of cells expressing C-peptide. For example, at least about 30%, 40%, 50%, 60%, 70%, 80%, 90%, 95%, or 99% cells in a cell cluster can express C-peptide. In some cases, at least about 60% cells in a cell cluster can express C-peptide. In some cases, all cells in a cell cluster can express C-peptide.

The cell cluster can comprise a plurality of cells expressing both NKX6.1 and C-peptide. For example, at least about 10%, 20%, 30%, 40%, 50%, 60%, 70%, 80%, 90%, 95%, or 99% cells in a cell cluster can express C-peptide. In some cases, at least about 35% cells in a cell cluster can express NKX6.1 and C-peptide. In some cases, at least about 40% cells in a cell cluster can express NKX6.1 and C-peptide. In some cases, at least about 35% cells in a cell cluster can express NKX6.1 and C-peptide. In some cases, a cell cluster can comprise about 60% cells expressing NKX6.1 and C-peptide. In some cases, a cell cluster can comprise about 75% cell expressing NKX6.1 and C-peptide. In some cases, all cells in a cell cluster can express NKX6.1 and C-peptide.

The cell cluster can comprise very few to none of stem cells or progenitor cells, e.g., pancreatic progenitor cells. For example, a cell cluster as provided herein can comprise at most about 5% cells, at most about 5% cells, at most about 5% cells, at most about 5% cells, at most about 5% cells, at most about 2% cells, at most about 1% cells, at most about 0.5% cells, at most about 0.1% cells, at most about 0.05% cells, at most about 0.01% cells, or no cells expressing LIN28. In some examples, a cell cluster as provided herein can comprise at most about 5% cells, at most about 5% cells, at most about 5% cells, at most about 5% cells, at most about 5% cells, at most about 2% cells, at most about 1% cells, at most about 0.5% cells, at most about 0.1% cells, at most about 0.05% cells, at most about 0.01% cells, or no cells expressing Ki67.

In some cases, a cell cluster can comprise at most 3% cells, at most about 2% cells, at most about 1% cells, at most about 0.5% cells, at most about 0.1% cells, at most about 0.05% cells, at most about 0.01% cells, or no cells expressing SOX2. In some cases, a cell cluster can comprise about 1% cells expressing SOX2. In some cases, a cell cluster can comprise about 0.6% cells expressing SOX2. In some cases, a cell cluster can comprise about 0.3% cells expressing SOX2. In some cases, a cell cluster can comprise about 0.1% cells expressing SOX2.

In some examples, a cell cluster can comprise at most 10% cells, at most about 8% cells, at most about 6% cells, at most about 5% cells, at most about 2% cells, at most about 1% cells, at most about 0.5% cells, at most about 0.1% cells, at most about 0.05% cells, at most about 0.01% cells, or no cells expressing SOX9. In some cases, a cell cluster can comprise about 2% cells expressing SOX9. In some cases, a cell cluster can comprise about 6% cells expressing SOX9. In some cases, a cell cluster can comprise about 1.2% cells expressing SOX9.

A cell cluster herein can exhibit one or multiple glucose stimulated insulin secretion (GSIS) response(s) *in vitro* when exposed to glucose challenge(s). The GSIS responses can resemble the GSIS responses of an endogenous pancreatic islet. In some cases, the cell cluster exhibits an *in vitro* GSIS response to a glucose challenge. In some cases, the cell cluster exhibits *in vitro* GSIS responses to multiple glucose challenges, such as sequential glucose challenges. For example, the cell cluster can exhibit *in vitro* GSIS responses to at least 2, 3, 4, 5, 6, 7, 8, 9, 10 sequential glucose challenges.

A cell cluster as provided herein can comprise at least one cell exhibiting *in vitro* GSIS. For example, at least one cell in the cell cluster can be referred to as a mature pancreatic β cell. In some cases, the at least one cell is a non-native pancreatic β cell. In some cases, the at least one cell is a pancreatic β cell resembling a native/endogenous β cell. In some cases, the cell exhibits an *in vitro* glucose stimulated insulin secretion (GSIS) response. In some cases, the at least one cell exhibits a GSIS response to at least one glucose challenge. In some cases, the cell exhibits a GSIS response to at least two sequential glucose challenges. In some cases, the cell exhibits a GSIS response to at least three sequential glucose challenges

As provided herein, a cell cluster can exhibit GSIS stimulation index similar to an endogenous pancreatic islet. Stimulation index of a cell cluster or a cell can be characterized by the ratio of insulin secreted in response to high glucose concentrations compared to low glucose concentrations. For example, a stimulation index of a cell cluster or a cell as provided herein can be calculated as a ration of insulin secreted in response to 20 mM glucose stimulation versus insulin secreted in response to 2.8 mM glucose stimulation. In some examples, the stimulation index of a cell cluster or a cell as provided herein is greater than or equal to 1 , or greater than or equal to 1.1 , or greater than or equal to 1 .3, or greater than or equal to 2, or greater than or equal to 2.3, or greater than or equal to 2.6. In some instances, the cell cluster or the cell exhibits cytokine-induced apoptosis in response to a cytokine. In some cases, the cytokine comprises interleukin-β (IL-β), interferon-γ (INF- γ), tumor necrosis factor-α (TNF-α), or any combination thereof. In some cases, insulin secretion from the cell cluster or the cell is enhanced in response to an anti-diabetic agent. In some cases, the anti-diabetic agent comprises a secretagogue selected from the group consisting of an incretin mimetic, a sulfonylurea, a meglitinide, and combinations thereof. In some cases, the cell cluster or the cell is monohormonal. In some cases, the cell cluster or the cell exhibits a morphology that resembles the morphology of an endogenous mature pancreatic β cell. In some cases, the cell cluster or the cell exhibits encapsulated crystalline insulin granules under electron microscopy that resemble insulin granules of an endogenous mature pancreatic β cell. In some cases, the cell cluster or the cell exhibits a low rate of replication. In some cases, the cell cluster or the cell exhibits a glucose stimulated Ca²⁺ flux (GSCF) that resembles the GSCF of an endogenous mature pancreatic β cell. In some cases, the cell cluster or the cell exhibits a GSCF response to at least one glucose challenge. In some cases, the cell cluster or the cell exhibits a GSCF response to at least two glucose challenges. In some cases, the cell cluster or the cell exhibits a GSCF response to at least three glucose challenges. In some cases, the cell cluster or the cell exhibits an increased calcium flux. In some cases, the increased calcium flux comprises an increased amount of influx or a ratio of influx at low relative to high glucose concentrations.

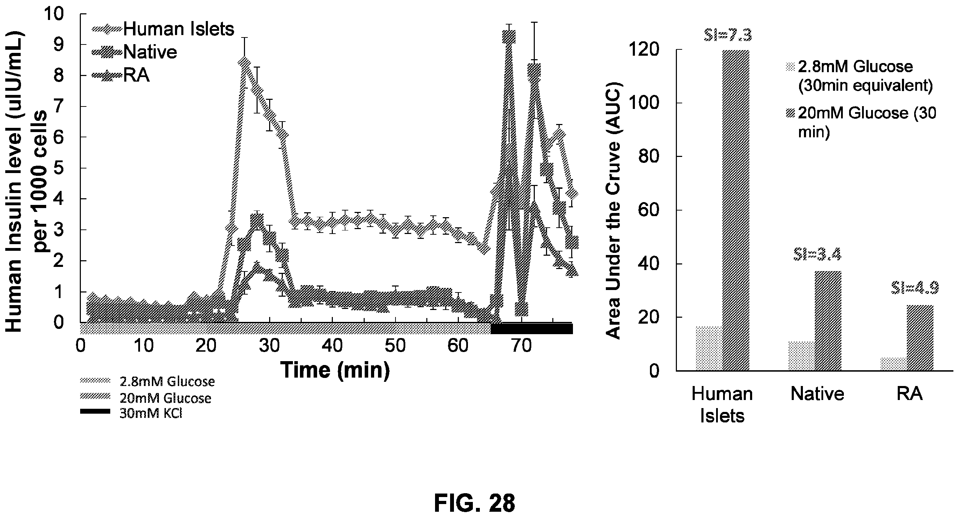

A cell cluster as provided herein can exhibit biphasic insulin secretion in response to a high glucose concentration stimulation similar to an endogenous pancreatic islet, e.g., a human pancreatic islet. A biphasic insulin secretion can be a phenomenon characteristic of an endogenous pancreatic islet, e.g., human islet. As demonstrated in **FIG. 28****,** in response to a high glucose concentration challenge, e.g., 10mM, 15mM, 20 mM, or 30mM, a cell cluster as provided herein, e.g., a reaggregated pancreatic cell cluster, can exhibit a transient increase in insulin secretion to a peak value followed by a rapid decrease to a relatively elevated insulin secretion level, e.g., a level that is higher than an insulin secretion level in response to a lower glucose concentration, e.g., 2.8 mM glucose. Such a transient increase and decrease process can be termed as a first phase of the biphasic insulin secretion pattern. With a persistent high glucose challenge, the first phase can be thus followed by a second phase, in which the insulin secretion by the cell cluster can be maintained at the relatively elevated level. The second phase can last for an extended period, e.g., as long as the high glucose concentration challenge lasts, or relatively longer than the first phase. Such a biphasic insulin secretion pattern can be due to intrinsic cellular signaling changes that are characteristic of a mature native pancreatic β cell.

When transplanted to a subject, a cell cluster can exhibit one or more *in vivo* GSIS responses when exposed to glucose challenge(s). The cell cluster herein can be capable of exhibiting an *in vivo* GSIS response within a short period of time after transplanted to a subject. For example, the cell cluster can exhibit an *in vivo* GSIS within about 6, 12, or 24 hours after transplantation. In some cases, the cell cluster exhibits an *in vivo* GSIS within about 2 days, 4 days, 6 days, 8 days, 10 days, 12 days, 14 days, 21 days, 28 days, 35 days, or 42 days after transplantation. The amount of insulin secreted by the cell cluster can be similar or higher than an endogenous pancreatic islet. The term "about" in relation to a reference numerical value as used through the application can include a range of values plus or minus 10% from that value. For example, the amount "about 10" includes amounts from 9 to 11. For example, the term "about" in relation to a reference numerical value can also include a range of values plus or minus 10%, 9%, 8%, 7%, 6%, 5%, 4%, 3%, 2%, or 1% from that value.

The cell cluster can maintain the ability of exhibiting *in vivo* GSIS responses for a period of time after transplanted into a subject. For example, an *in vivo* GSIS response of the cell cluster can be observed up to at least 2 weeks, 3 weeks, 4 weeks, 5 weeks, 10 weeks, 15 weeks, 20 weeks, 6 months, 7 months, 8 months, 9 months, 10 months, 11 months, 1 year, 2 years, 3 years, 4 years, 5 years, 10 years, 20 years, 30 years, 40 years, 60 years, 80 years, or 100 years after transplantation of the cell cluster into a subject (e.g., a human).

The GSIS of a cell cluster can be measured by a stimulation index. A stimulation index of a cell cluster can equal to the ratio of insulin secreted in response to a high glucose concentration compared to insulin secreted in response to a low glucose concentration. A cell cluster can have a stimulation index similar to an endogenous pancreatic islet. In some cases, a cell cluster has a stimulation index of at least 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0.

The amount of insulin secreted by a cell cluster in response to a glucose challenge (e.g., a high concentration, such as 20mM, of glucose) can range from about 0.1 µIU/10³ cells to about 5 µIU/10³ cells, from about 0.2 µIU/10³ cells to about 4 µIU/10³ cells, from about 0.2 µIU/10³ cells to about 3 µIU/10³ cells, or from about 0.23 µIU/10³ cells to about 2.7 µIU/10³ cells. In some cases, the amount of insulin secreted by a cell cluster in response to a glucose challenge (e.g., a high concentration, such as 20mM, of glucose) is at least 0.05, 0.1, 0.15, 0.2, 0.21, 0.22, 0.23, 0.24, 0.25, 0.26, 0.27, 0.28, 0.29, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, or 3 µIU/10³ cells.

A cell cluster can secrete both pro-insulin and insulin. For example, a cell cluster can secrete pro-insulin and insulin at a proinsulin-to-insulin ratio substantially the same as the ratio of pro-insulin to insulin secreted by an endogenous pancreatic islet. In some cases, a cell cluster secretes pro-insulin and insulin at a proinsulin-to-insulin ratio of from about 0.01 to about 0.05, from about 0.02 to about 0.04, from about 0.02 to about 0.03, or from 0.029 to about 0.031. In some cases, a cell cluster secretes pro-insulin and insulin at a proinsulin-to-insulin ratio of about 0.02, 0.021, 0.022, 0.023, 0.024, 0.025, 0.026, 0.027, 0.028, 0.029, 0.03, 0.031, 0.032, 0.033, 0.034, 0.035, 0.036, 0.037, 0.038, 0.039, or 0.04.

A cell cluster can be in a size similar to an endogenous pancreatic islet. For example, a cell cluster can have a diameter similar to an endogenous pancreatic islet. A diameter of a cell cluster can refer to the largest linear distance between two points on the surface of the cell cluster. In some cases, the diameter of a cell cluster is at most 300 µm, 200 µm, 150 µm, 100 µm, 90 µm, 80 µm, 70 µm, 60 µm, 50 µm, or 40 µm. The diameter of a cell cluster can be from about 75 µm to about 250 µm. The diameter of a cell cluster can be at most 100 µm.

A cell cluster can comprise very few or no dead cells. The cell cluster can be in a size that allows effective diffusion of molecules (e.g., nutrition and gas) from surrounding environment into the core of the cell cluster. The diffused molecule can be important for the survival and function of the cells in the core. In some cases, the cell cluster can have less than about 1%, 2%, 3%, 4%, 5%, 6%, 7%, 8%, 9%, 10% of dead cells, e.g., dead cells in its core. In some cases, a cell cluster can have no dead cell. The dead cells can be apoptotic cells, narcotic cells or any combination thereof.

A cell cluster can comprise one or multiple types of cells. In some cases, a cell cluster comprises one or more types of pancreatic cells. For example, the cell cluster can comprise one or more pancreatic β cell, pancreatic α cells, pancreatic Δ cells, pancreatic γ cells, and any combination thereof. In some cases, the pancreatic cells can be non-native pancreatic cells, *e.g.,* cells derived from stem cells, such as ESCs and/or iPSCs. In some cases, the cell cluster can also comprise one or more progenitor cells of mature pancreatic cells, including iPSCs, ESCs, definitive endoderm cells, primitive gut tube cells, Pdx1-positive pancreatic progenitor cells, Pdx1-positive/ NKX6.1-positive pancreatic progenitor cells, Ngn3-positive endocrine progenitor cells, and any combination thereof.

A cell cluster can exhibit cytokine-induced apoptosis in response to cytokines. For example, the cell cluster can exhibit cytokine-induced apoptosis in response to a cytokine such as interleukin-1β (IL-β), interferon-γ (INF-γ), tumor necrosis factor-α (TNF-α), and combinations thereof.

Insulin secretion from a cell cluster herein can be enhanced by an anti-diabetic drug (e.g., an anti-diabetic drug acting on pancreatic β cells *ex vivo, in vitro,* and/or *in vivo*)*.* The disclosure can contemplate any known anti-diabetic drug. In some cases, insulin secretion from a cell cluster can be enhanced by a secretagogue. The secretagogue can be an incretin mimetic, a sulfonylurea, a meglitinide, and combinations thereof.

A cell cluster can comprise a monohormonal. For example, the cell cluster can comprise a pancreatic cell (e.g., a pancreatic β cell, pancreatic α cells, pancreatic β cells, pancreatic Δ cells, or pancreatic γ cells) that is monohormonal. In some cases, the cell cluster comprises an insulin-secreting non-native pancreatic cell that is monohormonal. A cell cluster can comprise a polyhormonal. In some case, a cell cluster comprises a monohormonal cell and a polyhormonal cell.

A cell cluster can comprise a cell (e.g., a non-native pancreatic cell) having a morphology that resembles the morphology of an endogenous mature pancreatic β cell. In some cases, the cell cluster can comprise cell encapsulating crystalline insulin granules that resemble insulin granules of an endogenous mature pancreatic β cell, e.g., as detected by electron microscopy. A cell cluster can comprise a plurality cells having a morphology that resembles the morphology of an endogenous mature pancreatic β cell. For example, at least about 10%, 20%, 30%, 40%, 50%, 60%, 70%, 80%, 90% cells in a cell cluster can encapsulate crystalline insulin granules that resemble insulin granules of an endogenous mature pancreatic β cell. In some cases, 100% cells in a cell cluster encapsulate crystalline insulin granules that resemble insulin granules of an endogenous mature pancreatic β cell.

A cell cluster can exhibit glucose-stimulated calcium (Ca²⁺) flux to one or more glucose challenges. In some cases, a cell cluster exhibits a glucose-stimulated Ca²⁺ flux (GSCF) that resembles the GSCF of an endogenous pancreatic islet. In some cases, a cell cluster exhibits a GSCF response to at least 1, 2, 3, 4, 5, 6, 8, or 10 sequential glucose challenges in a manner that resembles the GSCF response of an endogenous pancreatic islet to multiple glucose challenges. A cell cluster can exhibit an *in vitro* and/or *in vivo* GSCF response when exposed to a glucose challenge.

A cell cluster can comprise cells originated from any species. For example, a cell cluster can comprise cells from a mammalian species, with non-limiting examples including a murine, bovine, simian, porcine, equine, ovine, or human cell. In some cases, at least one cell in the cell cluster is a human cell.

Provided herein also include compositions comprising a cell clusters disclosed through the application. In addition to the cell cluster, the compositions can further comprise a scaffold or matrix that can be used for transplanting the cell clusters to a subject. A scaffold can provide a structure for the cell cluster to adhere to. The cell cluster can be transplanted to a subject with the scaffold. The scaffold can be biodegradable. In some cases, a scaffold comprises a biodegradable polymer. The biodegradable polymer can be a synthetic polymer, such as poly(lactide) (PLA), poly(glycolic acid) (PGA), poly(lactide-co-glycolide) (PLGA), and other polyhydroxyacids, poly(caprolactone), polycarbonates, polyamides, polyanhydrides, polyphosphazene, polyamino acids, polyortho esters, polyacetals, polycyanoacrylates, and biodegradable polyurethanes. The biodegradable polymer can also be a natural polymer, such as albumin, collagen, fibrin, polyamino acids, prolamines, and polysaccharides (e.g., alginate, heparin, and other naturally occurring biodegradable polymers of sugar units). Alternatively, the scaffold can be non-biodegradable. For example, a scaffold can comprise a non-biodegradable polymer, such as polyacrylates, ethylene- vinyl acetate polymers and other acyl-substituted cellulose acetates and derivatives thereof, polyurethanes, polystyrenes, polyvinyl chloride, polyvinyl fluoride, poly(vinyl imidazole), chlorosulphonated polyolefins, and polyethylene oxide.

### III Pharmaceutical compositions

In some cases, the present disclosure provides pharmaceutical compositions that can utilize non-native pancreatic beta cell populations and cell components and products in various methods for treatment of a disease (e.g., diabetes). Certain cases encompass pharmaceutical compositions comprising live cells (e.g., non-native pancreatic beta cells alone or admixed with other cell types). Other cases encompass pharmaceutical compositions comprising non-native pancreatic beta cell components (e.g., cell lysates, soluble cell fractions, conditioned medium, ECM, or components of any of the foregoing) or products (e.g., trophic and other biological factors produced by non-native pancreatic beta cells or through genetic modification, conditioned medium from non-native pancreatic beta cell culture). In either case, the pharmaceutical composition may further comprise other active agents, such as antiinflammatory agents, exogenous small molecule agonists, exogenous small molecule antagonists, anti-apoptotic agents, antioxidants, and/or growth factors known to a person having skill in the art.

Pharmaceutical compositions of the present disclosure can comprise non-native pancreatic beta cell, or components or products thereof, formulated with a pharmaceutically acceptable carrier (e.g. a medium or an excipient). The term pharmaceutically acceptable carrier (or medium), which may be used interchangeably with the term biologically compatible carrier or medium, refers to reagents, cells, compounds, materials, compositions, and/or dosage forms that are not only compatible with the cells and other agents to be administered therapeutically, but also are suitable for use in contact with the tissues of human beings and animals without excessive toxicity, irritation, allergic response, or other complication. Suitable pharmaceutically acceptable carriers can include water, salt solution (such as Ringer's solution), alcohols, oils, gelatins, and carbohydrates, such as lactose, amylose, or starch, fatty acid esters, hydroxymethylcellulose, and polyvinyl pyrolidine. Such preparations can be sterilized, and if desired, mixed with auxiliary agents such as lubricants, preservatives, stabilizers, wetting agents, emulsifiers, salts for influencing osmotic pressure, buffers, and coloring. Pharmaceutical compositions comprising cellular components or products, but not live cells, can be formulated as liquids. Pharmaceutical compositions comprising living non-native pancreatic beta cells can be formulated as liquids, semisolids (e.g., gels, gel capsules, or liposomes) or solids (e.g., matrices, scaffolds and the like).

Pharmaceutical compositions may comprise auxiliary components as would be familiar to a person having skill in the art. For example, they may contain antioxidants in ranges that vary depending on the kind of antioxidant used. Reasonable ranges for commonly used antioxidants are about 0.01% to about 0.15% weight by volume of EDTA, about 0.01% to about 2.0% weight volume of sodium sulfite, and about 0.01% to about 2.0% weight by volume of sodium metabisulfite. One skilled in the art may use a concentration of about 0.1% weight by volume for each of the above. Other representative compounds include mercaptopropionyl glycine, N-acetyl cysteine, beta-mercaptoethylamine, glutathione and similar species, although other anti-oxidant agents suitable for renal administration, e.g. ascorbic acid and its salts or sulfite or sodium metabisulfite may also be employed.

A buffering agent may be used to maintain the pH of formulations in the range of about 4.0 to about 8.0; so as to minimize irritation in the target tissue. For direct intraperitoneal injection, formulations should be at pH 7.2 to 7.5, preferably at pH 7.35-7.45. The compositions may also include tonicity agents suitable for administration to the kidney. Among those suitable is sodium chloride to make formulations approximately isotonic with blood.

In certain cases, pharmaceutical compositions are formulated with viscosity enhancing agents. Exemplary agents are hydroxyethylcellulose, hydroxypropylcellulose, methylcellulose, and polyvinylpyrrolidone. The pharmaceutical compositions may have cosolvents added if needed. Suitable cosolvents may include glycerin, polyethylene glycol (PEG), polysorbate, propylene glycol, and polyvinyl alcohol. Preservatives may also be included, e.g., benzalkonium chloride, benzethonium chloride, chlorobutanol, phenylmercuric acetate or nitrate, thimerosal, or methyl or propylparabens.

Pharmaceutical compositions comprising cells, cell components or cell products may be delivered to the kidney of a patient in one or more of several methods of delivery known in the art. In some cases, the compositions are delivered to the kidney (e.g.,on the renal capsule and/or underneath the renal capsule). In another embodiment, the compositions may be delivered to various locations within the kidney via periodic intraperitoneal or intrarenal injection. Alternatively, the compositions may be applied in other dosage forms known to those skilled in the art, such as pre-formed or in situ-formed gels or liposomes.

Pharmaceutical compositions comprising live cells in a semi-solid or solid carrier are may be formulated for surgical implantation on or beneath the renal capsule. It should be appreciated that liquid compositions also may be administered by surgical procedures. In particular cases, semi-solid or solid pharmaceutical compositions may comprise semi-permeable gels, lattices, cellular scaffolds and the like, which may be non-biodegradable or biodegradable. For example, in certain cases, it may be desirable or appropriate to sequester the exogenous cells from their surroundings, yet enable the cells to secrete and deliver biological molecules (e.g., insulin) to surrounding cells or the blood stream. In these cases, cells may be formulated as autonomous implants comprising living non-native pancreatic beta cells or cell population comprising non-native pancreatic beta cell surrounded by a non-degradable, selectively permeable barrier that physically separates the transplanted cells from host tissue. Such implants are sometimes referred to as "immunoprotective," as they have the capacity to prevent immune cells and macromolecules from killing the transplanted cells in the absence of pharmacologically induced immunosuppression.

In other cases, various degradable gels and networks can be used for the pharmaceutical compositions of the present disclosure. For example, degradable materials particularly suitable for sustained release formulations include biocompatible polymers, such as poly(lactic acid), poly (lactic-co-glycolic acid), methylcellulose, hyaluronic acid, collagen, and the like.

In other cases, it may be desirable or appropriate to deliver the cells on or in a biodegradable, preferably bioresorbable or bioabsorbable, scaffold or matrix. These typically three-dimensional biomaterials contain the living cells attached to the scaffold, dispersed within the scaffold, or incorporated in an extracellular matrix entrapped in the scaffold. Once implanted into the target region of the body, these implants become integrated with the host tissue, wherein the transplanted cells gradually become established.

Examples of scaffold or matrix (sometimes referred to collectively as "framework") material that may be used in the present disclosure include nonwoven mats, porous foams, or self-assembling peptides. Nonwoven mats, for example, may be formed using fibers comprising a synthetic absorbable copolymer of glycolic and lactic acids (PGA/PLA), foams, and/or poly(epsilon-caprolactone)/poly(glycolic acid) (PCL/PGA) copolymer.

In another embodiment, the framework is a felt, which can be composed of a multifilament yarn made from a bioabsorbable material, e.g., PGA, PLA, PCL copolymers or blends, or hyaluronic acid. The yarn is made into a felt using standard textile processing techniques consisting of crimping, cutting, carding and needling. In another embodiment, cells are seeded onto foam scaffolds that may be composite structures. In many of the abovementioned cases, the framework may be molded into a useful shape. Furthermore, it will be appreciated that non-native pancreatic beta cells may be cultured on pre-formed, non-degradable surgical or implantable devices.

The matrix, scaffold or device may be treated prior to inoculation of cells in order to enhance cell attachment. For example, prior to inoculation, nylon matrices can be treated with 0.1 molar acetic acid and incubated in polylysine, PBS, and/or collagen to coat the nylon. Polystyrene can be similarly treated using sulfuric acid. The external surfaces of a framework may also be modified to improve the attachment or growth of cells and differentiation of tissue, such as by plasma coating the framework or addition of one or more proteins (e.g., collagens, elastic fibers, reticular fibers), glycoproteins, glycosaminoglycans (e.g., heparin sulfate, chondroitin-4-sulfate, chondroitin-6-sulfate, dermatan sulfate, keratin sulfate), a cellular matrix, and/or other materials such as, but not limited to, gelatin, alginates, agar, agarose, and plant gums, among others.

In one aspect, the present disclosure provided devices comprising a cell cluster comprising at least one pancreatic β cell. A device provided herein can be configured to produce and release insulin when implanted into a subject. A device can comprise a cell cluster comprising at least one pancreatic β cell, e.g., a non-native pancreatic β cell. A cell cluster in the device can exhibit *in vitro* GSIS. A device can further comprise a semipermeable membrane. The semipermeable membrane can be configured to retain the cell cluster in the device and permit passage of insulin secreted by the cell cluster. In some cases of the device, the cell cluster can be encapsulated by the semipermeable membrane. The encapsulation can be performed by any technique available to one skilled in the art. The semipermeable membrane can also be made of any suitable material as one skilled in the art would appreciate and verify. For example, the semipermeable membrane can be made of polysaccharide or polycation. In some cases, the semipermeable membrane can be made of poly(lactide) (PLA), poly(glycolic acid) (PGA), poly(lactide-co-glycolide) (PLGA), and other polyhydroxyacids, poly(caprolactone), polycarbonates, polyamides, polyanhydrides, polyphosphazene, polyamino acids, polyortho esters, polyacetals, polycyanoacrylates, biodegradable polyurethanes, albumin, collagen, fibrin, polyamino acids, prolamines, alginate, agarose, agarose with gelatin, dextran, polyacrylates, ethylene- vinyl acetate polymers and other acyl-substituted cellulose acetates and derivatives thereof, polyurethanes, polystyrenes, polyvinyl chloride, polyvinyl fluoride, poly(vinyl imidazole), chlorosulphonated polyolefins, polyethylene oxide, or any combinations thereof. In some cases, the semipermeable membrane comprises alginate. In some cases, the cell cluster is encapsulated in a microcapsule that comprises an alginate core surrounded by the semipermeable membrane. In some cases, the alginate core is modified, for example, to produce a scaffold comprising an alginate core having covalently conjugated oligopeptides with an RGD sequence (arginine, glycine, aspartic acid). In some cases, the alginate core is modified, for example, to produce a covalently reinforced microcapsule having a chemoenzymatically engineered alginate of enhanced stability. In some cases, the alginate core is modified, for example, to produce membrane-mimetic films assembled by in-situ polymerization of acrylate functionalized phospholipids, In some cases, microcapsules are composed of enzymatically modified alginates using epimerases, In some cases, microcapsules comprise covalent links between adjacent layers of the microcapsule membrane. In some embodiment, the microcapsule comprises a subsieve-size capsule comprising alginate coupled with phenol moieties. In some cases, the microcapsule comprises a scaffold comprising alginate-agarose. In some cases, the SC-β cell is modified with PEG before being encapsulated within alginate. In some cases, the isolated populations of cells, e.g., SC-β cells are encapsulated in photoreactive liposomes and alginate. It should be appreciated that the alginate employed in the microcapsules can be replaced with other suitable biomaterials, including, without limitation, polyethylene glycol (PEG), chitosan, polyester hollow fibers, collagen, hyaluronic acid, dextran with ROD, BHD and polyethylene glycol-diacrylate (PEGDA), poly(MPC-co-n-butyl methacrylate-co-4-vinylphenyl boronic acid) (PMBV) and poly(vinyl alcohol) (PVA), agarose, agarose with gelatin, and multilayer cases of these.

### IV Method for making the cell clusters

Further disclosed herein are methods for making cell clusters that resemble the function and characteristics of an endogenous tissue or cell cluster, e.g., an endogenous pancreatic islet. The methods can comprise dissociating a first cell cluster and re-aggregating the dissociated cells to a second cell cluster, where the second cell cluster more closely resembles the function and characteristics of an endogenous tissue or cell cluster, e.g., an endogenous pancreatic islet, compared to the first cell cluster. The term "re-aggregating" and its grammatical equivalences as used herein can refer to, when clusters are dissociated into smaller clusters or single cells, the dissociated cells then form new cell-to-cell contacts and form new clusters. The methods can be used for producing a cell cluster *in vitro* by a) dissociating a plurality of cells from a first cell cluster; and b) culturing the plurality of cells from a) in a medium, thereby allowing the plurality of cells to form a second cell cluster. In some cases, the second cell cluster is an *in vitro* cell cluster. The first cell cluster can be an *in vitro* cell cluster, e.g., a cluster formed by a suspension of single cells *in vitro* in a culture medium. In some cases, the first cell cluster can be an *ex vivo* cell cluster, e.g., a cell cluster that is formed in a body of a live organism and isolated from said organism. For example, a first cell cluster that the method provided herein is applicable to can be a human pancreatic islet. In some cases, the first cell cluster can be a cadaveric pancreatic islet.

A method provided herein can enrich pancreatic cells in a cell cluster, e.g., a pancreatic β cell, an endocrine cell, or an endocrine progenitor cell. In some examples, the method can reduce or eliminate stem cells or pancreatic progenitor cells from a cell cluster. In some cases, the second cell cluster comprises a higher percentage cells that express chromogranin A as compared the first cell cluster. In some cases, the second cell cluster comprises a higher percentage cells that express NKX6.1 and C-peptide as compared the first cell cluster. In some cases, the second cell cluster comprises a lower percentage cells that express SOX2 as compared the first cell cluster. In some cases, the second *in vitro* cell cluster comprises a lower percentage of cells that express SOX9 as compared the first cell cluster.

In some cases, the medium comprises a thyroid hormone signaling pathway activator and a transforming growth factor β (TGF-β) signaling pathway inhibitor. In some cases, the medium comprises a) serum, and b) one or both of a thyroid hormone signaling pathway activator and a TGF-β signaling pathway inhibitor. In some cases, the medium for reaggregation as provided herein (reaggregation medium) can comprise no small molecule compounds. For example, the reaggregation medium can comprise no thyroid hormone signaling pathway activator. In some cases, the reaggregation medium does not comprise triiodothyronine (T3), or merely a trace amount of T3. The reaggregation medium can comprise no TGFβ signaling pathway inhibitor. In some cases, the reaggregation medium does not comprise an Alk5 inhibitor (Alk5i), or merely a trace amount of Alk5i.

Dissociating of the first cell cluster can be performed using methods known in the art. Non-limiting exemplary methods for dissociating cell clusters include physical forces (e.g., mechanical dissociation such as cell scraper, trituration through a narrow bore pipette, fine needle aspiration, vortex disaggregation and forced filtration through a fine nylon or stainless steel mesh), enzymatic dissociation using enzymes such as trypsin, collagenase, TrypLE^{™}, and the like, or a combination thereof. After dissociation, cells from the first cell cluster can be in a cell suspension, e.g., a single cell suspension. The term "suspension" as used herein can refer to cell culture conditions in which cells are not attached to a solid support. Cells proliferating in suspension can be stirred while proliferating using apparatus well known to those skilled in the art.

In some cases, the method provided herein does not comprise an active cell sorting process, e.g., flow cytometry. In some cases, a cell cluster as described herein can be an unsorted cell cluster. In some cases, a method provided herein does not rely on an active cell sorting for the enrichment or elimination of a particular type of cells in the first cell cluster. In some cases, a method merely requires dissociating the first cell cluster and culturing the plurality of cells dissociated from the first cell cluster in a medium, thereby allowing formation of a second cell cluster.

In some cases, the method provided herein can be applied to dissociate a cell cluster and reaggregate into a new cluster for more than once. For instance, a first cell cluster can be dissociate and reaggregated to form a second cell cluster according to the method provided herein, and the second cell cluster can be further dissociated and reaggregated to form a third cell cluster, and so on. Reaggregation as provided herein can be performed sequentially to a cell cluster for at least 1, 2, 3, 4, 5, 6, 7, 8, 9, or 10 times.

Cell sorting as described herein can refer to a process of isolating a group of cells from a plurality of cells by relying on differences in cell size, shape (morphology), surface protein expression, endogenous signal protein expression, or any combination thereof. In some cases, cell sorting comprises subjecting the cells to flow cytometry. Flow cytometry can be a laser- or impedance-based, biophysical technology. During flow cytometry, one can suspend cells in a stream of fluid and pass them through an electronic detection apparatus. In one type of flow cytometry, fluorescent-activated cell sorting (FACS), based on one or more parameters of the cells' optical properties (e.g., emission wave length upon laser excitation), one can physically separate and thereby purify cells of interest using flow cytometry. As described herein, an unsorted cell cluster can be cell cluster that formed by a plurality of cells that have not been subject to an active cell sorting process, e.g., flow cytometry. An unsorted cell cluster, in some cases referred to as "reaggregated cell cluster," can be formed by a plurality of cells that are dissociated from an existing cell cluster, and before their reaggregation into the new cell cluster, there can be no active cell sorting process, e.g., flow cytometry or other methods, to isolate one or more particular cell types for the reaggregation as provided herein. In some cases, flow cytometry as discussed herein can be based on one or more signal peptides expressed in the cells. For example, a cell cluster can comprise cells that express a signal peptide (e.g., a fluorescent protein, e.g., green fluorescent protein (GFP) or tdTomato). In some cases, the signal peptide is expressed as an indicator of insulin expression in the cells. For instance, a cell cluster can comprise cell harboring an exogenous nucleic acid sequence coding for GFP under the control of an insulin promoter. The insulin promoter can be an endogenous or exogenous promoter. In some cases, the expression of GFP in these cells can be indicative of insulin expression in said cells. The GFP signal can thus be a marker of a pancreatic β cell. In some cases, cell sorting as described herein can comprise magnetic-activated flow cytometry, where magnetic antibody or other ligand is used to label cells of different types, and the differences in magnetic properties can be used for cell sorting.

The cells dissociated from the first cell cluster can be cultured in a medium for re-aggregating to a second cell cluster. The medium can comprise Connought Medical Research Laboratories 1066 supplemented islet media (CMRLS). In some cases, the suitable culture medium comprises a component of CMRLS (e.g., supplemental zinc). The CMRLS can be supplemented, e.g., with serum (e.g., human serum, human platelet lysate, fetal bovine serum, or serum replacements such as Knockout Serum Replacement).

The medium can comprise one or more compounds that regulate certain signaling pathways in cells. For example, the medium can comprise a thyroid hormone signaling pathway activator, a transforming growth factor β (TGF-β) signaling pathway inhibitor, or both.

The thyroid hormone signaling pathway activator in the medium used herein can be triiodothyronine (T3). In some cases, the thyroid hormone signaling pathway activator can be an analog or derivative of T3. Non-limiting exemplary analogs of T3 include selective and non-selective thyromimetics, TPβ selective agonist-GC-1, GC-24,4-Hydroxy-PCB 106, MB07811, MB07344,3,5-diiodothyropropionic acid (DITPA); the selective TR-β agonist GC-1; 3-Iodothyronamine (T(1)AM) and 3,3',5-triiodothyroacetic acid (Triac) (bioactive metabolites of the hormone thyroxine (T(4)); KB-2115 and KB-141; thyronamines; SKF L-94901; DIBIT; 3'-AC-T2; tetraiodothyroacetic acid (Tetrac) and triiodothyroacetic acid (Triac) (via oxidative deamination and decarboxylation of thyroxine (T4) and triiodothyronine (T3) alanine chain), 3,3',5'-triiodothyronine (rT3) (via T4 and T3 deiodination), 3,3'-diiodothyronine (3,3'-T2) and 3,5-diiodothyronine (T2) (via T4, T3, and rT3 deiodination), and 3-iodothyronamine (T1AM) and thyronamine (T0AM) (via T4 and T3 deiodination and amino acid decarboxylation), as well as for TH structural analogs, such as 3,5,3'-triiodothyropropionic acid (Triprop), 3,5-dibromo-3-pyridazinone-1-thyronine (L-940901), N-[3,5-dimethyl-4-(4'-hydroxy-3'-isopropylphenoxy)-phenyl]-oxamic acid (CGS 23425), 3,5-dimethyl-4-[(4'-hydroxy-3'-isopropylbenzyl)-phenoxy]acetic acid (GC-1), 3,5-dichloro-4-[(4-hydroxy-3-isopropylphenoxy)phenyl]acetic acid (KB-141), and 3,5-diiodothyropropionic acid (DITPA). In some cases, the thyroid hormone signaling pathway activator is a prodrug or prohormone of T3, such as T4 thyroid hormone (e.g., thyroxine or L-3,5,3',5'-tetraiodothyronine). The thyroid hormone signaling pathway activator can also be an iodothyronine composition described in U.S. Pat. No. 7,163,918, which is incorporated by reference herein in its entirety.

The concentration of the thyroid hormone signaling pathway activator in the medium can be in a range suitable for cell aggregation. In some cases, the concentration of the thyroid hormone signaling pathway activator in the medium is from about 0.1 µM to about 10 µM, such as from about 0.5 µM to about 2 µM, from about 0.8 µM to about 1.5 µM, from about 0.9 µM to about 1.5 µM, from about 0.9 µM to about 1.2 µM, or from about 0.9 µM to about 1.2 µM. In some cases, the contraction of the thyroid hormone signaling pathway activator in the medium is at least about 0.1 µM, 0.2 µM, 0.4 µM, 0.8 µM, 0.9 µM, 1 µM, 1.1 µM, 1.2 µM, 1.3 µM, 1.4 µM, 1.5 µM, 2 µM, 3 µM, 4 µM, 5 µM, 6 µM, 7 µM, 8 µM, 9 µM, or 10 µM. In some case, the contraction of the thyroid hormone signaling pathway activator (e.g., T3) in the medium is about 1 µM.

The TGF-β signaling pathway inhibitor used in the medium herein can be an inhibitor of TGF-β receptor type I kinase (TGF-β RI) signaling. The TGF-β signaling pathway inhibitor can be an activin receptor-like kinase-5 (Alk5) inhibitor, e.g., ALK5 inhibitor II (CAS 446859-33-2, an ATP-competitive inhibitor of TGF-β RI kinase, also known as RepSox, IUPAC Name: 2-[5-(6-methylpyridin-2-yl)-1H-pyrazol-4-yl]-1,5-naphthyridine). In some cases, the TGF-β signaling pathway inhibitor is an analog or derivative of ALK5 inhibitor II, including those described in in U.S. Patent Publication Nos. 2012/0021519, 2010/0267731, 2009/0186076, and 2007/0142376, which are incorporated by reference herein in their entireties. In some cases, examples of TGF-β signaling pathway inhibitor that can be used in the medium herein also include D 4476, SB431542, A-83-01, also known as 3-(6-Methyl-2-pyridinyl)-N-phenyl-4-(4-quinolinyl)-lH-p yrazole-l-carbothioamide; 2-(3-(6- Methylpyridin-2-yl)-lH-pyrazol-4-yl)-l, 5-naphthyridine, Wnt3a/BIO, BMP4, GW788388 (- (4-[3-(pyridin-2-yl)-lH-pyrazol-4-yl]pyridm-2-yl}-N-(tetrahydro-2H-pyran-4- yl)benzamide), SMI 6, ΓN- 1 130 (3-((5-(6-methylpyridin-2-yl)-4-(quinoxalin-6-yl)-lH-imidazol-2-yl)methyl)benzamide, GW6604 (2-phenyl-4-(3-pyridin-2-yl-lH-pyrazol-4-yl)pyridine), SB-505124 (2-(5-benzol1,3]dioxol-5-yl-2-tert-butyl-3H- imidazol-4-yl)-6-methylpyridine hydrochloride), SU5416, lerdelimumb (CAT-152), metelimumab (CAT-192), GC-1008, ID1 1, AP-12009, AP-1 1014, LY550410, LY580276, LY364947, LY2109761, SD-208, SM16, NPC-30345, KÏ26894, SB-203580, SD-093, ALX-270-448, EW-7195, SB-525334, FN-1233, SKI2162, Gleevec, 3,5,7,2',4'-pentahydroxyfiavone (Morin), activin-M108A, P144, soluble TBR2-Fc, pyrimidine derivatives and indolinones. Inhibition of the TGF-β/activin pathway can have similar effects. Thus, any inhibitor (e.g., upstream or downstream) of the TGF-β/activin pathway can be used in combination with, or instead of, TGF-β/ALK5 inhibitors as described herein. Exemplary TGF-β /activin pathway inhibitors include, but are not limited to, TGF-β receptor inhibitors, inhibitors of SMAD 2/3 phosphorylation, inhibitors of the interaction of SMAD 2/3 and SMAD 4, and activators/agonists of SMAD 6 and SMAD 7. Furthermore, the categorizations described herein are merely for organizational purposes and one of skill in the art would know that compounds can affect one or more points within a pathway, and thus compounds may function in more than one of the defined categories. TGF-β receptor inhibitors may include any inhibitors of TGF signaling in general or inhibitors specific for TGF-β receptor (e.g., ALK5) inhibitors, which can include antibodies to, dominant negative variants of, and siRNA and antisense nucleic acids that suppress expression of, TGF-β receptors.

The concentration of the TGF-β signaling pathway inhibitor in the medium can be in a range suitable for cell aggregation. In some cases, the concentration of the TGF-β signaling pathway inhibitor in the medium is from about 1 µM to about 50 µM, such as from about 5 µM to about 15 µM, from about 8 µM to about 12 µM, or from about 9 µM to about 11 µM. In some cases, the contraction of the TGF-β signaling pathway inhibitor in the medium is at least about 1 µM, 5 µM, 8 µM, 9 µM, 10 µM, 11 µM, 12 µM, 13 µM, 14 µM, 15 µM, 20 µM, 25 µM, 30 µM, 35 µM, 40 µM, 45 µM, or 50 µM. In some case, the contraction of the TGF-β signaling pathway inhibitor (e.g., Alk5 inhibitor II) in the medium is about 10 µM.

The medium used to culture the cells dissociated from the first cell cluster can be xeno-free. A xeno-free medium for culturing cells and/or cell clusters of originated from an animal can have no product from other animals. In some cases, a xeno-free medium for culturing human cells and/or cell clusters can have no products from any non-human animals. For example, a xeno-free medium for culturing human cells and/or cell clusters can comprise human platelet lysate (PLT) instead of fetal bovine serum (FBS). For example, a medium can comprise from about 1% to about 20%, from about 5% to about 15%, from about 8% to about 12%, from about 9 to about 11% serum. In some cases, medium can comprise about 10% of serum. In some cases, the medium can be free of small molecules and/or FBS. For example, a medium can comprise MCDB131 basal medium supplemented with 2% BSA. In some cases, the medium is serum-free. In some examples, a medium can comprise no exogenous small molecules or signaling pathway agonists or antagonists, such as, growth factor from fibroblast growth factor family (FGF, such as FGF2, FGF8B, FGF 10, or FGF21), Sonic Hedgehog Antagonist (such as Sant1, Sant2, Sant 4, Sant4, Cur61414, forskolin, tomatidine, AY9944, triparanol, cyclopamine, or derivatives thereof), Retinoic Acid Signaling agonist (e.g., retinoic acid, CD1530, AM580, TTHPB, CD437, Ch55, BMS961, AC261066, AC55649, AM80, BMS753, tazarotene, adapalene, or CD2314), inhibitor of Rho-associated, coiled-coil containing protein kinase (ROCK) (e.g., Thiazovivin, Y-27632, Fasudil/HA1077, or 14-1152), activator of protein kinase C (PKC) (e.g., phorbol 12,13-dibutyrate (PDBU), TPB, phorbol 12-myristate 13-acetate, bryostatin 1, or derivatives thereof), antagonist of TGF beta super family (e.g, Alk5 inhibitor II (CAS 446859-33-2), A83-01, SB431542, D4476, GW788388, LY364947, LY580276, SB505124, GW6604, SB-525334, SD-208, SB-505124, or derivatives thereof), inhibitor of Bone Morphogenic Protein (BMP) type 1 receptor (e.g., LDN193189 or derivatives thereof), thyroid hormone signaling pathway activator (e.g., T3 or derivatives thereof), gamma-secretase inhibitor (e.g., XXI, DAPT, or derivatives thereof), activator of TGF-β signaling pathway (e.g., WNT3a or Activin A) growth factor from epidermal growth factor (EGF) family (e.g., betacellulin or EGF), broad kinase (e.g., staurosporine or derivatives thereof), non-essential amino acids, vitamins or antioxidants (e.g., cyclopamine, vitamin D, vitamin C, vitamin A, or derivatives thereof), or other additions like N- acetyl cysteine, zinc sulfate, or heparin. In some cases, the reaggregation medium can comprise no exogenous extracellular matrix molecule. In some cases, the reaggregation medium does not comprise Matrigel^{™}. In some cases, the reaggregation medium does not comprise other extracellular matrix molecules or materials, such as, collagen, gelatin, poly-L-lysine, poly-D-lysine, vitronectin, laminin, fibronectin, PLO laminin, fibrin, thrombin, and RetroNectin and mixtures thereof, for example, or lysed cell membrane preparations.

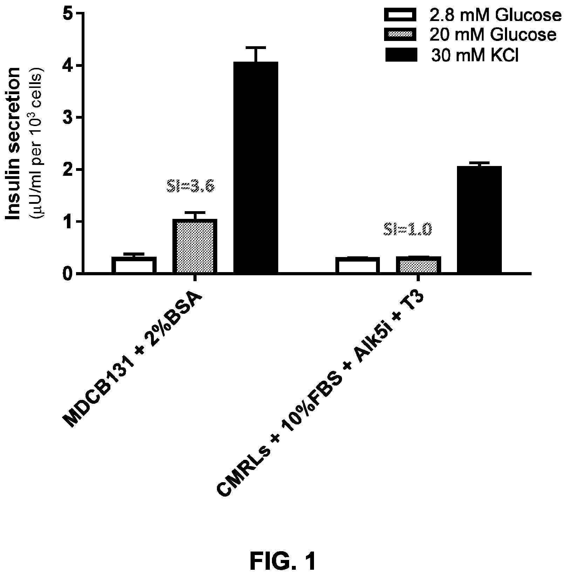

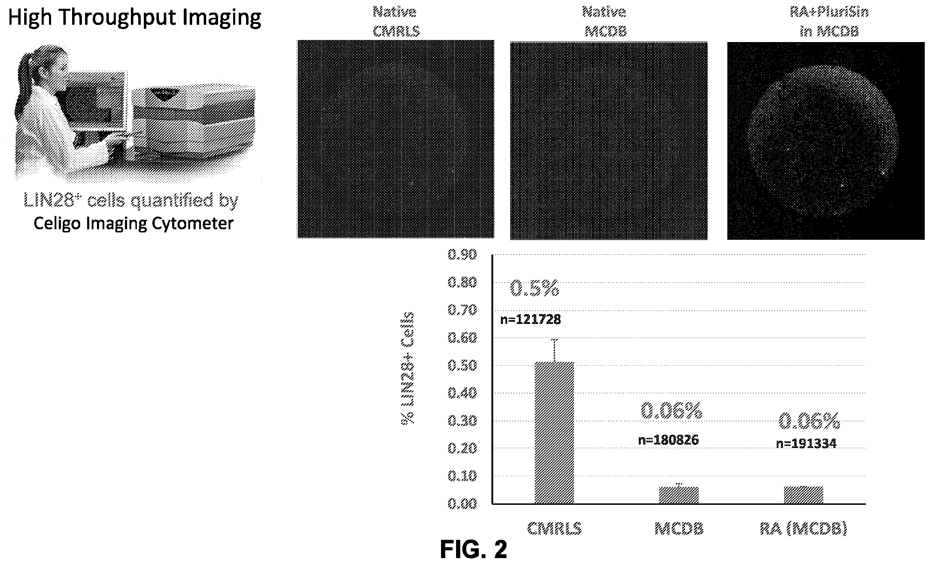

A person of ordinary skill in the art will appreciate that that the concentration of BSA supplemented into the medium may vary. For example, a medium (e.g., MCDB131) can comprise about 0.01%, 0.05%, 0.1%, 1%, about 2%, about 3%, about 4%, about 5%, about 10%, or about 15% BSA. The medium used (e.g., MCDB131 medium) can contain components not found in traditional basal media, such as trace elements, putrescine, adenine, thymidine, and higher levels of some amino acids and vitamins. These additions can allow the medium to be supplemented with very low levels of serum or defined components. The medium can be free of proteins and/or growth factors, and may be supplemented with EGF, hydrocortisone, and/or glutamine. In some cases, the composition of the medium may be determined empirically. For example, as shown in **FIG. 1****,** Stage 6 culture medium was determined based on glucose-stimulated insulin response of cells *in vitro.* Cells were exposed to various concentrations of Glucose or KCL (as a positive control), and insulin secretion was determined for medium compositions with and without small molecules. Cells cultured in MCDB131+2% BSA exhibited a greater glucose-stimulated insulin secretion as compared to cells cultured in CMRLs + 10% FBS + Alk5i + T3. In some cases cell culture medium composition may be determined by staining cells cultured in different media with cell-specific markers. Generally, any marker corresponding to a cell desired in the final population or a cell that is not desired in the final population may be used as a marker to empirically determine the optimal medium to culture cells. In one example, as shown in **FIG. 2****,** cells were stained for LIN28, and counted using a high throughput imaging technique (e.g., image cytometry). LIN28 is a marker of undifferentiated stem cells, and can enhance the efficiency of the formation of induced pluripotent stem cells (iPS) cells from fibroblasts. Culturing native cells and re-aggregated cells in MCDB131 medium resulted in approximately a 90% reduction in LIN28 positive cells (e.g., undifferentiated cells), as compared to native cells cultured in CMRLs medium. A person having ordinary skill in the art will appreciate that any cell marker may be used (e.g., SOX2, SOX9 and/or Ki67).

The medium can comprise one or more extracellular matrix molecules (e.g., extracellular proteins). Non-limiting exemplary extracellular matrix molecules used in the medium can include collagen, placental matrix, fibronectin, laminin, merosin, tenascin, heparin, heparin sulfate, chondroitin sulfate, dermatan sulfate, aggrecan, biglycan, thrombospondin, vitronectin, and decorin. In some cases, the medium comprises laminin, such as LN-332. In some cases, the medium comprises heparin.

The medium can be changed periodically in the culture, e.g., to provide optimal environment for the cells in the medium. When culturing the cells dissociated from the first cell cluster for re-aggregation, the medium can be changed at least or about every 4 hours, 12 hours, 24 hours, 48 hours, 3 days or 4 days. For example, the medium can be changed about every 48 hours.

Cells dissociated from the first cell cluster can be seeded in a container for re-aggregation. The seeding density can correlate with the size of the re-aggregated second cell cluster. The seeding density can be controlled so that the size of the second cell cluster can be similar to an endogenous pancreatic islet. In some cases, the seeding density is controlled so that the size of the second cell cluster can be from about 75 µm to about 250 µm. Cells dissociated from the first cell cluster can be seeded at a density of from about 0.1 million cells per mL to about 10 million cells per mL, e.g., from about 0.5 million cells per mL to about 1.5 million cells per mL, from about 0.8 million cells per mL to about 1.2 million cells per mL, from about 0.9 million cells per mL to about 1.1 million cells per mL, from about 2 million cells per mL to about 3 million cells per mL. In some cases, the cells dissociated from the first cell cluster can be seeded at a density of about 1 million cells per mL. In some cases, the cells dissociated from the first cell cluster can be seeded at a density of about 1.5 million cells per mL. In some cases, the cells dissociated from the first cell cluster can be seeded at a density of about 2 million cells per mL. In some cases, the cells dissociated from the first cell cluster can be seeded at a density of about 2.5 million cells per mL. In some cases, the cells dissociated from the first cell cluster can be seeded at a density of about 3 million cells per mL.

The cell dissociated from the first cell cluster can be cultured in a culture vessel. The culture vessel can be suitable for culturing a suspension of culture of cells. The culture vessel used for culturing the cells or cell clusters herein can include, but is not limited to: flask, flask for tissue culture, dish, petri dish, dish for tissue culture, multi dish, micro plate, micro-well plate, multi plate, multi-well plate, micro slide, chamber slide, tube, tray, culture bag, and roller bottle, stir tank bioreactors, or polymer (e.g., biopolymer or gel) encapsulation as long as it is capable of culturing the cells therein. The cells and/or cell clusters can be cultured in a volume of at least or about 0.2 ml, 0.5 ml, 1 ml, 5 ml, 10 ml, 20 ml, 30 ml, 40 ml, 50 ml, 100 ml, 150 ml, 200 ml, 250 ml, 300 ml, 350 ml, 400 ml, 450 ml, 500 ml, 600 ml, 800 ml, 1000 ml, 1500 ml, 2000 ml, 3000ml or any range derivable therein, depending on the needs of the culture.

In some cases, cells can be cultured under dynamic conditions (e.g., under conditions in which the cells are subject to constant movement or stirring while in the suspension culture). For dynamic culturing of cells, the cells can be cultured in a container (e.g., an non-adhesive container such as a spinner flask (e.g., of 200 ml to 3000 ml, for example 250 ml; of 100 ml; or in 125 ml Erlenmeyer), which can be connected to a control unit and thus present a controlled culturing system. In some cases, cells can be cultured under non-dynamic conditions (e.g., a static culture) while preserving their proliferative capacity. For non-dynamic culturing of cells, the cells can be cultured in an adherent culture vessel. An adhesive culture vessel can be coated with any of substrates for cell adhesion such as extracellular matrix (ECM) to improve the adhesiveness of the vessel surface to the cells. The substrate for cell adhesion can be any material intended to attach stem cells or feeder cells (if used). The substrate for cell adhesion includes collagen, gelatin, poly-L-lysine, poly-D-lysine, vitronectin, laminin, fibronectin, PLO laminin, fibrin, thrombin, and RetroNectin and mixtures thereof, for example, Matrigel^{™}, and lysed cell membrane preparations.

Medium in a dynamic cell culture vessel (e.g., a spinner flask) can be stirred (e.g., by a stirrer). The spinning speed can correlate with the size of the re-aggregated second cell cluster. The spinning speed can be controlled so that the size of the second cell cluster can be similar to an endogenous pancreatic islet. In some cases, the spinning speed is controlled so that the size of the second cell cluster can be from about 75 µm to about 250 µm. The spinning speed of a dynamic cell culture vessel (e.g., a spinner flask) can be about 20 rounds per minute (rpm) to about 100 rpm, e.g., from about 30 rpm to about 90 rpm, from about 40 rpm to about 60 rpm, from about 45 rpm to about 50 rpm. In some cases, the spinning speed can be about 50 rpm.

The cells dissociated from the first cell cluster can be cultured for a period of time to allow them for re-aggregating. The cells dissociated from the first cell cluster can be cultured for at least 12 hours, 24 hours, 1 day, 2 days, 3 days, 4 days, 5 days, 6 days, 7 days 8 days, 9 days 10 days, 15 days, 20 days, 25 days, or 30 days. In some cases, the cells dissociated from the first cell cluster can be cultured for at least 4 days.

The methods herein can also be used to enrich cells resembling endogenous cells, e.g., endogenous mature pancreatic β cells in a cell cluster. The methods can comprise dissociating a first cell cluster and re-aggregating the cells from the first cluster to a second cluster. The second cluster can comprise more cells resembling endogenous mature pancreatic β cells compared to the first cluster. The dissociating and re-aggregating can be performed using any methods and reagents disclosed through the application.

After re-aggregation, the second cell cluster can comprise more cells expressing one or more markers of an endogenous cell compared to the first cell cluster. For example, the second cluster can comprise more cells expressing one or more markers of an endogenous mature pancreatic β cell, the markers including insulin, C-peptide, PDX1, NKX6.1, CHGA, MAFA, ZNT8, PAX6, NEUROD1, glucokinase (GCK), SLC2A, PCSK1, KCNJ11, ABCC8, SLC30A8, SNAP25, RAB3A, GAD2, and PTPRN, compared to the first cell cluster. In some cases, the second cluster can comprise more cells expressing CHGA. In some cases, the second cluster can comprise more cells expressing NKX6.1. In some cases, the second cluster can comprise more cells expressing C-peptide. In some cases, the second cluster can comprise more cells expressing NKX6.1 and C-peptide. In some cases, the second cluster can comprise more cells expressing CHGA, NKX6.1 and C-peptide.

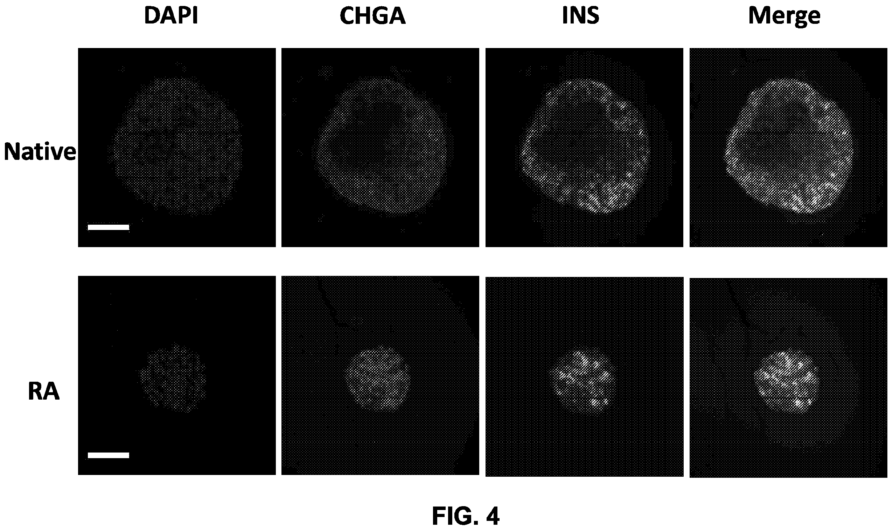

After re-aggregation, the second cell cluster can have a smaller size (e.g., a smaller diameter) compared to the first cell cluster. For example, as shown in **FIG. 4****,** immunofluorescence images of native cell clusters (top) and re-aggregated (RA) cell clusters (bottom) stained for endocrine cell markers (e.g., CHGA, INS, and DAPI) show a decrease in a cluster diameter in RA cells, as compared to native cell clusters. The smaller size can allow better exchange of molecules between the cell cluster and the surrounding environment. For example, a smaller size can allow better diffusion of molecules (e.g., reagents, gas, and/or nutrition) from the medium to the cells in a cell cluster. Thus, being in a smaller size, the second cell cluster can exchange molecules with the surrounding environment in a more efficient way compared to the first cell cluster. Thus the second cell cluster can have less dead cells (e.g., cells died due to insufficient nutrition and/or gas) compared to the first cell cluster.

A method provided herein can enrich endocrine cells, e.g., cells expressing chromogranin A (CHGA). For examples, a percentage of cells in the second cell cluster that express chromogranin A is at least 1.2, at least 1.3, at least 1.4, or at least 1.5 times more than a percentage of cells in the first cell cluster that express chromogranin A. In some cases, the second cell cluster comprises at least about 70%, at least about 80%, at least about 90%, at least about 95%, at least about 99%, or 100% cells expressing CHGA. In some cases, at least about 85% cells in the second cell cluster can express CHGA. In some cases, the second cell cluster can comprise about 90% cell expressing CHGA. In some cases, the second cell cluster can comprise about 95% cells expressing CHGA. In certain cases, all cells in the second cell cluster can express CHGA.

A method provided herein can generate or enrich pancreatic β cell. For example, the second cell cluster comprises at least one pancreatic β cell, e.g., at least one non-native pancreatic β cell. For examples, a percentage of cells in the second cell cluster that express both NKX6.1 and C-peptide is at least 1.5, at least 1.75, or at least 2 times more than a percentage of cells in the first cell cluster that express both NKX6.1 and C-peptide. In some cases, the second cell cluster comprises at least about 35%, at least about 40%, at least about 50%, at least about 60%, at least about 70%, at least about 80%, at least about 90%, at least about 95%, at least about 99%, or 100% cells expressing NKX6.1 and C-peptide. In some cases, at least about 35% cells in the second cell cluster can express NKX6.1 and C-peptide. In some cases, a cell cluster can comprise about 60% cells expressing NKX6.1 and C-peptide. In some cases, the second cell cluster can comprise about 75% cell expressing NKX6.1 and C-peptide. In some cases, all cells in the second cell cluster can express NKX6.1 and C-peptide. In some cases, at least about 70% of the at least one non-native pancreatic β cell in the second cell cluster express chromogranin A as measured by flow cytometry. In some cases, at least about 25% of the at least one non-native pancreatic β cell in the second cell cluster express NKX6.1 and C-peptide as measured by flow cytometry.

A method provided herein can reduce or eliminate stem cells or precursor cells of an pancreatic endocrine cells. In some cases, a percentage of cells in the second cell cluster that express SOX2 is at least 2, at least 3, at least 5, or at least 10 times lower than a percentage of cells in the first cell cluster that express LIN28, Ki67, SOX2, or SOX9. For example, the second cell cluster can comprise at most about 5% cells, at most about 5% cells, at most about 5% cells, at most about 5% cells, at most about 5% cells, at most about 2% cells, at most about 1% cells, at most about 0.5% cells, at most about 0.1% cells, at most about 0.05% cells, at most about 0.01% cells, or no cells expressing LIN28. In some examples, the second cell cluster as provided herein can comprise at most about 5% cells, at most about 5% cells, at most about 5% cells, at most about 5% cells, at most about 5% cells, at most about 2% cells, at most about 1% cells, at most about 0.5% cells, at most about 0.1% cells, at most about 0.05% cells, at most about 0.01% cells, or no cells expressing Ki67. For example, the second cell cluster can comprise at most 3% cells, at most about 2% cells, at most about 1% cells, at most about 0.5% cells, at most about 0.1% cells, at most about 0.05% cells, at most about 0.01% cells, or no cells expressing SOX2. In some cases, the second cell cluster can comprise about 1% cells expressing SOX2. In some cases, the second cell cluster can comprise about 0.6% cells expressing SOX2. In some cases, the second cell cluster can comprise about 0.3% cells expressing SOX2. In some cases, the second cell cluster can comprise about 0.1% cells expressing SOX2. For examples, the second cell cluster can comprise at most 10% cells, at most about 8% cells, at most about 6% cells, at most about 5% cells, at most about 2% cells, at most about 1% cells, at most about 0.5% cells, at most about 0.1% cells, at most about 0.05% cells, at most about 0.01% cells, or no cells expressing SOX9. In some cases, the second cell cluster can comprise about 2% cells expressing SOX9. In some cases, the second cell cluster can comprise about 6% cells expressing SOX9. In some cases, the second cell cluster can comprise about 1.2% cells expressing SOX9.

The second cell cluster can also function more similarly to an endogenous pancreatic islet compared to the first cell cluster. The second cell cluster can have a higher insulin content than the first cell cluster, for instance, at least 1.1, at least 1.25 or at least 1.5 times higher insulin content as compared to the first cell cluster. The second cluster can exhibit a greater *in vitro* GSIS than the first cell cluster, as measured by stimulation indexes. The second cluster can also exhibit a greater *in vivo* GSIS than the first cell cluster, as measured by stimulation indexes. In some cases, the second cluster can exhibit a greater *in vitro* GSIS and a greater *in vivo* GSIS compared to the first cell cluster, as measured by stimulation indexes. For example, the second cell cluster can secrete more insulin than the first cell cluster under the same stimulation conditions. The second cell cluster can also exhibit insulin secretion response to a potassium challenge (K⁺), e.g., a concentration of KCl, e.g., 30 mM KCl.

In some cases, the method provided herein can retain a large percentage of cells from the first cell cluster in the second cell cluster, e.g., pancreatic β cells or endocrine cells. For example, at least about 95%, at least about 98%, or at least about 99% of cells that express both NKX6.1 and C-peptide in the first cell cluster can be retained in the second *in vitro* cell cluster. In some cases, at most about 5%, at most about 2%, at most about 1%, at most about 0.5%, or at most about 0.1% of cells that express both NKX6.1 and C-peptide in the first cell cluster are lost during the dissociation and reaggregation process.

In some cases, the cell cluster as described herein is generated from any starting cell population *in vitro.* For example, the starting cell can include, without limitation, insulin-positive endocrine cells (e.g., chromogranina A-positive cells) or any precursor thereof, such as a Nkx6.1-positive pancreatic progenitor cell, a Pdx1-positive pancreatic progenitor cell, and a pluripotent stem cell, an embryonic stem cell, and induced pluripotent stern cell. In some cases, the method include differentiation of a reprogrammed cell, a partially reprogrammed cell (e.g., a somatic cell, e.g., a fibroblast which has been partially reprogrammed such that it exists in an intermediate state between an induced pluripotency cell and the somatic cell from which it has been derived), a transdifferentiated cell. In some cases, the cell cluster comprising the pancreatic β cell disclosed herein can be differentiated *in vitro* from an insulin-positive endocrine cell or a precursor thereof. In some cases, the cell cluster comprising the pancreatic β cell is differentiated *in vitro* from a precursor selected from the group consisting of a NKX6.1-positive pancreatic progenitor cell, a Pdx1-positive pancreatic progenitor cell, and a pluripotent stem cell. In some cases, the pluripotent stem cell is selected from the group consisting of an embryonic stem cell and induced pluripotent stem cell. As discussed above, the non-native pancreatic β cells can also be referred to as stem cell-derived β cells (SC-β cells) as they can be derived from stem cells *in vitro.* In some cases, the SC-β cell or the pluripotent stem cell from which the SC-β cell is derived is human. In some cases, the SC-β cell is human.

One aspect of the present disclosure provides a method of generating non-native pancreatic β cells. In some cases, the method can be any currently available protocol, such as those described in U.S. Patent Application Nos. 14/684,129 and 14/684,101, each of which is incorporated herein by its entirety. Aspects of the disclosure involve definitive endoderm cells, Definitive endoderm cells of use herein can be derived from any source or generated in accordance with any suitable protocol, In some aspects, pluripotent stem cells, e.g., iPSCs or hESCs, are differentiated to endoderm cells. In some aspects, the endoderm cells (stage 1) are further differentiated, e.g., to primitive gut tube cells (stage 2), Pdx1-positive pancreatic progenitor cells (stage 3), NKX6.1-positive pancreatic progenitor cells (stage 4), or Ngn3-positive endocrine progenitor cells or insulin-positive endocrine cells (stage 5), followed by induction or maturation to SC-β cells (stage 6).

In some cases, definitive endoderm cells can be obtained by differentiating at least some pluripotent cells in a population into definitive endoderm cells, e.g., by contacting a population of pluripotent cells with i) at least one growth factor from the TGF-β superfamily, and ii) a WNT signaling pathway activator, to induce the differentiation of at least some of the pluripotent cells into definitive endoderm cells, wherein the definitive endoderm cells express at least one marker characteristic of definitive endoderm.

Any growth factor from the TGF-β superfamily capable of inducing the pluripotent stem cells to differentiate into definitive endoderm cells (e.g., alone, or in combination with a WNT signaling pathway activator) can be used in the method provided herein. In some cases, the at least one growth factor from the TGF-β superfamily comprises Activin A. In some cases, the at least one growth factor from the TGF-β superfamily comprises growth differentiating factor 8 (GDF8). Any WNT signaling pathway activator capable of inducing the pluripotent stem cells to differentiate into definitive endoderm cells (e.g., alone, or in combination with a growth factor from the TGF-β superfamily) can be used in the method provided herein. In some cases, the WNT signaling pathway activator comprises CHIR99Q21 . In some cases, the WNT signaling pathway activator comprises Wnt3a recombinant protein.

In some cases, differentiating at least some pluripotent cells in a population into definitive endoderm cells is achieved by a process of contacting a population of pluripotent cells with i) Activin A, and ii) CHIR99021 for a period of 3 days, to induce the differentiation of at least some of the pluripotent cells in the population into definitive endoderm cells, wherein the definitive endoderm cells express at least one marker characteristic of definitive endoderm.

In some cases, a definitive endoderm cell produced by the methods as disclosed herein expresses at least one marker selected from the group consisting of: Nodal, Tmprss2, Tmem30b, St14, Spink3, Sh3gl2, Ripk4, Rab l S, Npnt, Clic6, CldnS, Cacna l b, Bnipl, Anxa4, Emb, FoxA 1, Sox 17, and Rbm35a, wherein the expression of at least one marker is upregulated to by a statistically significant amount in the definitive endoderm cell relative to the pluripotent stem cell from which it was derived. In some cases, a definitive endoderm cell produced by the methods as disclosed herein does not express by a statistically significant amount at least one marker selected the group consisting of: Gata4, SPARC, AFP and Dab2 relative to the pluripotent stem cell from which it was derived. In some cases, a definitive endoderm cell produced by the methods as disclosed herein does not express by a statistically significant amount at least one marker selected the group consisting of: Zicl, Pax6, Flk l and CD3 1 relative to the pluripotent stem cell from which it was derived. In some cases, a definitive endoderm cell produced by the methods as disclosed herein has a higher level of phosphorylation of Smad2 by a statistically significant amount relative to the pluripotent stem cell from which it was derived. In some cases, a definitive endoderm cell produced by the methods as disclosed herein has the capacity to form gut tube *in vivo.* In some cases, a definitive endoderm cell produced by the methods as disclosed herein can diferentiate into a cell with morphology characteristic of a gut cell, and wherein a cell with morphology characteristic of a gut cell expresses FoxA2 and/or Claudin6, In some cases, a definitive endoderm cell produced by the methods as disclosed herein can be further differentiated into a cell of endoderm origin.

In some cases, a population of pluripotent stem cells are cultured in the presence of at least one β cell maturation factor prior to any differentiation or during the first stage of differentiation. One can use any pluripotent stem cell, such as a human pluripotent stem cell, or a human iPS cell or any of pluripotent stem cell as discussed herein or other suitable pluripotent stem cells. In some cases, a β cell maturation factor as described herein can be present in the culture medium of a population of pluripotent stem cells or may be added in bolus or periodically during growth (e.g. replication or propagation) of the population of pluripotent stem cells. In certain examples, a population of pluripotent stem cells can be exposed to at least one β cell maturation factor prior to any differentiation. In other examples, a population of pluripotent stem cells may be exposed to at least one β cell maturation factor during the first stage of differentiation.

Aspects of the disclosure involve primitive gut tube cells. Primitive gut tube cells of use herein can be derived from any source or generated in accordance with any suitable protocol . In some aspects, definitive endoderm cells are differentiated to primitive gut tube cells. In some aspects, the primitive gut tube cells are further differentiated, e.g., to Pdx1-positive pancreatic progenitor cells, NKX6.1-positive pancreatic progenitor cells, Ngn3-positive endocrine progenitor cells, insulin-positive endocrine cells, followed by induction or maturation to SC-β cells.

In some cases, primitive gut tube cells can be obtained by differentiating at least some definitive endoderm cells in a population into primitive gut tube cells, e.g., by contacting definitive endoderm cells with at least one growth factor from the fibroblast growth factor (FGF) family, to induce the differentiation of at least some of the definitive endoderm cells into primitive gut tube cells, wherein the primitive gut tube cells express at least one marker characteristic of primitive gut tube cells.

Any growth factor from the FGF family capable of inducing definitive endoderm cells to differentiate into primitive gut tube cells (e.g., alone, or in combination with other factors) can be used in the method provided herein. In some cases, the at least one growth factor from the FGF family comprises keratinocyte growth factor (KGF). In some cases, the at least one growth factor from the FGF family comprises FGF2. In some cases, the at least one growth factor from the FGF family comprises FGF8B. In some cases, the at least one growth factor from the FGF family comprises FGF 10. In some cases, the at least one growth factor from the FGF family comprises FGF21.

In some cases, primitive gut tube cells can be obtained by differentiating at least some definitive endoderm cells in a population into primitive gut tube cells, e.g., by contacting definitive endoderm cells with KGF for a period of 2 days, to induce the differentiation of at least some of the definitive endoderm cells into primitive gut tube cells.

Aspects of the disclosure involve Pdxl-positive pancreatic progenitor cells. Pdxl-positive pancreatic progenitor cells of use herein can be derived from any source or generated in accordance with any suitable protocol. In some aspects, primitive gut tube cells are differentiated to Pdx1 -positive pancreatic progenitor cells. In some aspects, the Pdxl -positive pancreatic progenitor cells are further differentiated, e.g., NKX6.1 -positive pancreatic progenitor cells, Ngn3-positive endocrine progenitor cells, insulin-positive endocrine cells, followed by induction or maturation to SC-β cells,

In some aspects, Pdxl-positive pancreatic progenitor cells can be obtained by differentiating at least some primitive gut tube cells in a population into Pdxl-positive pancreatic progenitor cells, e.g., by contacting primitive gut tube cells with i) at least one bone morphogenic protein (BMP) signaling pathway inhibitor, ii) at least one growth factor from the FGF family, in) at least one SHH pathway inhibitor, iv) at least one retinoic acid (RA) signaling pathway activator; and v) at least one protein kinase C activator, to induce the differentiation of at least some of the primitive gut tube cells into Pdxl-positive pancreatic progenitor cells, wherein the Pdxl-positive pancreatic progenitor cells express Pdxl. In some cases, Pdxl-positive pancreatic progenitor cells can be obtained by differentiating at least some primitive gut tube cells in a population into Pdxl-positive pancreatic progenitor cells, e.g., by contacting primitive gut tube cells with i) at least one growth factor from the FGF family, and ii) at least one retinoic acid (RA) signaling pathway activator, to induce the differentiation of at least some of the primitive gut tube cells into Pdxl-positive pancreatic progenitor cells, wherein the Pdxl -positive pancreatic progenitor cells express Pdxl.

Any BMP signaling pathway inhibitor capable of inducing primitive gut tube cells to differentiate into Pdxl-positive pancreatic progenitor cells (e.g., alone, or with any combination of at least one growth factor from the FGF family, at least one SHH pathway inhibitor, at least one retinoic acid signaling pathway activator, and at least one protein kinase C activator) can be used in the method provided herein. In some cases, the BMP signaling pathway inhibitor comprises LDN193189.

Any growth factor from the FGF family capable of inducing primitive gut tube cells to differentiate into Pdxl-positive pancreatic progenitor cells (e.g., alone, or with any combination of at least one BMP signaling pathway inhibitor, at least one SHH pathway inhibitor, at least one retinoic acid signaling pathway activator, and at least one protein kinase C activator) can be used. In some cases, the at least one growth factor from the FGF family comprises keratinocyte growth factor (KGF). In some cases, the at least one growth factor from the FGF family is selected from the group consisting of FGF2, FGF8B, FGF 1 0, and FGF21.

Any SHH pathway inhibitor capable of inducing primitive gut tube cells to differentiate into Pdxl-positive pancreatic progenitor cells (e.g., alone, or with any combination of at least one BMP signaling pathway inhibitor, at least one growth factor from the FGF family, at least one retinoic acid signaling pathway activator, and at least one protein kinase C activator) can be used. In some cases, the SHH pathway inhibitor comprises Sant l.

Any RA signaling pathway activator capable of inducing primitive gut tube cells to differentiate into Pdxl-positive pancreatic progenitor cells (e.g., alone, or with any combination of at least one BMP signaling pathway inhibitor, at least one growth factor from the FGF family, at least one SHH pathway inhibitor, and at least one protein kinase C activator) can be used. In some cases, the RA signaling pathway activator comprises retinoic acid.

Any PKC activator capable of inducing primitive gut tube cells to differentiate into Pdxl-positive pancreatic progenitor cells (e.g., alone, or with any combination of at least one BMP signaling pathway inhibitor, at least one growth factor from the FGF family, at least one SHH pathway inhibitor, and at least one RA signaling pathway activator) can be used. In some cases, the PKC activator comprises PdbU. In some cases, the PKC activator comprises TPB.

In some cases, Pdxl-positive pancreatic progenitor cells can be obtained by differentiating at least some primitive gut tube cells in a population into Pdxl-positive pancreatic progenitor cells, e.g., by contacting primitive gut tube cells with retinoic acid, KGF, Sant1, LDN193189, PdBU for a period of 2 days. In some cases, Pdxl-positive pancreatic progenitor cells can be obtained by differentiating at least some primitive gut tube cells in a population into Pdxl-positive pancreatic progenitor cells, e.g., by contacting primitive gut tube cells with retinoic acid and KGF for a period of 2 days. In some cases, Pdxl-positive pancreatic progenitor cells can be obtained by differentiating at least some primitive gut tube cells in S3 medium

Aspects of the disclosure involve NKX6.1-positive pancreatic progenitor cells. NKX6.1-positive pancreatic progenitor cells of use herein can be derived from any source or generated in accordance with any suitable protocol. In some aspects, Pdxl-positive pancreatic progenitor cells are differentiated to NKX6.1-positive pancreatic progenitor cells. In some aspects, the NKX6.1-positive pancreatic progenitor cells are further differentiated, e.g., to Ngn3-positive endocrine progenitor cells, or insulin-positive endocrine cells, followed by induction or maturation to SC-β cells.

In some aspects, a method of producing a NKX6.1-positive pancreatic progenitor cell from a Pdx I-positive pancreatic progenitor cell comprises contacting a population of cells (e.g., under conditions that promote cell clustering) comprising Pdx1-positive pancreatic progenitor cells with at least two β cell-maturation factors comprising a) at least one growth factor from the fibroblast growth factor (FGF) family, b) a sonic hedgehog pathway inhibitor, and optionally c) a low concentration of a retinoic acid (RA) signaling pathway activator, to induce the differentiation of at least one Pdxl-positive pancreatic progenitor cell in the population into NKX6.1-positive pancreatic progenitor cells, wherein the NKX6.1-positive pancreatic progenitor cells expresses NKX6.1.

In some cases, the Pdxl-positive, NKX6.1-positive pancreatic progenitor cells are obtained by contacting Pdxl-positive pancreatic progenitor cells under conditions that promote cell clustering with i) at least one growth factor from the FGF family, ii) at least one SHH pathway inhibitor, and optionally iii) low concentrations of a RA signaling pathway activator, to induce the differentiation of at least some of the Pdxl-positive pancreatic progenitor cells into Pdxl-positive, NKX6.1-positive pancreatic progenitor cells, wherein the Pdxl-positive, NKX6.1-positive pancreatic progenitor cells expresses Pdxl and NKX6.1. In some cases, the Pdxl-positive, NKX6.1-positive pancreatic progenitor cells are obtained by contacting Pdxl-positive pancreatic progenitor cells under conditions that promote cell clustering with i) at least one growth factor from the FGF family, ii) at least one SHH pathway inhibitor, and optionally iii) low concentrations of a RA signaling pathway activator, iv) ROCK inhibitor, and v) at least one growth factor from the TGF-β superfamily, to induce the differentiation of at least some of the Pdxl-positive pancreatic progenitor cells into Pdxl-positive, NKX6.1-positive pancreatic progenitor cells. In some cases, the Pdx1-positive, NKX6.1-positive pancreatic progenitor cells are obtained by contacting Pdxl-positive pancreatic progenitor cells under conditions that promote cell clustering with at least one growth factor from the FGF family.

In some cases, the Pdxl-positive pancreatic progenitor cells are produced from a population of pluripotent cells, In some cases, the Pdxl-positive pancreatic progenitor cells are produced from a population of iPS cells. In some cases, the Pdxl-positive pancreatic progenitor cells are produced from a population of ESC cells. In some cases, the Pdxl-positive pancreatic progenitor cells are produced from a population of definitive endoderm cells. In some cases, the Pdxl-positive pancreatic progenitor cells are produced from a population of primitive gut tube cells.

Any growth factor from the FGF family capable of inducing Pdxl-positive pancreatic-progenitor cells to differentiate into NKX6.1-positive pancreatic progenitor cells (e.g., alone, or with any combination of at least one SHH pathway inhibitor, or optionally at least one retinoic acid signaling pathway activator) can be used in the method provided herein. In some cases, the at least one growth factor from the FGF family comprises keratinocyte growth factor (KGF). In some cases, the at least one growth factor from the FGF family is selected from the group consisting of FGF2, FGF8B, FGF 10, and FGF21.

Any SHH pathway inhibitor capable of inducing Pdxl-positive pancreatic progenitor cells to differentiate into NKX6.1-positive pancreatic progenitor cells (e.g., alone, or with any combination of at least one growth factor from the FGF family, at least one retinoic acid signaling pathway activator, ROCK inhibitor, and at least one growth factor from the TGF-β superfamily) can be used in the method provided herein. In some cases, the SHH pathway inhibitor comprises Sant l.

Any RA signaling pathway activator capable of inducing Pdx1-positive pancreatic progenitor cells to differentiate into NKX6.1-positive pancreatic progenitor cells (e.g., alone, or with any combination of at least one growth factor from the FGF family, at least one SHH pathway inhibitor, ROCK inhibitor, and at least one growth factor from the TGF-β superfamily) can be used. In some cases, the RA signaling pathway activator comprises retinoic acid.

Any ROCK inhibitor capable of inducing Pdx1-positive pancreatic progenitor cells to differentiate into NKX6.1-positive pancreatic progenitor cells (e.g., alone, or with any combination of at least one growth factor from the FGF family, at least one SHH pathway inhibitor, a RA signaling pathway activator, and at least one growth factor from the TGF-β superfamily) can be used. In some cases, the ROCK inhibitor comprises Thiazovivin, Y-27632, Fasudil/HA1077, or 14-1152.

Any activator from the TGF-β superfamily capable of inducing Pdx1-positive pancreatic progenitor cells to differentiate into NKX6.1-positive pancreatic progenitor cells (e.g., alone, or with any combination of at least one growth factor from the FGF family, at least one SHH pathway inhibitor, a RA signaling pathway activator, and ROCK inhibitor) can be used. In some cases, the activator from the TGF-β superfamily comprises Activin A or GDF8.

In some cases, the Pdxl-positive, NKX6.1-positive pancreatic progenitor cells are obtained by contacting Pdxl-positive pancreatic progenitor cells under conditions that promote cell clustering with KGF, Sant1, and RA, for a period of 5 days. In some cases, the Pdxl-positive, NKX6.1-positive pancreatic progenitor cells are obtained by contacting Pdxl-positive pancreatic progenitor cells under conditions that promote cell clustering with KGF, Sant1, RA, Y27632, and Activin A, for a period of 5 days. In some cases, the Pdxl-positive, NKX6.1-positive pancreatic progenitor cells are obtained by contacting Pdxl-positive pancreatic progenitor cells under conditions that promote cell clustering with KGF for a period of 5 days. In some cases, the Pdxl-positive, NKX6.1-positive pancreatic progenitor cells are obtained by contacting Pdxl-positive pancreatic progenitor cells in a S3 medium.

Aspects of the disclosure involve insulin-positive endocrine cells. Insulin-positive endocrine cells of use herein can be derived from any source or generated in accordance with any suitable protocol, In some aspects, NKX6.1-positive pancreatic progenitor cells are differentiated to insulin-positive endocrine cells, In some aspects, the insulin-positive endocrine cells are further differentiated, e.g., by induction or maturation to SC-β cells.

In some aspects, a method of producing an insulin-positive endocrine cell from an NKX6.1-positive pancreatic progenitor cell comprises contacting a population of cells (e.g., under conditions that promote cell clustering) comprising NKX6-l-positive pancreatic progenitor cells with a) a TGF-β signaling pathway inhibitor, and b) a thyroid hormone signaling pathway activator, to induce the differentiation of at least one NKX6.1-positive pancreatic progenitor cell in the population into an insulin-positive endocrine cell, wherein the insulin-positive endocrine ceil expresses insulin. In some cases, insulin-positive endocrine cells express Pdxl, NKX6.1, NKX2.2, Mafb, glis3, Sur l, Kir6.2, Znt8, SLC2A1, SLC2A3 and/or insulin.

Any TGF-β signaling pathway inhibitor capable of inducing the differentiation of NKX6.1-positive pancreatic progenitor cells to differentiate into insulin-positive endocrine cells (e.g., alone, or in combination with other β cell-maturation factors, e.g., a thyroid hormone signaling pathway activator) can be used. In some cases, the TGF-β signaling pathway comprises TGF-β receptor type I kinase signaling. In some cases, the TGF-β signaling pathway inhibitor comprises Alk5 inhibitor II.

Any thyroid hormone signaling pathway activator capable of inducing the differentiation of NKX6.1-positive pancreatic progenitor cells to differentiate into insulin-positive endocrine cells (e.g., alone, or in combination with other β cell-maturation factors, e.g., a TGF-β signaling pathway inhibitor) can be used. In some cases, the thyroid hormone signaling pathway activator comprises triiodothyronine (T3).

In some cases, the method comprises contacting the population of cells (e.g., NKX6.1-positive pancreatic progenitor cells) with at least one additional factor. In some cases, the method comprises contacting the Pdx1-positive NKX6.1-positive pancreatic progenitor cells with at least one of i) a SHH pathway inhibitor, ii) a RA signaling pathway activator, iii) a γ-secretase inhibitor, iv) at least one growth factor from the epidermal growth factor (EGF) family, and optionally v) a protein kinase inhibitor.

In some cases, the method comprises contacting the population of cells (e.g., NKX6.1-positive pancreatic progenitor cells) with at least one additional factor. In some cases, the method comprises contacting the Pdx1-positive NKX6.1-positive pancreatic progenitor cells with at least one of i) a SHH pathway inhibitor, ii) a RA signaling pathway activator, iii) a γ-secretase inhibitor, iv) at least one growth factor from the epidermal growth factor (EGF) family, and v) at least one bone morphogenic protein (BMP) signaling pathway inhibitor.

Any γ-secretase inhibitor that is capable of inducing the differentiation of NKX6.1-positive pancreatic progenitor cells in a population into insulin-positive endocrine cells (e.g., alone, or in combination with any of a TGF-β signaling pathway inhibitor and/or a thyroid hormone signaling pathway activator). In some cases, the γ-secretase inhibitor comprises XXI. In some cases, the γ-secretase inhibitor comprises DAPT.

Any growth factor from the EGF family capable of inducing the differentiation of NKX6.1-positive pancreatic progenitor cells in a population into insulin-positive endocrine cells (e.g., alone, or in combination with any of a TGF-β signaling pathway inhibitor and/or a thyroid hormone signaling pathway activator) can be used. In some cases, the at least one growth factor from the EG F fami ly comprises betacellulin. In some cases, at least one growth factor from the EGF family comprises EGF.

Any RA signaling pathway activator capable of inducing the differentiation of NKX6.1-positive pancreatic progenitor cells to differentiate into insulin-positive endocrine cells (e.g., alone, or in combination with any of a TGF-β signaling pathway inhibitor and/or a thyroid hormone signaling pathway activator) can be used. In some cases, the RA signaling pathway activator comprises RA.

Any SHH pathway inhibitor capable of inducing the differentiation of NKX6.1-positive pancreatic progenitor cells to differentiate into insulin-positive endocrine cells (e.g., alone, or in combination with any of a TGF-β signaling pathway inhibitor and/or a thyroid hormone signaling pathway activator) can be used in the method provided herein. In some cases, the SHH pathway inhibitor comprises Santl.

Any BMP signaling pathway inhibitor capable of inducing the differentiation of NKX6.1-positive pancreatic progenitor cells to differentiate into insulin-positive endocrine cells (e.g., alone, or in combination with any of a TGF-β signaling pathway inhibitor and/or a thyroid hormone signaling pathway activator) can be used. In some cases, the BMP signaling pathway inhibitor comprises LDN193189.

In some cases, the population of cells is optionally contacted with a protein kinase inhibitor. In some cases, the population of cells is not contacted with the protein kinase inhibitor. In some cases, the population of cells is contacted with the protein kinase inhibitor. Any protein kinase inhibitor that is capable of inducing the differentiation of NKX6.1-positive pancreatic progenitor cells in a population into insul in-positive endocrine cells (e.g., alone, or in combination with any of a TGF-β signaling pathway inhibitor and/or a thyroid hormone signaling pathway activator). In some cases, the protein kinase inhibitor comprises staurosporine.

In some cases, the method comprises contacting the population of cells (e.g., NKX6.1-positive pancreatic progenitor cells) with XXI, Alk5i, T3, RA, Sant1, and betacellulin for a period of 7 days, to induce the differentiation of at least one NKX6.1-positive pancreatic progenitor cell in the population into an insulin-positive endocrine cell, wherein the insulin-positive endocrine cell expresses insulin. In some cases, the method comprises contacting the population of cells (e.g., NKX6.1-positive pancreatic progenitor cells) with XXI, Alk5i, T3, RA, Sant1, betacellulin, and LDN193189 for a period of 7 days, to induce the differentiation of at least one NKX6.1-positive pancreatic progenitor cell in the population into an insulin-positive endocrine cell, wherein the insulin-positive endocrine ceil expresses insulin.

In some cases, the method comprises culturing the population of cells (e.g., NKX6.1-positive pancreatic progenitor cells) in a BE5 medium, to induce the differentiation of at least one NKX6.1-positive pancreatic progenitor cell in the population into an insulin-positive endocrine cell, wherein the insulin-positive endocrine cell expresses insulin.

Aspects of the disclosure involve generating non-native pancreatic β cells which resemble endogenous mature β cells in form and function, but nevertheless are distinct from native β cells.

In some cases, the insulin-positive pancreatic endocrine cells generated using the method provided herein can form a cell cluster, alone or together with other types of cells, e.g., precursors thereof, e.g., stem cell, definitive endoderm cells, primitive gut tube cell, Pdx1-positive pancreatic progenitor cells, or NKX6.1-positive pancreatic progenitor cells (e.g., stage 6 cells as shown in **FIG. 1**). In some cases, the cell cluster comprising the insulin-positive endocrine cells can be reaggregated using the method provided herein. The reaggregation of the cell cluster can enrich the insulin-positive endocrine cells. In some cases, the insulin-positive endocrine cells in the cell cluster can be further matured into pancreatic β cells. For example, after reaggregation, the second cell cluster can exhibit *in vitro* GSIS, resembling native pancreatic islet. For example, after reaggregation, the second cell cluster can comprise non-native pancreatic β cell that exhibits *in vitro* GSIS.

Stage 6 cells as provided herein may or may not be subject to the dissociation and reaggregation process as described herein. In some cases, the cell population comprising the insulin-positive endocrine cells can be directly induced to mature into Sc-β cells. In some cases, the maturation factors can comprise at least one inhibitor of TGF-β signaling pathway and thyroid hormone signaling pathway activator as described herein. In some cases, Sc-β cells can be obtained by contacting a population of cells comprising insulin-positive endocrine cells with Alk5i and T3. In some cases, the insulin-positive endocrine cells can be matured in a CMRLs medium supplemented with 10% FBS. In other cases, Sc-β cells can be obtained by culturing the population of cells containing the insulin-positive endocrine cells in a MCDB131 medium that can be supplemented by 2% BSA. In some cases, the MCDB131 medium with 2% BSA for maturation of insulin-positive endocrine cells into Sc-β cells can be comprise no small molecule factors as described herein. In some case, the MCDB131 medium with 2% BSA for maturation of insulin-positive endocrine cells into Sc-β cells can comprise no serum (e.g., no FBS).

One aspect of the present disclosure provides a method of cryopreservation. As provided herein, the cell population comprising non-native pancreatic β cells can be stored via cryopreservation. For instances, the cell population comprising non-native β cells, e.g., Stage 6 cells in some cases, can be dissociated into cell suspension, e.g., single cell suspension, and the cell suspension can be cryopreserved, e.g., frozen in a cryopreservation solution. The dissociation of the cells can be conducted by any of the technique provided herein, for example, by enzymatic treatment. The cells can be frozen at a temperature of at highest -20 °C, at highest -30 °C, at highest -40 °C, at highest -50 °C, at highest -60 °C, at highest -70 °C, at highest -80 °C, at highest -90 °C, at highest -100 °C, at highest -110 °C, at highest -120 °C, at highest -130°C, at highest -140 °C, at highest -150 °C, at highest -160 °C, at highest -170 °C, at highest -180 °C, at highest -190 °C, or at highest -200 °C. In some cases, the cells are frozen at a temperature of about -80 °C. In some cases, the cells are frozen at a temperature of about -195 °C. Any cooling methods can be used for providing the low temperature needed for cryopreservation, such as, but not limited to, electric freezer, solid carbon dioxide, and liquid nitrogen. In some cases, any cryopreservation solution available to one skilled in the art can be used for incubating the cells for storage at low temperature, including both custom made and commercial solutions. For example, a solution containing a cryoprotectant can be used. The cryoprotectant can be an agent that is configured to protect the cell from freezing damage. For instance, a cryoprotectant can be a substance that can lower the glass transition temperature of the cryopreservation solution. Exemplary cryoprotectants that can be used include DMSO (dimethyl sulfoxide), glycols (e.g., ethylene glycol, propylene glycol and glycerol), dextran (e.g., dextran-40), and trehalose. Additional agents can be added in to the cryopreservation solution for other effects. In some cases, commercially available cryopreservation solutions can be used in the method provided herein, for instance, FrostaLife^{™}, pZerve^{™}, Prime-XV^{®}, Gibco Synth-a-Freeze Cryopreservation Medium, STEM-CELLBANKER^{®}, CryoStor^{®} Freezing Media, HypoThermosol^{®} FRS Preservation Media, and CryoDefend^{®} Stem Cells Media.

In some cases, a cell cluster can be cryopreserved before subject to reaggregation using the method provided herein. In some cases, a cell cluster can be dissociated into cell suspension as provided herein and then cryopreserved. After cryopreservation for a certain period of time, the cryopreserved cells can be thawed and cultured for reaggregation using the method as provided herein. Cryopreservation as provided herein can prolong the availability of the pancreatic β cells or their precursors. In some cases, during differentiation of non-native pancreatic β cells from precursors thereof or stem cells, the intermediate cell population can be preserved following the method provided herein until the non-native pancreatic β cells are desired, e.g., for transplanting into a human patient. In some cases, the cells can be cryopreserved for any desired period of time before their further use or further processing of the cells, e.g., reaggregation. For example, the cells can be cryopreserved for at least 1 day, at least 5 days, at least 10 days, at least 1 month, at least 2 months, at least 3 months, at least 4 months, at least 5 months, at least 6 months, at least 7 months, at least 8 months, at least 9 months, at least 10 months, at least 11 months, at least 1 year, at least 2 years, at least 3 years, at least 4 years, at least 5 years, or at least 10 years.

Sc-β cells can exhibit a response to at least one glucose challenge. In some cases, the SC-β cells exhibit a response to at least two sequential glucose challenges. In some cases, the SC-β cells exhibit a response to at least three sequential glucose challenges. In some cases, the SC-β cell exhibits a response to multiple (e.g., sequential) glucose challenges that resembles the response of endogenous human islets to multiple glucose challenges, In some cases, the SC-β cells are capable of releasing or secreting insulin in response to two consecutive glucose challenges. In some cases, the SC-β cells are capable of releasing or secreting insulin in response to three consecutive glucose challenges, In some cases, the SC-β cells are capable of releasing or secreting insulin in response to four consecutive glucose challenges. In some cases, the SC-β cells are capable of releasing or secreting insulin in response to five consecutive glucose challenges. In some cases, the SC-β cells release or secrete insulin in response to perpetual consecutive glucose challenges. In some cases, cells can be assayed to determine whether they respond to sequential glucose challenges by determining whether they repeatedly increase intracellular Ca²⁺, as described in the examples herein.

In some cases, a method as provided herein can start with a cell population comprising NKX6.1-positive pancreatic progenitor cells. NKX6.1-positive cells can be differentiated into NKX6.1-positive and C-peptide-positive endocrine cells by contacting the NKX6.1-positive cells with at least one factor from EGF superfamily, e.g., betacellulin. In some cases, NKX6.1-positive and C-peptide-positive endocrine cells can also be referred to as insulin-positive endocrine cells. In some cases, one characteristic of insulin-positive endocrine cells can be expression of chromogranin A. In some cases, the population comprising insulin-endocrine cells can be dissociated and reaggregated into a cell cluster as described above.

In some cases, conditions that promote cell clustering comprise a suspension culture, In some cases, the period of time comprises a period of time sufficient to maximize the number of cells co-expressing C-peptide and Nkx6- l. In some cases, the period of time is at least 5 days, In some cases, the period of time is between 5 days and 7 days. In some cases, the period of time is at least 7 days. In some cases, the suspension culture is replenished every day (e.g., with β cell-maturation factors). In some cases, a period of time of between 5 days and 7 days maximizes the number of cells co-expressing C-peptide and NKX6.1.

In some cases, at least 15% of the NKX6.1-positive pancreatic progenitor cells in the population are induced to differentiate into insulin-positive endocrine cells. In some cases, at least 99% of the NKX6.1-positive pancreatic progenitor cells in the population are induced to differentiate into insulin-positive endocrine cells.

In some aspects, the disclosure provides a method of generating SC-β cells from pluripotent cells, the method comprising: a) differentiating pluripotent stem cells in a population into definitive endoderm cells by contacting the pluripotent stem cells with at least one factor from TGFβ superfamily and a WNT signaling pathway activator for a period of 3 days; b) differentiating at least some of the definitive endoderm cells into primitive gut tube cells by a process of contacting the definitive endoderm cells with at least one factor from the FGF family for a period of 3 days; c) differentiating at least some of the primitive gut tube cells into Pdx 1-positive pancreatic progenitor cells by a process of contacting the primitive gut tube cells with i)retinoic acid signaling pathway activator, ii) at least one factor from the FGF family, iii) a SHH pathway inhibitor, iv) a BMP signaling pathway inhibitor, v) a PKC activator, and optionally vi) a ROCK inhibitor, for a period of 2 days; d) differentiating at least some of the Pdx1-positive pancreatic progenitor cells into Pdxl-positive, NKX6.1-positive pancreatic progenitor cells by a process of contacting the Pdx1-positive pancreatic progenitor cells under conditions that promote cell clustering with i) at least one growth factor from the FGF family, ii) at least one SHH pathway inhibitor, and optionally iii) a RA signaling pathway activator, and optionally iv) ROCK inhibitor and v) at least one factor from TGFβ superfamily, every other day for a period of 5 days, wherein the NKX6.1-positive pancreatic progenitor cells expresses Pdx l and NKX6.1 ; e) differentiating at least some of the Pdx1-positive, NKX6.1-positive pancreeitic progenitor cells into Pdxl-positive, NKX6.1-positive, insulin-positive endocrine cells by a process of contacting the Pdxl-positive, NKX6.1-positive pancreatic progenitor cells under conditions that promote cell clustering with i) a TGF-β signaling pathway inhibitor, ii) a TH signaling pathway activator, iii) at least one SHH pathway inhibitor, iv) a RA signaling pathway activator, v) a γ-secretase inhibitor, and vi) at least one growth factor from the epidermal growth factor (EGF) family, every other day for a period of between five and seven days, wherein the Pdxl-positive, NKX6.1 , insulin-positive endocrine cells express Pdx1, NKX6.1, NKX2.2, Mafb, glis3, Sur l , Kir6.2, Znt8, SLC2A1 , SLC2A3 and/or insulin; and f) differentiating at least some of the Pdx l-positive, NKX6.1-positive, insulin-positive endocrine cells into SC-β cells by a process of contacting the Pdxl-positive, NKX6.1-positive, insulin-positive endocrine cells under conditions that promote cell clustering with i) a transforming growth factor β (TGF-β) signaling pathway inhibitor, ii) a thyroid hormone signaling pathway activator, and optionally iii) a protein kinase inhibitor, every other day for a period of between 7 and 14 days to induce the *in vitro* maturation of at least some of the Pdx1-positive, NKX6.1-positive, insulin-positive endocrine cells into SC-β cells, wherein the SC-β cells exhibit a GSIS response *in vitro* and/or *in vivo.* In some cases, the GSIS response resembles the GSIS response of an endogenous mature β cells.

In some aspects, the disclosure provides a method of generating SC-β cells from pluripotent cells, the method comprising: a) differentiating pluripotent stem cells in a population into definitive endoderm cells by contacting the pluripotent stem cells with at least one factor from TGFβ superfamily and a WNT signaling pathway activator for a period of 3 days; b) differentiating at least some of the definitive endoderm cells into primitive gut tube cells by a process of contacting the definitive endoderm cells with at least one factor from the FGF family for a period of 3 days; c) differentiating at least some of the primitive gut tube cells into Pdx 1-positive pancreatic progenitor cells by a process of contacting the primitive gut tube cells with i)retinoic acid signaling pathway activator and ii) at least one factor from the FGF family for a period of 2 days; d) differentiating at least some of the Pdx1-positive pancreatic progenitor cells into Pdxl-positive, NKX6.1-positive pancreatic progenitor cells by a process of contacting the Pdx1-positive pancreatic progenitor cells under conditions that promote cell clustering with at least one growth factor from the FGF family every other day for a period of 5 days, wherein the NKX6.1-positive pancreatic progenitor cells expresses Pdx l and NKX6.1 ; e) differentiating at least some of the Pdx1-positive, NKX6.1-positive pancreeitic progenitor cells into Pdxl-positive, NKX6.1-positive, insulin-positive endocrine cells by a process of contacting the Pdxl-positive, NKX6.1-positive pancreatic progenitor cells under conditions that promote cell clustering with i) a TGF-β signaling pathway inhibitor, ii) a TH signaling pathway activator, iii) at least one SHH pathway inhibitor, iv) a RA signaling pathway activator, v) a γ-secretase inhibitor, vi) at least one growth factor from the epidermal growth factor (EGF) family, and vii) BMP signaling pathway inhibitor, every other day for a period of between five and seven days, wherein the Pdxl-positive, NKX6.1 , insulin-positive endocrine cells express Pdx1, NKX6.1, NKX2.2, Mafb, glis3, Sur l , Kir6.2, Znt8, SLC2A1 , SLC2A3 and/or insulin; and f) differentiating at least some of the Pdx l-positive, NKX6.1-positive, insulin-positive endocrine cells into SC-β cells by culturing the Pdxl-positive, NKX6.1-positive, insulin-positive endocrine cells in MCBD131 medium that is supplemented with 2% BSA to induce the *in vitro* maturation of at least some of the Pdx1-positive, NKX6.1-positive, insulin-positive endocrine cells into SC-β cells, wherein the SC-β cells exhibit a GS1S response *in vitro* and/or *in vivo.* In some cases, the GSIS response resembles the GSIS response of an endogenous mature β cells.

In some aspects, the disclosure provides a method of generating SC-β cells from pluripotent cells, the method comprising: a) differentiating pluripotent stem cells in a population into Pdxl-positive, NKX6.1-positive pancreatic progenitor cells under suitable conditions; b) differentiating at least some of the Pdx l-positive, NKX6.1-positive pancreeitic progenitor cells into Pdxl-positive, NKX6.1-positive, insulin-positive endocrine cells by a process of contacting the Pdxl-positive, NKX6.1-positive pancreatic progenitor cells under conditions that promote cell clustering with i) a TGF-β signaling pathway inhibitor, ii) a TH signaling pathway activator, iii) at least one SHH pathway inhibitor, iv) a RA signaling pathway activator, v) a γ-secretase inhibitor, vi) at least one growth factor from the epidermal growth factor (EGF) family, and vii) BMP signaling pathway inhibitor, every other day for a period of between five and seven days, wherein the Pdxl-positive, NKX6.1 , insulin-positive endocrine cells express Pdx1, NKX6.1, NKX2.2, Mafb, glis3, Sur l , Kir6.2, Znt8, SLC2A1 , SLC2A3 and/or insulin; and c) differentiating at least some of the Pdx l-positive, NKX6.1-positive, insulin-positive endocrine cells into SC-β cells by culturing the Pdxl-positive, NKX6.1-positive, insulin-positive endocrine cells in MCBD131 medium that is supplemented with 2% BSA to induce the *in vitro* maturation of at least some of the Pdx1-positive, NKX6.1-positive, insulin-positive endocrine cells into SC-β cells, wherein the SC-β cells exhibit a GSIS response *in vitro* and/or *in vivo.* In some cases, the GSIS response resembles the GSIS response of an endogenous mature β cells.

In some aspects, the disclosure provides a method of generating a cell cluster containing pancreatic β cells, the method comprising: a) obtaining a cell population comprising NKX6.1-positive pancreatic progenitor cells; b) differentiating at least some of the NKX6.1-positive pancreeitic progenitor cells into NKX6.1-positive, insulin-positive (or C-peptide-positive) endocrine cells by a process of contacting the NKX6.1-positive pancreatic progenitor cells with at least one growth factor from the epidermal growth factor (EGF) family, wherein the Pdxl-positive, NKX6.1 , insulin-positive endocrine cells express Pdx1, NKX6.1, NKX2.2, Mafb, glis3, Sur l , Kir6.2, Znt8, SLC2A1 , SLC2A3 and/or insulin; and c) differentiating at least some of the Pdx l-positive, NKX6.1-positive, insulin-positive endocrine cells into pancreatic β cells by culturing the Pdxl-positive, NKX6.1-positive, insulin-positive endocrine cells in MCBD131 medium that is supplemented with 2% BSA to induce the *in vitro* maturation of at least some of the Pdx1-positive, NKX6.1-positive, insulin-positive endocrine cells into pancreatic β cells, wherein the pancreatic β cells exhibit a GSIS response *in vitro* and/or *in vivo.* In some cases, the GSIS response resembles the GSIS response of an endogenous mature β cells.

### V Method of Treating

Further provided herein are methods for treating or preventing a disease in a subject. A composition comprising the cell clusters resembling endogenous pancreatic islets can be administered into a subject to restore a degree of pancreatic function in the subject. For example, the cell clusters resembling endogenous pancreatic islets can be transplanted to a subject to treat diabetes.

The methods can comprise transplanting the cell cluster disclosed in the application to a subject, e.g., a subject in need thereof. The terms "transplanting" and "administering" can be used interchangeably and can refer to the placement of cells or cell clusters, any portion of the cells or cell clusters thereof, or any compositions comprising cells, cell clusters or any portion thereof, into a subject, by a method or route which results in at least partial localization of the introduced cells or cell clusters at a desired site. The cells or cell clusters can be implanted directly to the pancreas, or alternatively be administered by any appropriate route which results in delivery to a desired location in the subject where at least a portion of the implanted cells or cell remain viable. The period of viability of the cells or cell clusters after administration to a subject can be as short as a few hours, e.g. twenty-four hours, to a few days, to as long as several years. In some instances, the cells or cell clusters, or any portion of the cells or cell clusters thereof, can also be transadministered at a non-pancreatic location, such as in the liver or subcutaneously, for example, in a capsule (e.g., microcapsule) to maintain the implanted cells or cell clusters at the implant location and avoid migration.

A subject that can be treated by the methods herein can be a human or a non-human animal. In some cases, a subject can be a mammal. Examples of a subject include but are not limited to primates, e.g., a monkey, a chimpanzee, a bamboo, or a human. In some cases, a subject is a human. A subject can be non-primate animals, including, but not limited to, a dog, a cat, a horse, a cow, a pig, a sheep, a goat, a rabbit, and the like. In some cases, a subject receiving the treatment is a subject in need thereof, e.g., a human in need thereof.

As used herein, the term "treating" and "treatment" can refer to administering to a subject an effective amount of a composition (e.g., cell clusters or a portion thereof) so that the subject as a reduction in at least one symptom of the disease or an improvement in the disease, for example, beneficial or desired clinical results. For purposes of this disclosure, beneficial or desired clinical results include, but are not limited to, alleviation of one or more symptoms, diminishment of extent of disease, stabilized (e.g., not worsening) state of disease, delay or slowing of disease progression, amelioration or palliation of the disease state, and remission (e.g., partial or total), whether detectable or undetectable. Treating can refer to prolonging survival as compared to expected survival if not receiving treatment. Thus, one of skill in the art realizes that a treatment may improve the disease condition, but may not be a complete cure for the disease. As used herein, the term "treatment" includes prophylaxis.

The methods and compositions provided herein may be used to treat a subject who has, or has a risk (e.g., an increased risk) of developing a disease. In some cases, the disease is diabetes, including, but not limited to, type I diabetes, type II diabetes, type 1.5 diabetes, prediabetes, cystic fibrosis-related diabetes, surgical diabetes, gestational diabetes, and mitochondrial diabetes The disease may also be a diabetes complication, including heart and blood vessel diseases, diabetic nephropathy, diabetic neuropathy, diabetic retinopathy, foot damages, and hearing damages.

The methods can comprise transplanting the cell cluster to a subject using any means in the art. For example the methods can comprise transplanting the cell cluster via the intraperitoneal space, renal subcapsule, renal capsule, omentum, subcutaneous space, or via pancreatic bed infusion. For example, transplanting can be subcapsular transplanting, intramuscular transplanting, or intraportal transplanting, e.g., intraportal infusion. Immunoprotective encapsulation can be implemented to provide immunoprotection to the cell clusters.

### EXAMPLES

### Example 1. Method of re-aggregating cell clusters comprising non-native β cells.

This example shows exemplary methods for re-aggregating cells disclosed from cell clusters comprising stage 6 cells generated using methods described in U.S. Patent Application Nos. 14/684,129 and 14/684,101, which are incorporated herein in their entireties. Through the example, the starting clusters are referred as the native clusters. Compared with the native clusters, the re-aggregated cell clusters comprised higher percentage of cells expressing markers of an endogenous mature pancreatic β cell, and exhibited greater glucose-stimulated insulin secretion both *in vitro* and *in vivo.*

### Dissociating native clusters

Native clusters cultured in 500mL spinner flask were taken out of the incubator and allowed to settle to the bottom of the flask for 3-5 minutes. The flask was tilted at a 45° angle and the liquid inside the flask was aspirated as much as possible through the lid on one of the two arms of the flask using an aspiration tip attached to a vacuum trap.

The sediment cell clusters were transferred to a 50 mL conical tube using a serological pipette. The spinner flask was washed by adding 10 mL of PBS. The remaining clusters were transferred to the 50 mL conical tube. The transferring was repeated until substantially all clusters are collected in the 50mL conical tube. The transferred clusters were allowed to settle for 2-4 minutes to the bottom of the 50mL conical tube, then the PBS was aspirated away with an aspiration tip.

20mL of pre-warmed (to 37°C) TrypLE^{™} (for ~500 million cells) were added to the cells in the conical tube and incubated in water bath at 37C for 15 minutes. The conical tube was swirled at 3 minute intervals to stir the cluster-TrypLE^{™} mixture.

After 15 minutes incubation in TrypLE^{™}, the cells were forcefully pipetted up and down by a P1000 pipette tip until substantially no clusters were visible. Majority of the cells were in a single cell suspension at this stage. The TrypLE^{™}-cell mixture were then quenched by 20mL of Quench Media (at 1: 1 ratio of TrypLE^{™}:Quench Media).

The cell suspension was passed through a 20 µm mesh filter to remove any clusters that were not dissociated. Only a few small clusters were on the mesh as most of them had been dissociated into single cells and passed through the filter.

The single cell suspension is centrifuged at 200g for 4 minutes. The supernatant was removed and the cell pellet was re-suspended in 30mL pre-warmed (to 37°C) MCDB Culture Media and mixed well till uniform single cell suspension was seen..

### Seeding dissociated cells into spinner flasks

0.5 mL of the single-cell suspension was taken. The cell viability (membrane-integrity assay) and concentration were measured with the Vi-Cell. The cells were seeded into spinner flasks at 1X106 cells/mL (or higher density depending on desired cluster size and experimental obj ective).

### Culturing cells in spinner flasks for re-aggregation

Flasks with seeded cells were placed into a 37°C incubator with 5% CO₂ on a magnetic stir plate at 50 rpm. The flasks were placed directly above the stirrer such that the impeller rotates uniformly. If 30mL Biott was used, Biott was placed on the designated magnetic stirring system and stir at 60rpm.

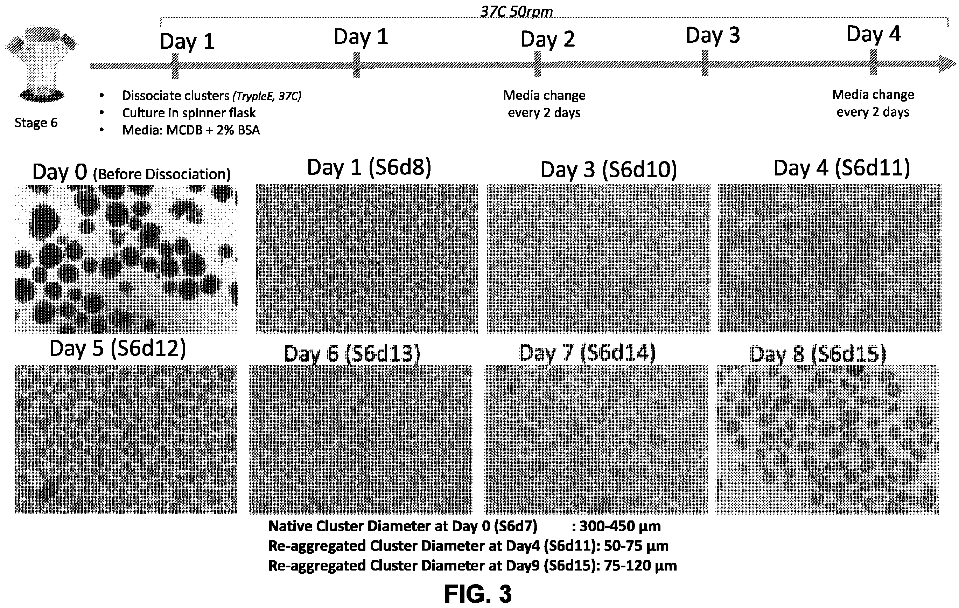

The cells were allowed to re-aggregate for 48 hours. The medium was changed for every 48 hours. For the first media change, the re-aggregated clusters were transferred to 50 mL conical tubes and spun down for 3 minutes at 300g. The pellets were re-suspended in fresh MCDB Culture Media. For subsequent media changes (every 48 hours), the re-aggregated clusters were filtered with a 20µm mesh filter. Non-aggregated cells passed through the filter, while re-aggregated clusters were captured by the mesh. The filtered was flipped over and the clusters were flushed using fresh MCDB Culture Media into the flask. After 4-7 days of culture, re-aggregated clusters became round and spherical. The morphology of cells during the re-aggregation process was shown in **FIG. 3****.**

### Cryopreservation of cells

The supply of viable tissues and cells for autologous implantation and heterologous transplantation and study is limited in part by the time a tissue or organ can be maintained in a viable state. Increasing the length of time that a tissue or organ remains viable may drastically increase the likelihood that a particular tissue reaches a recipient or researcher in a viable state. In some cases of the present disclosure, cryopreservation of cells was performed as described below to freeze and store SC beta cells upon the completion of differentiation to ensure functional cells are preserved.

In order to cryopreserve SC beta cells, TrypLE, PBS, and Stage 6 media may be pre-warmed to 37C. Additionally, Prime-XV cryopreservation solution may be kept on ice to keep cold for the cryopreservation. Standard QC may be performed prior to the dispersal process to determine an estimated of the number of cells within the flask. Target spinner flask(s) were removed from the incubator and into a cell culture hood to allow cells to settle for 3 to 5 minutes. Lids are removed from one arm of the flask to aspirate all the old media from the flask. For 0.05% HSA media filled flasks, media is removed with aspirating tip until the level reaches the rim at the bottom of the flask. After removing the aspirating pipette and replace the lid to the arm, the main lid is unscrewed from the spinner flask. A 25mL serological pipette is prepared and once the flask is tilted, the remaining media is carefully removed. The Native cell cluster pellet is collected with a 5mL serological pipette (there is no rim inside the 5mL serological pipette so no clusters will be trapped) and transfered to a 50mL conical tube. The spinner flask is washed with 10mL of pre-warmed PBS to collect remaining Native clusters and transferred to the 50mL conical tube. The wash process is repeated three more times. The clusters are allowed to settle for 2 to 4 minutes before aspirating all but 5mL of the PBS from the 50mL conical tube. 40mL of pre-warmed PBS is added to the conical tube to wash cells again, and the Native clusters are allowed to settle for 2 to 4 minutes before aspirating out as much PBS as possible. 20mL of warm TrypLE is added to every 300M cells and the conical tube is incubated for 20 minutes in the incubator. After the first 10 minutes, the incubator door is opened to swirl the cells in the conical tube to ensure an even distribution of TrypLE to all cells. The cells are incubated for another 10 minutes. After incubation, the conical tube is transferred to the hood without agitating the pellet. The TrypLE is aspirated from the conical tube and 10mL of warm Stage 6 media is added to the conical tube. With a P1000 pipette tip, the solution is pipetted up and down 10 to 15 times to break up clusters. The conical tube is topped off to 40mL with warm Stage 6 media. After pre-wetting a 40um filter, the cells are transferred through the filter to remove any non-dissociated clusters from the solution. The conical tube is rinsed with 10mL of Stage 6 media to wash the tube and filter through the 40um mesh for a final volume of 50mL. A sample is taken to the Vi Cell cell counter for cell count and concentration measurements. Once the cell count is determined, the conical tube is spun down at 300xg for 3 minutes. Bring the conical tube back to the hood and aspirate all the Stage 6 media. The cells are re-suspended in cold PRIME-XV to the concentration of 100M cells per milliliter and aliquoted accordingly in labeled cryovials. Finally, the filled cryovials are placed in Cool Cells to sit overnight in the -80 freezer and transferred to liquid nitrogen storage 24 hours later. Cryovials can also be rapidly frozen with the Control Rate Freezer then transferred immediately to liquid nitrogen.

### Example 2. Method of thawing and culturing cryopreserved re-aggregated cell clusters

### Thawing Cells

The supply of viable tissues and cells for autologous implantation and heterologous transplantation and study is limited in part by the time a tissue or organ can be maintained in a viable state. Increasing the length of time that a tissue or organ remains viable may drastically increase the likelihood that a particular tissue reaches a recipient or researcher in a viable state. In some cases of the present disclosure, thawing cryopreserved cells was performed as described below obtain functional SC beta cells.

In order to thaw and culture cryopreserved SC beta cells, a cryopreserved vial is taken and rapidly thawed at 37C in water bath for 2-3 minutes. When the vial is thawed with a small amount of ice crystal left, the vial is removed from the water bath and transferred to the cell culture hood after wiping down vial with ethanol. A 15mL conical tube is prepared containing at least the same volume of the cryo vials content with pre-warmed Stage 6 media. For example, if each cryo vial contains 2ml, if 1 vial is thawed, ensure that there is at least 2ml of media in the conical tube. For thawing 1 vial, a 5mL serological pipette is used to aspirate up 3mL of Stage 6 media, and then, using the same pipette, aspirate up the 2ml contents of the cryo vial. The entire 5mL cells+media is dispensed in the serological pipette into the 15mL conical tube containing the cells with Stage 6 media drop by drop while swirling it. Adding cells to a conical tube rapidly (instead of drop by drop) can cause osmotic shock and may damage cells. Up an additional 1 or 2mL of Stage 6 media is aspirated to wash the vial containing the cells, and then added to the 15mL conical tube. The media and cells is topped off with warmed Stage 6 media up to 14 or 15mL. This it to dilute out the freezing media, and to ensure that any cells stuck to the walls of the conical tube are washed down during centrifugation.

A triple spin down is subsequently performed by centrifuging the cells at 100g for 3 minutes. The supernatant is collected, transferred into another 15mL conical tube, and centrifuged again at 200g for 3 minutes. The supernatant is collected, transferred into another 15mL conical tube, and centrifuged again at 300g for 3 minutes. An additional spin down can be done if supernatant is still cloudy. When all spin downs are completed, the supernatant is aspirated out from the conical tubes. The cell pellet from all spin downs is collected by resuspending in 10mL of Stage 6 media in one of the 15mL conical tubes. A small sample of the single-cell suspension is taken for cell count using either a hemacytometer or Vi-Cell Counter. The cells are seeded into Biott(s) at a density of 2x106 viable cells per mL (or higher density depending on desired cluster size and experimental objective). For example, if the total viable cell count is 60 million cells of the 10mL solution, transfer the 10mL of cells into the Biott, then use 20mL of Stage 6 media to rinse the conical tube and add it back to the flask, making the final volume 30mL. The re-aggregated cluster diameter is <120um at St6d4 when seeded at 2X106 cells/mL. Small Biotts are able to contain up to 40mL of media (80 million viable cells). Other protocols describe using 500mL spinner flasks, however thawed cells may not aggregate very well in them. Cell-seeded flask(s) are placed into a 37 °C incubator with 5% CO2 on a magnetic stir plate at 60 rpm. Ensure that the flask sits directly above the stirrer such that the impeller rotates uniformly, otherwise cluster formation may be poor. Cells are allowed to re-aggregate for 24 hours. Change media 24 hours post thaw.

### Changing Media

Media may be changed at 24 hours (Day 2) and will require triple-spin down of re-aggregated clusters. Cells are transferred to a 50mL conical and centrifuge for 3 minute at 100g. The supernatant is collected and transfer using a serological pipette to another 50mL conical tube. The cell pellet is re-suspended in fresh 5mL of Stage 6 media and transfer them back into the spinner Biott. The supernatant is spun down again for 3 minutes at 200g. The cell pellet is re-suspended in fresh Stage 6 media and transferred back into the spinner flask/Biott. The supernatant is collected again and transfer using a serological pipette to another conical tube and centrifuge at 300g for 3 minutes. The supernatant is aspirated out. The cell pellet is re-suspended in fresh Stage 6 media and transferred them back into the spinner flask/Biott. With the remaining media needed to fill the Biott/flask to its full volume, the conical tubes are rinsed for any remaining cells. The rest of the flask/Biott is topped up with the Stage 6 media used to wash the conical tubes to the original volume (where cell density is at 2x106 cells/mL). The first spindown is only for 100g to avoid the newly re-aggregated clusters from clumping together. The subsequent two centrifugations of the supernatant is to collect all the single cells that did not cluster together. This is to allow the desired cells sufficient time to cluster together into aggregates. Filtering them after 24 hours will cause a loss of many good cells and give lower yield. Media may be changed a second time at Day 4 (72 hours after initial seeding) by filtering all the re-aggregated clusters through a 20-µm mesh filter. The mesh filter is pre-wet with media before using. Non-aggregated cells pass through the filter, while re-aggregated clusters are caught by the mesh. The filter is flipped straight over the Biott, and the clusters are flushed into the Biott using fresh Stage 6 media. When flushing the cells from the filter back into the Biott, a 25mL serological pipette is used and pressed against the mesh. The serological pipette is moved around the entire mesh while dispensing the media. Media change is done using a filter to remove the unwanted cells that did not aggregate. After all the cells have been allowed sufficient time to re-aggregate, those cells that are still single cell after 4 days may not form clusters. Subsequent media changes at S6d6, S6d8 (e.g., every 48 hours) are performed similarly. All the re-aggregated clusters are filtered through a 20-µm mesh filter. The mesh filter is pre-wet with media before using. Non-aggregated cells pass through the filter, while re-aggregated clusters are caught by the mesh. The filter is flipped over and the clusters are flushed into the flask using fresh Stage 6 media. Re-aggregated clusters at the third media change are usually compact and spherical. Other protocols suggest that cells be centrifuged for media change. However, there is a bigger loss of cells when cells are centrifuged. Furthermore, centrifuging can cause cells to clump together, making the cluster size bigger. It is important to ensure all the clusters on the mesh are collected by flushing them down with a moving serological pipette pressed against the mesh. Cell yield is based off the viable cells seeded (not the total number). For example, 60 million viable cells were seeded at S6d1 immediately post thaw. At S6d10, after the non-re-aggregated single cells have been filtered out, a sample is taken, dissociated in TrypLE, and counted. If the Biott is calculated to have 30million cells left, that corresponds to a yield of 50%. It is common to see ~70% viability immediately post thaw. At S6d4, cell yield may be between 30-50%. SC-Islets are optimal for GSIS and testing between S6d7-S6d12. Beyond S6d12, a decrease in cell yield, and an increase in cluster size, may be observed.

### Example 3. Characterizing re-aggregated cell clusters

The re-aggregated cell clusters generated in Example 1 were characterized to determine whether they had function and characteristics of endogenous pancreatic islets. The function and features of the re-aggregated cell clusters were compared to those of the native clusters. The comparison showed that the re-aggregated cell clusters were more similar to endogenous pancreatic islets compared to the native clusters.

### Flow Cytometry

The percentage of cells expressing markers of endogenous mature pancreatic β cells were detected in the re-aggregated clusters and native clusters by flow cytometry and compared.

The native clusters and re-aggregated clusters were dispersed into single-cell suspension by incubation in TrypLE^{™} Express. Cells, generally 1-2 million, were washed once in PBS (1mL) and transferred to a 1.7ml microcentrifuge tube (Bioscience; 11510). Cells were re-suspended in 4%PFA and incubated on ice for 30 min. Cells were then washed once in PBS followed by incubation in blocking buffer (PBS + 0.1% Triton X-100 + 5% donkey serum) on ice for 1 hr.

Cells were then re-suspended in blocking buffer with primary antibodies and incubated at 4°C overnight. Primary antibodies against human NKX6.1, human CHGA, and human C-peptide were diluted and incubated with the cells. Cells were washed twice in blocking buffer and then incubated in blocking buffer with secondary antibodies on ice for 2 hr. Secondary antibodies conjugated to Alexa Fluor 488 or 647 (Life Technologies) were used to visualize primary antibodies.

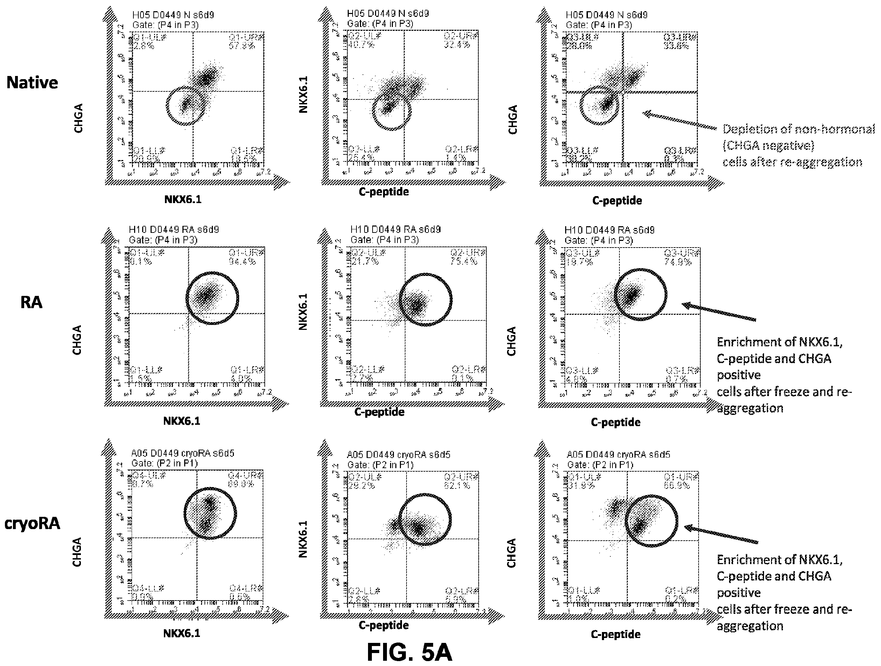

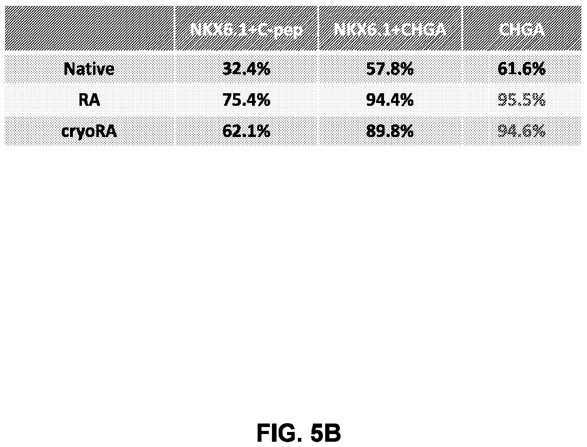

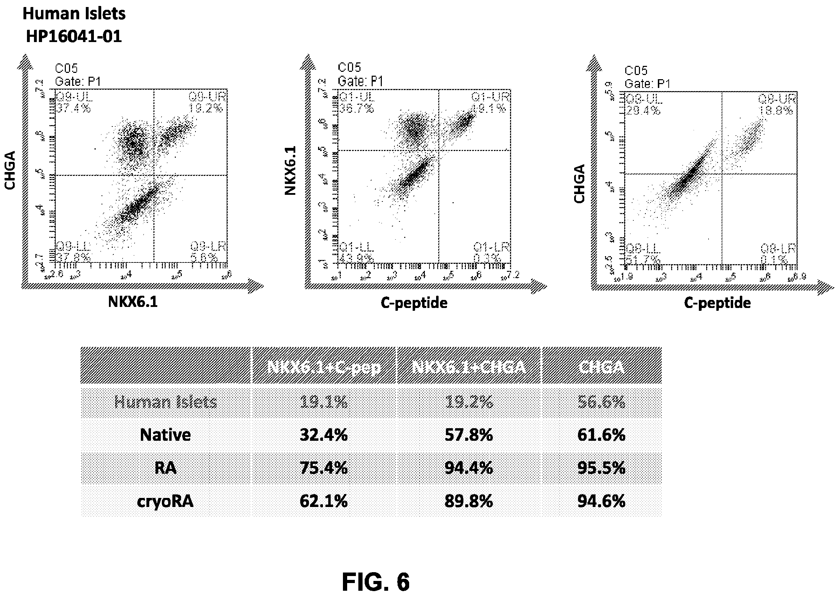

Cells were then washed 3 times in sorting buffer (PBS + 0.5% BSA (Sigma; A8412) and finally re-suspended in 500-700ml sorting buffer, filtered through a 40mmnylon mash into flow cytometry tubes (BD Falcon; 352235), and analyzed using the LSR-II flow cytometer (BD Biosciences) with at least 30,000 events recorded. Analysis of the results was performed using FlowJo software. **FIG. 5** shows that, in re-aggregated clusters with and without cryopreservation, cells expressing CHGA, NKX6.1 and C-peptide were enriched, compared to the native clusters. In particular, approximately 95.5% and 94.6% of RA cells and cryoRA cells, respectively, expressed CHGA, as compared to 61.6% of native cells. Similarly, approximately 94.4% and 89.8% of RA cells and cryoRA cells, respectively, expressed both NKX6.1 and CHGA, as compared to 57.8% of native cells. Finally, using the methods of the present disclosure, approximately 75.4% and 62.1% of RA cells and cryoRA cells, respectively, expressed both NKX6.1 and C-peptide, as compared to 32.4% of native cells. Additionally, as shown in **FIG. 6****,** in re-aggregated cell clusters with and without cryopreservation, cells expressing CHGA, NKX6.1 and C-peptide were enriched, as compared to the human cadaveric islets. In particular, only 18.8%, 19.2%, and 56.6% of human cadaveric islets expressed markers for NKX6.1 and C-peptide, NKX6.1 and CHGA, or CHGA, respectively.

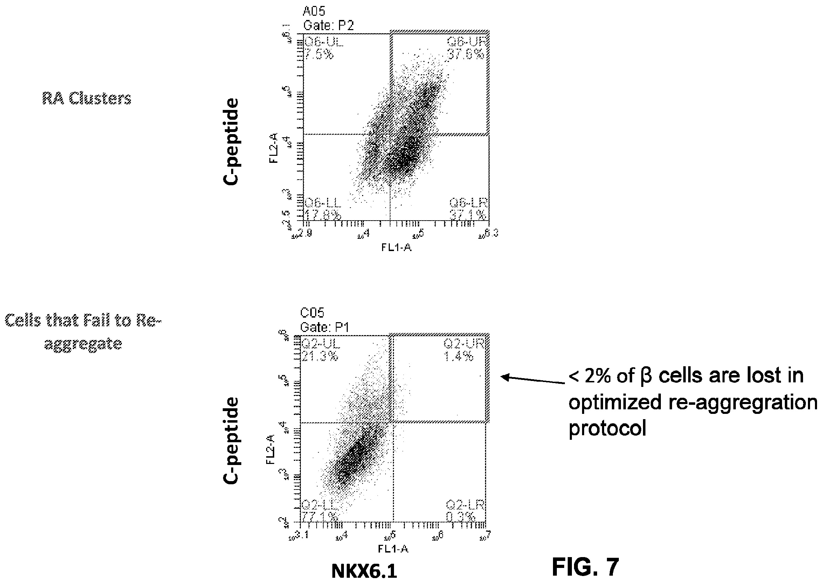

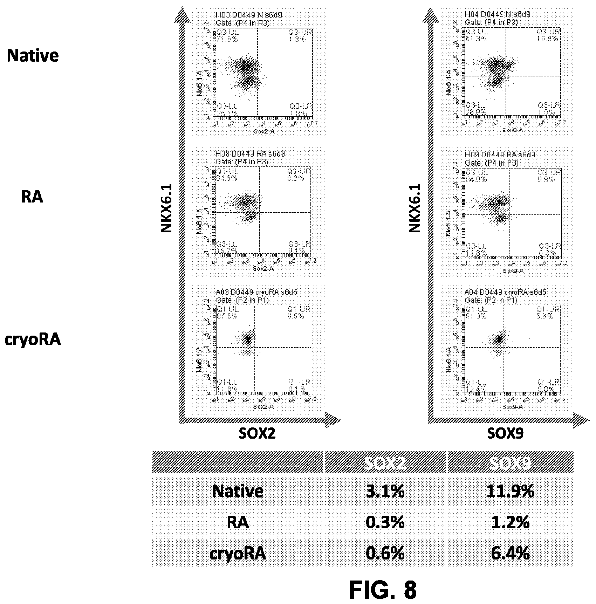

As described above, non-aggregated cells pass through the filter, while re-aggregated clusters are caught by the mesh. Flow cytometry characterization of cells that fail to re-aggregate (e.g., cells that pass through the filter) confirm that re-aggregation captures all NKX6.1 and C-peptide positive beta cells, and does not significantly impact cell yield. As shown in **FIG. 7****,** re-aggregation resulted in greater than 98% of cells expressing beta cell-specific markers (e.g., both NKX6.1 and C-peptide) being captured by the mesh, with less than 2% of cells being lost (e.g., passing through the mesh). Generally, of the cells that fail to form a cell cluster (e.g., cells that fail to reaggregate, and/or cells that pass through the mesh), less than about 10% express NKX6.1 and C-peptide as measured by flow cytometry. In some cases, of the cells that fail to form a cell cluster (e.g., cells that fail to reaggregate, and/or cells that pass through the mes), less than about 9%, 8%, 7%, 6%, 5%, 4%, 3%, 2%, or 1% express NKX6.1 and C-peptide as measured by flow cytometry. Similarly, re-aggregation of cells also results in in a reduction of SOX9-positive and SOX-2 positive off-target cell populations. As shown in **FIG. 8****,** in re-aggregated clusters with and without cryopreservation, cells expressing SOX2 and SOX9 were depleted, as compared to the native clusters. In particular, approximately 0.3% and 0.6% of RA cells and cryoRA cells, respectively, expressed SOX2, as compared to 3.1% of native cells. Similarly, approximately 1.2% and 6.4% of RA cells and cryoRA cells, respectively, expressed SOX9, as compared to 11.9% of native cells. SOX2- and/or SOX9-positive cells can represent off-target cell populations (e.g., non-beta cells), such as proliferative or partially differentiated cells.

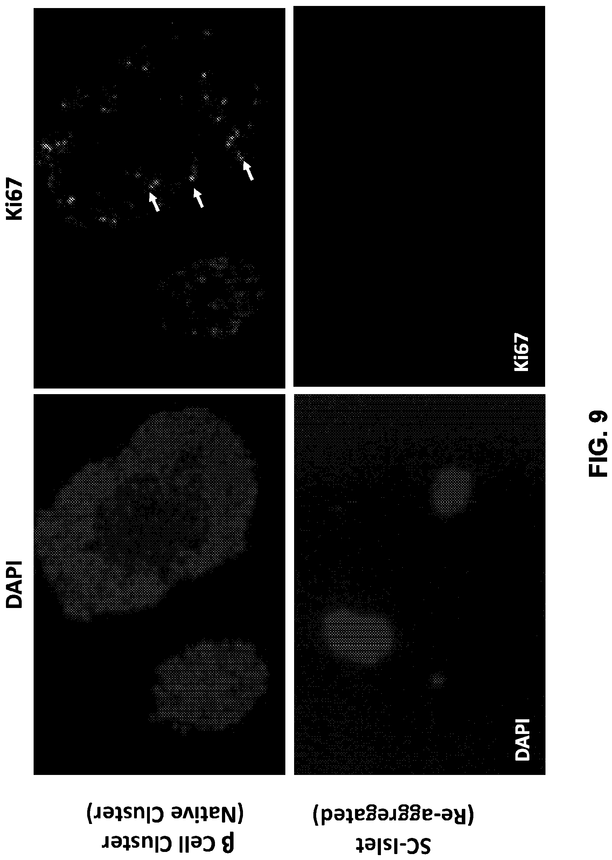

A person of ordinary skill in the art will appreciate that techniques other than flow cytometry may also be used to characterize cells following re-aggregation. Non-limiting examples of cell characterization methods include gene sequencing, microscopic techniques (fluorescence microscopy, atomic force microscopy), karyotyping, isoenzyme analysis, DNA properties, viral susceptibility. In one example, as shown in **FIG. 9****,** native cells (top) and re-aggregated cells (bottom) are immunostained for (left) DAPI (e.g., to indicate nuclei) and (right) Ki67 (e.g., a cellular marker for proliferation). As shown, re-aggregation of cells resulted in a decrease in the number of Ki67-positive cells, indicating a reduction in the number of proliferative (e.g., off target) cell population. A persona having ordinary skill will appreciate that any cell marker may be used to detect off-target cell populations (e.g., SOX2, SOX9, LIN28).

### In vitro Glucose-Stimulated Insulin Secretion

Krebs buffer (Krb) was prepared as follows: 128 mM NaCl, 5 mM KCl, 2.7 mM CaCl₂, 1.2 mM MgCl₂, 1 mM Na₂HPO₄, 1.2 mM KH₂PO₄, 5m MNaHCO₃, 10m MHEPES (Life Technologies; 15630080), 0.1% BSA (Proliant; 68700) in deionized water. Krb solutions containing 2mM glucose (low glucose), 20mM glucose (high glucose), or 2mM glucose and 30mM KCl (KCl polarization challenge) were prepared and equilibrated to 37°C.

Native, re-aggregated clusters, and cryopreserved re-aggregated clusters were washed twice with 1 ml Krb buffer and then pre-incubated in 3 ml low glucose Krb for two hours to remove residual insulin. Clusters were washed 2 times in Krb and then incubated in 1 ml low glucose Krb for 30 min. A sample of 200ul of the supernatant was collected after incubation for ELISA analysis (low glucose sample). Clusters were washed 2 times in Krb and then incubated in high glucose Krb for 30 min and 200ul of supernatant was collected after incubation (high glucose sample). Finally, clusters were washed twice in Krb and then incubated in Krb containing 2mM glucose and 30mM KCl (polarization challenge) for 30 min. A sample of 200ul of the supernatant was collected after incubation for ELISA analysis (KCl polarization challenge sample).

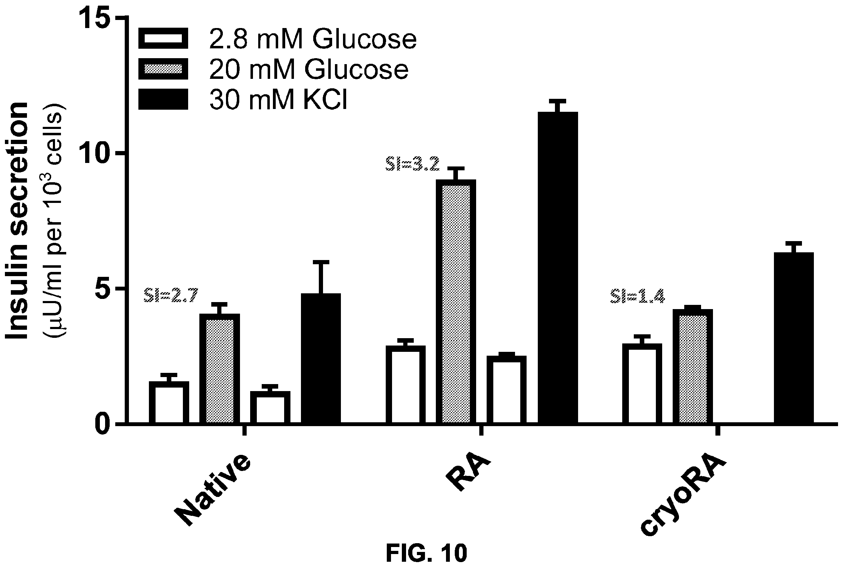

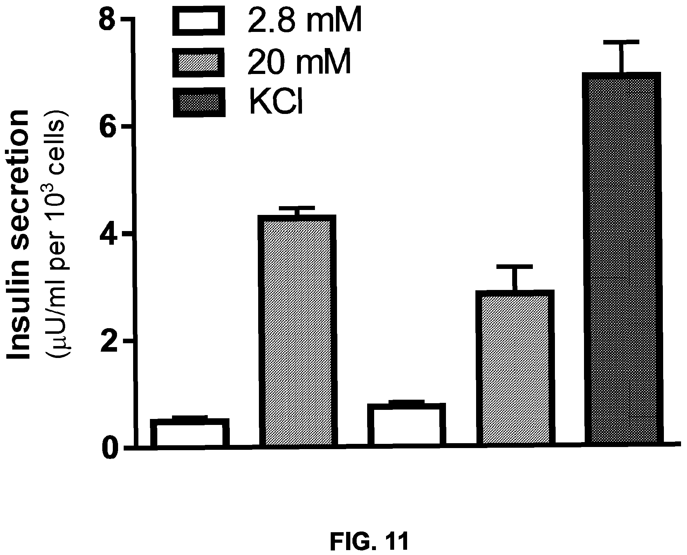

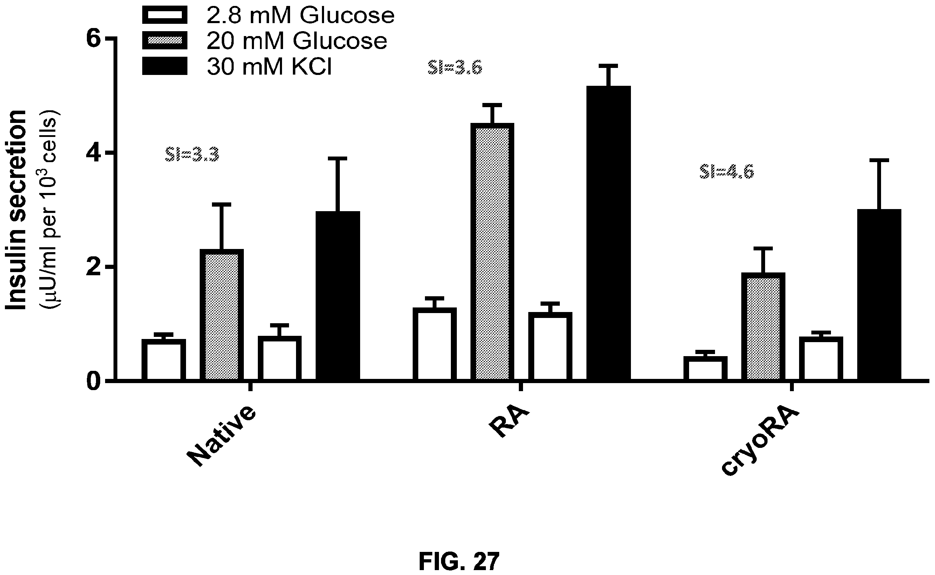

Supernatant samples containing secreted insulin were processed using the Human Ultrasensitive Insulin ELISA (ALPCO Diagnostics; 80-INSHUU-E01.1) and samples were measured by a FLUOstar optima spectrophotometer (BMG lantech) at 450 nm. The levels insulin secreted in response to glucose and KCl (as a positive control) in native cells, re-aggregated cells, and cryopreserved re-aggregated cells are shown in **FIG. 10****.** And **FIG. 27** shows another example of insulin responses of native cells, re-aggregated cells, and cryopreserved re-aggregated cells. Furthermore, cells were also treated with sequential doses of increasing or decreasing concentrations of glucose (e.g., glucose concentration changed every 1 hour), to determine if cells are capable of an acute temporal response. As shown in **FIG. 11****,** re-aggregated SC-islets were responsive to sequentially increasing and decreasing concentrations of glucose (e.g., insulin secretion in re-aggregated cells was proportional to glucose concentration).

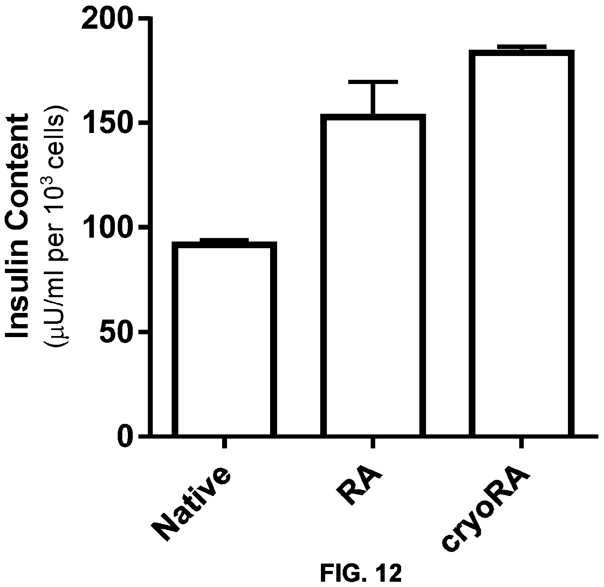

*In vitro* glucose-stimulated insulin content was also measured. Following cell lysis, insulin content (e.g., the amount of insulin within a cell) was measured in native cell lysate, re-aggregated cell lysate, and cryopreserved re-aggregated cell lysate. As shown in **FIG. 12****,** re-aggregated cells with (cryoRA) and without (RA) cryopreservation showed increased intracellular insulin content, as compared to native cells.

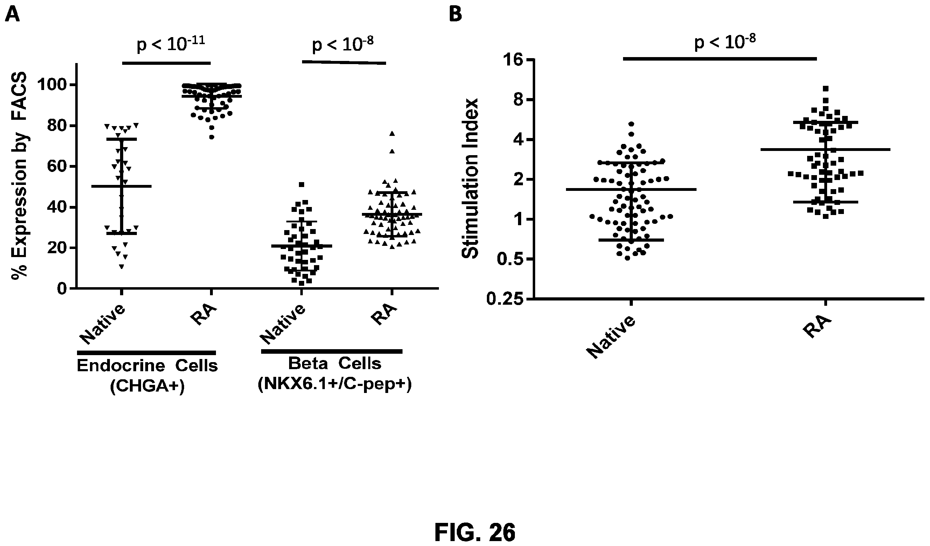

*In vitro* glucose-stimulated insulin secretion stimulation index was also tested in native and reaggregated cell clusters. As shown in **FIG. 26A****,** reaggregation significantly increased percentage of chromogranin A (CHGA)-positive cells from around 50% in native cell cluster to close to 100% in reaggregated cell cluster (RA), and reaggregation also increased percentage of pancreatic β cells (NKX6.1 and C-peptide double positive). The enrichment of endocrine cells and pancreatic β cells in the reaggregated cell cluster was correlated with enhancement of pancreatic function as demonstrated by stimulation index, as **FIG. 26B** demonstrates that the stimulation index of reaggregated cell clusters was also significantly higher than the native cell clusters.

### In vivo Glucose-Stimulated Insulin Secretion

To compare the *in vivo* GSIS responses of the native and re-aggregated clusters, NRG-Akita mice that have hyperglycemia were used. Blood glucose levels were determined in nonfasted mice prior to their entry into the experiment for confirmation of hyperglycemia prior to cell transplantation. For transplantation, the mice were fasted for 16 hr, fasted blood glucose levels were determined, and mice were transplanted with the native clusters or re-aggregated clusters.

The recipient mice were anesthetized by intraperitoneal injection of 5 ml/gram of a mixture of 15 mg/ml ketamine and 2 mg/ml xylazine. All surgery was performed using aseptic technique in a sterile hood under aseptic conditions using sterilized gauze pads as surgical draping. An incision approximately 2.0 cm was made through the skin and then through the underlying musculature and the kidney gently pulled through the incision space. A 16 gauge trocar was used to implant the blood clot containing the cells under the subrenal capsule and the graft bearing kidney was placed back into the peritoneal cavity. A 4-0 coated vicryl suture (Ethicon vicryl J464G) using an interrupted stitch was used to suture the musculature and the skin site was then closed using surgical wound clips (BD 427631).

Animals were kept warm during recovery from surgery and were given a single i.p. dose of 2.4mg/kg buprenorphine SR-LAB to provide pain relief for 72 hr (ZooPharm BZ8069317). The mice were monitored three times a week and fourteen days later, the surgical wound clips were removed.

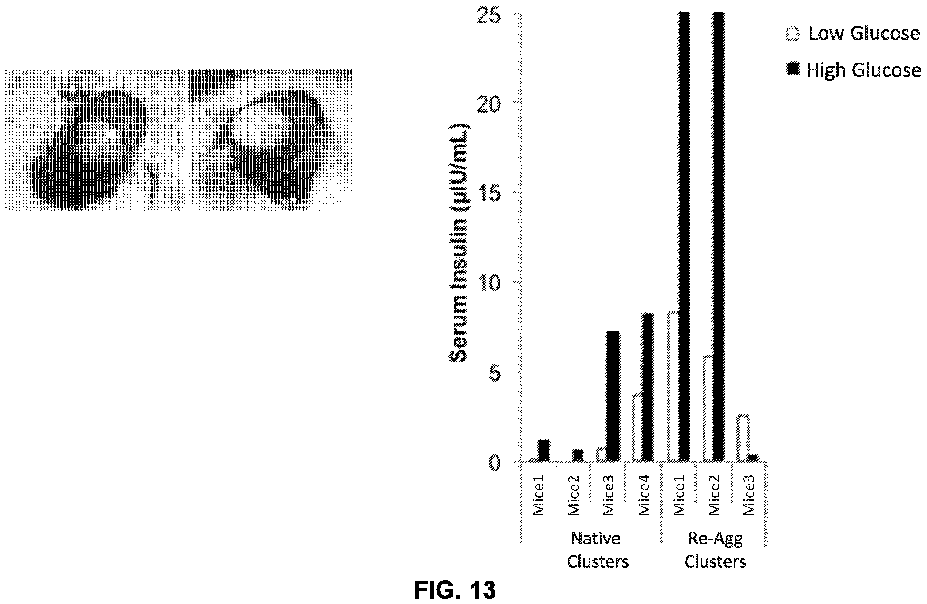

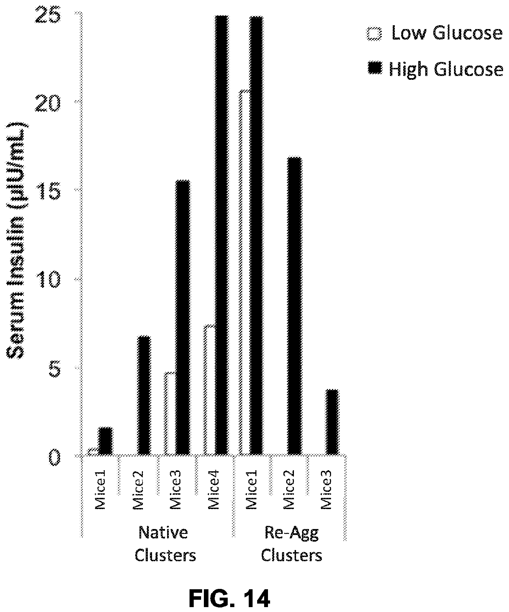

At select times, the animals were fasted for at least 16 hr, a baseline blood glucose was determined and the mice were challenged with an intraperitoneal injection of 2 g/kg D-(+)-glucose. Thirty minutes post glucose challenge blood glucose levels were determined and 150 ml of blood was obtained through facial vein puncture using a lancet, allowed to clot for 60 min, and serum recovered for the determination of human insulin were by ELISA, as in *in vitro* GSIS experiments. The results at 2 weeks (see, e.g., **FIG. 13**) and 4 weeks (see, e.g., **FIG. 14**) showed that re-aggregated clusters exhibited better *in vivo* GSIS responses compared to native clusters.

### Cell Metabolic Activity

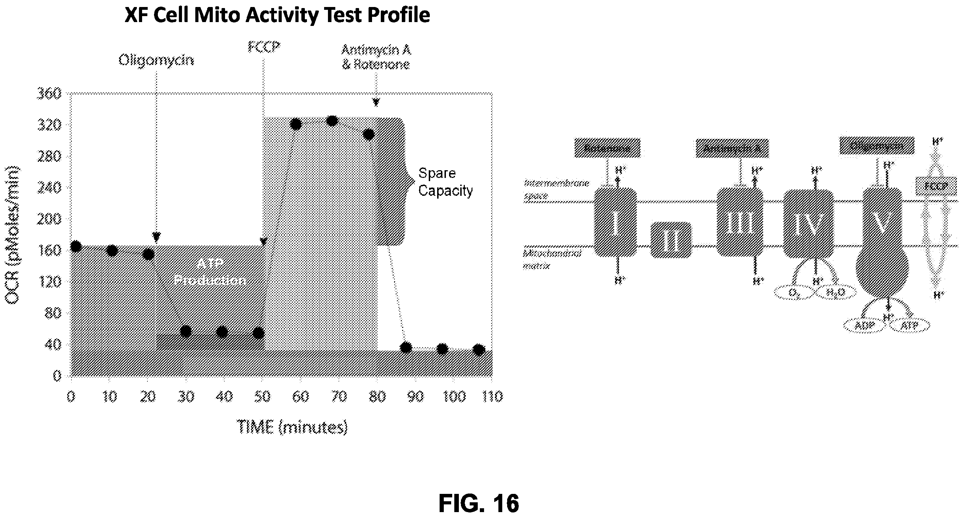

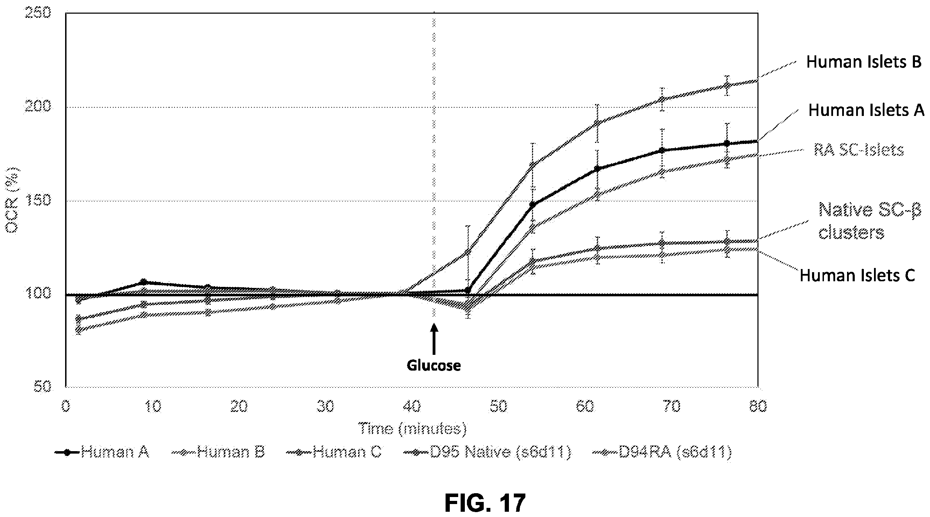

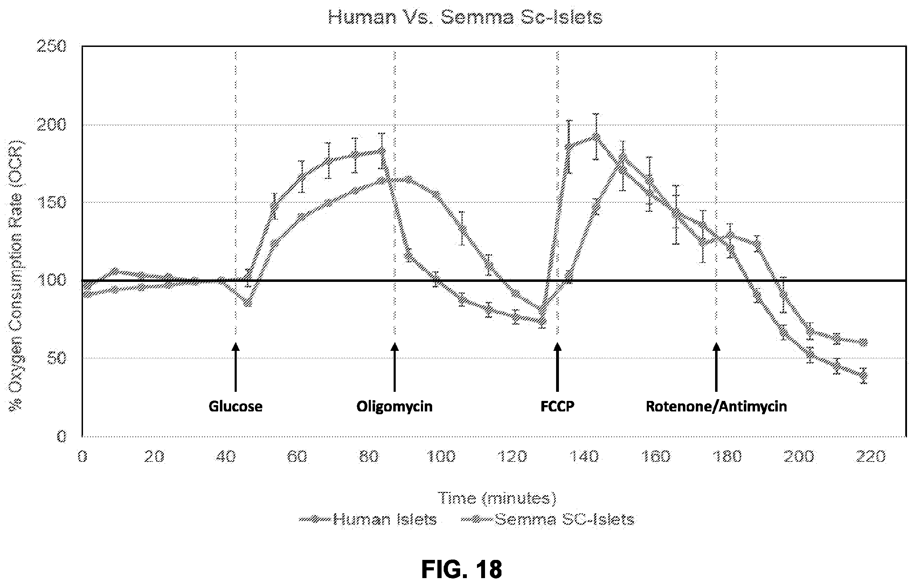

Mitochondrial function and glycolysis play critical roles in a variety of cellular processes, including cellular activation, proliferation, differentiation, cell death, and disease progression. Cellular mitochondrial function was measured by sequential injections of oligomycin, FCCP (Carbonyl cyanide p-trifluoromethoxy phenylhydrazone) and Antimycin A / rotenone to define basal OCR, ATP-linked OCR, proton leak, maximal respiratory capacity, reserve respiratory capacity and non-mitochondrial oxygen consumption (see, e.g., **FIG. 16**). Prior to treating the cells with any chemicals, the first recordings correspond to basal respiration. Various chemicals may be added to the cells to determine the effects of the chemical on oxygen consumption (e.g., mitochondrial function) of the cell. In one example, cells may be treated with glucose to determine the glucose-stimulated response in oxygen consumption rate. Oligomycin A, which inhibits ATP synthase by blocking proton channel and significantly reduce electron flow through the electron transport chain, is necessary for oxidative phosphorylation of ADP to ATP. To determine ATP production, oligomycin is added into the media. ATP production can be measured by the drop in basal respiration. The difference that is remaining from basal respiration, that ATP production does not account for, is called proton leak. Proton leak can be a sign of mitochondrial damage, however it can also be used as a mechanism to regulate mitochondrial ATP production or could be a normal process of the cell. Elevated amounts of proton leak can indicate damage. FCCP is a protonophore (H+ ionophore) and uncoupler of oxidative phosphorylation in mitochondria, which is capable of depolarizing plasma and mitochondrial membranes. After adding FCCP, electron flow through the electron transport chain (ETC) is uninhibited and oxygen is maximally consumed by complex IV. Spare capacity, which is measured as the difference between the top of the OCR curve (following addition of FCCP) to basal respiration, is how much "work" a cell could do under stressful conditions (ability of the cell to respond to increased energy demand). Rotenone, an inhibitor of mitochondrial electron transport at NADH:ubiquinone oxidoreductase, inhibits the transfer of electrons from iron-sulfur centers in complex I to ubiquinone and interferes with NADH. As shown in **FIG. 17****,** oxygen consumption rate in adult human islets, native cells, and re-aggregated cells was measured in response to glucose treatment to determine mitochondrial function. Re-aggregated cells exhibited approximately a 75% increase in OCR as compared to as compared to approximately a 25% increase in OCR exhibited by native cells. Furthermore, sequential addition of oligomycin A, FCCP and Rotenone/Antimycin A showed similar OCR profiles between adult human islets and re-aggregated cells (Semma SC-Islets), indicating that mitochondrial function in re-aggregated cells resemble adult human islets (**FIG. 18**).

### In vivo Graft Characterization

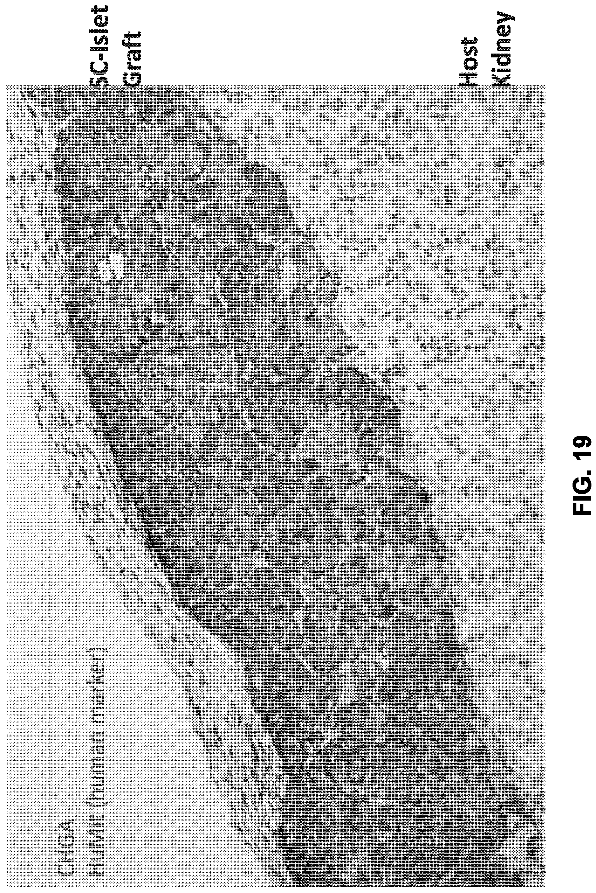

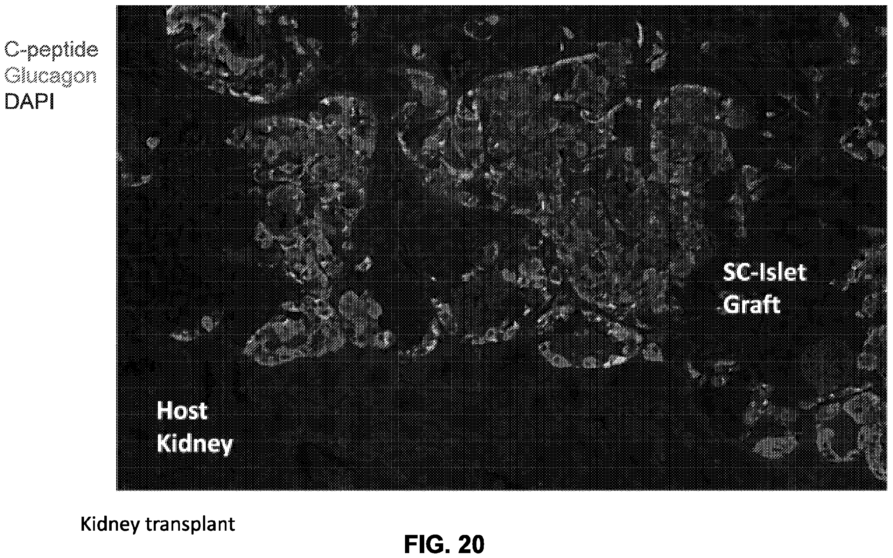

Following graft transplantation using re-aggregated cells, histological sections were obtained and stained for CHGA and HuMit (e.g., a human marker). As shown in **FIG. 19****,** tissue sections exhibited uniform distribution of endocrine cells through the transplanted graft with re-aggregated clusters at 6 months post transplantation. Additionally, tissue sections exhibited increased numbers of alpha and beta cells at 6 months post transplantation, as indicated by immunofluorescence staining of C-peptide and glucagon (**FIG. 20**).

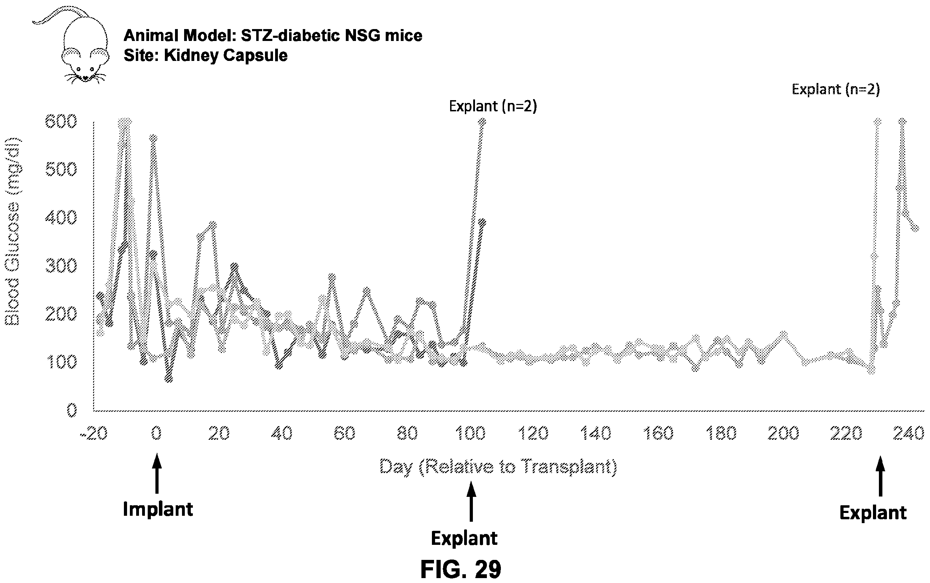

**FIG. 29** shows an experiment in which exemplary cell clusters were transplanted into kidney capsule of diabetic mouse model. In these experiments, STZ-diabetic NSG mice were transplanted with re-aggreated cell clusters and their blood glucose level was monitored before and after the implant, and after explant of the implanted cell clusters. As shown in the figure, all 4 mice tested showed improved glycemic control (decreased and stable blood glucose level) after implant and worsening of the glycemic control after the explant.

### Correlation with stirring speed

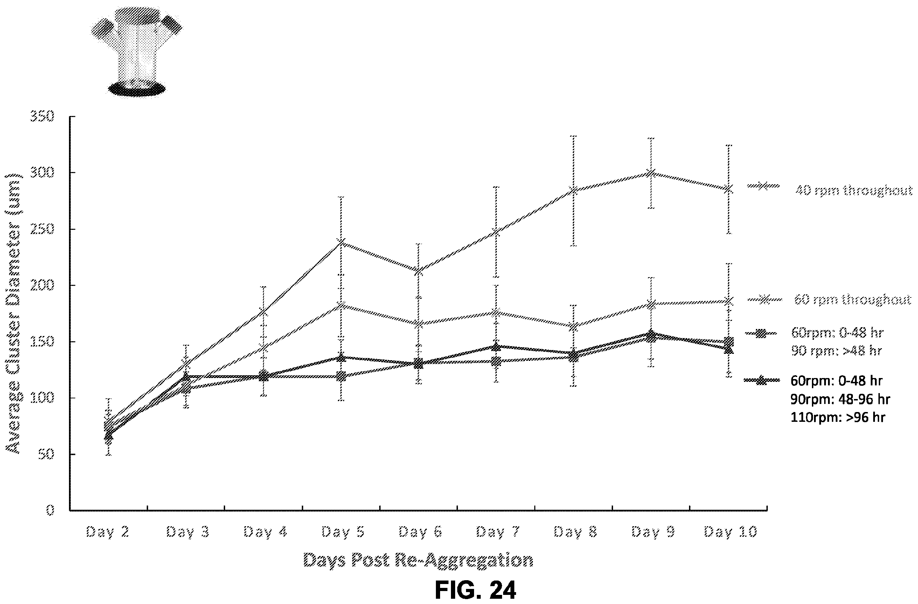

**FIG. 24** demonstrates exemplary correlation between stirring speed of the culture medium and the cell cluster size during reaggregation. Different stirring speed regimens were tested for a re-aggregation protocol: 40 rpm throughout the reaggregation period of 10 days, 60 rpm throughout, 60 rpm for the first 48 hours followed by 90 rpm for the remaining days, 60 rpm for the first 48 hours and 90 rpm for second 48 hours, followed by 110 rpm for the remaining days. As shown in the figure, increase in the stirring speed from 40 rpm to 60 rpm significantly reduced the cell cluster size, the regimens with 60 rpm and 90 rpm yield the smallest cluster size.

### Heterogeneous composition

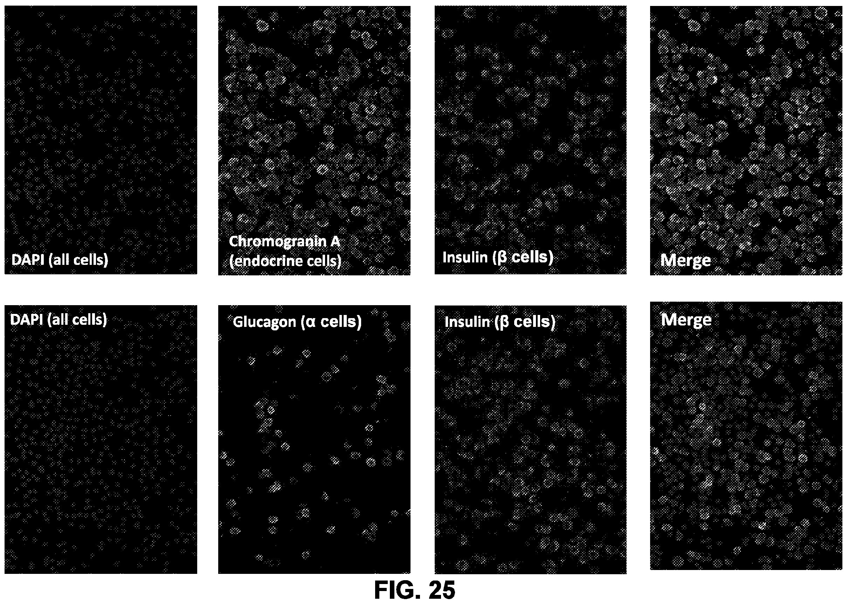

**FIG. 25** shows immunostaining graphs of cells expressing chromogranin A, insulin, or glucagon in a re-aggregated cell cluster, demonstrating the homogeneous composition in a reaggregated cell cluster.

### Biphasic insulin response

Dynamic insulin secretion in response to glucose challenges was also tested. As demonstrated in **FIG. 28****,** in response to a high glucose concentration 20 mM challenge, exemplary native cell cluster, reaggregated pancreatic cell cluster, both exhibited a biphasic insulin response pattern. The biphasic response pattern was characterized by the transient increase in insulin secretion to a peak value followed by a rapid decrease to a relatively elevated insulin secretion level (around 25 min to 35 min).This first phase response was followed by a second phase, in which the insulin secretion by the cell cluster was maintained at the relatively elevated level for an extended period (around 35 min to around 60 min).

In addition, **FIG. 28** also demonstrates the exemplary native cell cluster, reaggregated pancreatic cell cluster, both exhibited insulin secretion in response to 30 mM KCl challenge.

While preferred embodiments of the present disclosure have been shown and described herein, it will be obvious to those skilled in the art that such embodiments are provided by way of example only. It is not intended that the disclosure be limited by the specific examples provided within the specification. While the disclosure has been described with reference to the aforementioned specification, the descriptions and illustrations of the embodiments herein are not meant to be construed in a limiting sense. Numerous variations, changes, and substitutions will now occur to those skilled in the art without departing from the disclosure. Furthermore, it shall be understood that all aspects of the disclosure are not limited to the specific depictions, configurations or relative proportions set forth herein which depend upon a variety of conditions and variables. It should be understood that various alternatives to the embodiments of the disclosure described herein may be employed in practicing the disclosure. It is therefore contemplated that the disclosure shall also cover any such alternatives, modifications, variations or equivalents. It is intended that the following claims define the scope of the disclosure and that methods and structures within the scope of these claims and their equivalents be covered thereby.

### ASPECTS

### FURTHER ASPECTS OF THE INVENTION:

1. An *in vitro* cell cluster comprising at least one non-native pancreatic β cell that exhibits an *in vitro* glucose-stimulated insulin secretion response when exposed to a glucose challenge, wherein the cell cluster is an unsorted cell cluster, and wherein the cell cluster comprises:
   (a) at least about 35% cells that express NKX6.1 and C-peptide;
   (b) at least about 70% cells that express chromogranin A;
   (c) at most about 2% cells that express SOX2; or
   (d) at most about 10% cells that express SOX9.
2. An *in vitro* cell cluster comprising at least one NKX6.1⁺ and C-peptide⁺ cell that comprises no fluorescent protein, wherein the cell cluster exhibits an *in vitro* glucose-stimulated insulin secretion response when exposed to a glucose challenge, and wherein the cell cluster comprises:
   (a) at least about 35% cells that express NKX6.1 and C-peptide;
   (b) at least about 70% cells that express chromogranin A;
   (c) at most about 2% cells that express SOX2; or
   (d) at most about 10% cells that express SOX9.
3. The cell cluster of aspect 1, wherein the at least one NKX6.1⁺ and C-peptide⁺ cell is a non-native pancreatic β cell exhibiting an *in vitro* glucose-stimulated insulin secretion response when exposed to a glucose challenge.
4. The cell cluster of any one of aspects 1-3, comprising at least about 35% cells that express NKX6.1 and C-peptide.
5. The cell cluster of any one of aspects 1-4, comprising at least about 40% or at least about 60% cells that express both NKX6.1 and C-peptide.
6. The cell cluster of any one of aspects 1-5, comprising at least about 70% cells that express chromogranin A.
7. The cell cluster of any one of aspects 1-6, comprising at least about 80%, at least about 90%, at least about 95%, or 100% cells that express chromogranin A.
8. The cell cluster of any one of aspects 1-7, comprising at most about 2% cells in the cell cluster that express SOX2.
9. The cell cluster of any one of aspects 1-8, comprising at most about 1.5%, at most about 1%, or at most about 0.5% cells that express SOX2.
10. The cell cluster of any one of aspects 1-9, comprising at most about 10% cells that express SOX9.
11. The cell cluster of any one of aspects 1-10, comprising at most about 5%, at most about 3%, or at most about 2 % cells that express SOX9.
12. The cell cluster of any one of aspects 1-11, comprising at most about 2%, at most about 1%, at most about 0.5%, or at most about 0.1% cells that express Ki67.
13. The cell cluster of any one of aspects 1-12, wherein the *in vitro* cell cluster has a diameter that is about 150 µm.
14. The cell cluster of any one of aspects 1-12, wherein the *in vitro* cell cluster has a diameter that is from about 50 µm to about 250 µm.
15. The cell cluster of any one of aspects 1-14, wherein the *in vitro* cell cluster has a diameter that is from about 100 µm to about 200 µm.
16. The cell cluster of any one of aspects 1-15, wherein the *in vitro* cell cluster exhibits a higher insulin secretion in response to a first glucose concentration as compared to a second glucose concentration, wherein the first glucose concentration is higher than the second glucose concentration.
17. The cell cluster of any one of aspects 1-16, wherein the *in vitro* cell cluster further comprises at least one cell expressing glucagon or at least one cell expressing somatostatin.
18. The cell cluster of any one of aspects 1-17, wherein the *in vitro* cell cluster exhibits insulin secretion in response to a first concentration of K⁺.
19. The cell cluster of any one of aspects 1-18, wherein, when transplanted into a subject, the *in vitro* cell cluster exhibits an *in vivo* glucose-stimulated insulin secretion response to a glucose challenge in the subject within 28 days after transplantation.
20. The cell cluster of any one of aspects 1-18, wherein, when transplanted into a subject, the second *in vitro* cell cluster exhibits an *in vivo* glucose-stimulated insulin secretion response to a glucose challenge in the subject within 14 days after transplantation.
21. The cell cluster of any one of aspects 1 or 3-20, wherein:
   (a) the at least one non-native pancreatic β cell comprises one or more crystalline insulin granules;
   (b) the at least one non-native pancreatic β cell expresses the following genes: INS, PDX1, NKX6-1, and ZNT8; or
   (c) the at least one non-native pancreatic β cell does not express at least one of somatostatin and glucagon.
22. The cell cluster of any one of aspects 1 or 3-21, wherein the at least one non-native pancreatic β cell exhibits an *in vitro* glucose-stimulated insulin secretion response to a first glucose challenge, a second glucose challenge, and a third glucose challenge, when the first glucose challenge, the second glucose challenge, and the third glucose challenge are applied sequentially.
23. The cell cluster of any one of aspects 1 or 3-22, wherein secretion of insulin by the at least one non-native pancreatic β cell in response to a glucose challenge is proportional to glucose concentration in the glucose challenge.
24. The cell cluster of any one of aspects 1 or 3-23, wherein the at least one non-native pancreatic β cell is genetically modified.
25. The cell cluster of any one of aspects 1 or 3-24, wherein the at least one non-native pancreatic β cell does not comprise an exogenous nucleic acid sequence coding for a signal polypeptide indicative of insulin expression in the at least one non-native pancreatic β cell.
26. The cell cluster of aspect 25, wherein expression of the signal polypeptide in the non-native pancreatic β cell is driven by an insulin promoter.
27. The cell cluster of aspect 25, wherein the signal polypeptide comprises a fluorescent protein.
28. A composition comprising at least one cell cluster of any one of aspects 1-27 in a culture medium.
29. The composition of aspect 28, wherein the culture medium is serum-free.
30. The composition of aspect 28 or 29, wherein the reaggregation culture medium does not comprise triiodothyronine.
31. The composition of any one of aspects 28-30, wherein the reaggregation culture medium does not comprise an exogenous thyroid hormone signaling pathway activator.
32. The composition of any one of aspects 28-31, wherein the reaggregation culture medium does not comprise an activing receptor-like kinse-5 (Alk5) inhibitor.
33. The composition of any one of aspects 28-32, wherein the culture medium does not comprise an exogenous inhibitor of Rho-associated, coiled-coil containing protein kinase (ROCK).
34. The composition of any one of aspects 28-33, wherein the culture medium does not comprise an exogenous extracellular matrix molecule.
35. The composition of any one of aspects 28-34, wherein the cell cluster is in suspension in the culture medium.
36. The composition of any one of aspects 28-35, wherein the culture medium is stirred at a predetermined speed that is at least 30 rpm.
37. The composition of any one of aspects 28-36, wherein the culture medium is stirred at a predetermined speed that is at least 60 rpm.
38. The composition of any one of aspects 28-37, wherein the culture medium comprises MCDB131 basal medium.
39. The composition of aspect 38, wherein the MCDB131 basal medium is supplemented with 2% bovine serum albumin (BSA).
40. The composition of any one of aspects 28-39, wherein the culture medium comprises CMRLs medium.
41. The composition of any one of aspects 28-40, further comprising a bioreactor containing the at least one cell cluster and the culture medium.
42. The composition of aspect 41, wherein the bioreactor comprises a spinner flask.
43. A method, comprising:
   (a) differentiating a population of cells comprising at least one NKX6.1⁺ cell into a first cell cluster comprising at least one NKX6.1⁺ and C-peptide⁺ cell in a culture medium comprising a factor selected from the group consisting of: a transforming growth factor β (TGF-β) signaling pathway inhibitor, a thyroid hormone (TH) signaling pathway activator, at least one sonic-hedgehog (SHH) pathway inhibitor, a retinoic acid (RA) signaling pathway activator, a γ-secretase inhibitor, a bone morphogenic protein (BMP) signaling pathway inhibitor, at least one growth factor from epidermal growth factor (EGF) family, and any combination thereof;
   (b) dissociating a plurality of cells from the first cell cluster; and
   (c) culturing the plurality of cells from (b) in a reaggregation culture medium and allowing at least a portion of the plurality of cells to form a second *in vitro* cell cluster,
   wherein:
   (i) at least about 35% cells that express NKX6.1 and C-peptide;
   (ii) at least about 70% cells that express chromogranin A;
   (iii)at most about 2% cells that express SOX2; or
   (iv)at most about 10% cells that express SOX9.
44. A method, comprising:
   (a) obtaining a first cell cluster comprising at least one NKX6.1⁺ and C-peptide⁺ cell;
   (b) dissociating a plurality of cells from the first cell cluster; and
   (c) culturing the plurality of cells from (b) in a reaggregation culture medium and allowing at least a portion of the plurality of cells to form a second *in vitro* cell cluster,
   wherein:
   (i) at least about 35% cells that express NKX6.1 and C-peptide;
   (ii) at least about 70% cells that express chromogranin A;
   (iii)at most about 2% cells that express SOX2; or
   (iv)at most about 10% cells that express SOX9.
45. The method aspect 43, wherein the at least one growth factor from epidermal growth factor (EGF) family comprises betacellulin.
46. A method, comprising:
   (a) obtaining a first cell cluster comprising at least one NKX6.1⁺ and C-peptide⁺ cell;
   (b) dissociating a plurality of cells from the first cell cluster, wherein the dissociating does not comprise subjecting the plurality of cells to flow cytometry; and
   (c) culturing the plurality of cells from (b) in a serum-free reaggregation culture medium and allowing at least a portion of the plurality of cells to form a second *in vitro* cell cluster,
   wherein:
   (i) the second *in vitro* cell cluster comprises a higher percentage cells that express chromogranin A as compared the first cell cluster;
   (ii) the second *in vitro* cell cluster comprises a higher percentage cells that express NKX6.1 and C-peptide as compared the first cell cluster;
   (iii) the second *in vitro* cell cluster comprises a lower percentage cells that express SOX2 as compared the first cell cluster; or
   (iv) the second *in vitro* cell cluster comprises a lower percentage of cells that express SOX9 as compared the first cell cluster.
47. A method, comprising:
   (a) obtaining a first cell cluster comprising at least one NKX6.1⁺ and C-peptide⁺ cell;
   (b) dissociating a plurality of cells from the first cell cluster, wherein the dissociating does not comprise subjecting the plurality of cells to flow cytometry; and
   (c) culturing the plurality of cells from (b) in a reaggregation culture medium and allowing at least a portion of the plurality of cells to form a second *in vitro* cell cluster,

   wherein the second *in vitro* cell cluster comprises at least one non-native pancreatic β cell that exhibits an *in vitro* glucose-stimulated insulin secretion response when exposed to a glucose challenge; and
   wherein:
      (i) at least about 35% cells that express NKX6.1 and C-peptide;
      (ii) at least about 70% cells that express chromogranin A;
      (iii)at most about 2% cells that express SOX2; or
      (iv)at most about 10% cells that express SOX9.
48. The method of any one of aspects 43-46, wherein the second *in vitro* cell cluster comprises at least one non-native pancreatic β cell that exhibits an *in vitro* glucose-stimulated insulin secretion response when exposed to a glucose challenge.
49. The method of any one of aspects 43-48, wherein the dissociating comprises enzymatic dissociation of the first cell cluster.
50. The method of any one of aspects 43-49, wherein the dissociating comprises contacting the first cell cluster with trypsin.
51. The method of any one of aspects 43-45 or 47-50, wherein the reaggregation culture medium is serum-free.
52. The method of any one of aspects 43-51, wherein the reaggregation culture medium is stirred at a predetermined speed.
53. The method of aspect 52, wherein the predetermined speed is that is at least 30 rpm or at least 60 rpm.
54. The method of any one of aspects 43-53, wherein the reaggregation culture medium comprises MCDB131 basal medium.
55. The method of aspect 54, wherein the MCDB131 basal medium is supplemented with 2% bovine serum albumin (BSA).
56. The method of any one of aspects 43-53, wherein the reaggregation culture medium comprises CMRLs medium.
57. The method of any one of aspects 43-56, wherein the reaggregation culture medium comprises MCDB131 basal medium.
58. The method of any one of aspects 47-57, wherein:
   (a) the at least one non-native pancreatic β cell comprises one or more crystalline insulin granules;
   (b) the at least one non-native pancreatic β cell expresses the following genes: INS, PDX1, NKX6-1, and ZNT8; or
   (c) the at least one non-native pancreatic β cell does not express at least one of somatostatin and glucagon.
59. The method of any one of aspects 47-58, wherein the at least one non-native pancreatic β cell exhibits an *in vitro* glucose-stimulated insulin secretion response to a first glucose challenge, a second glucose challenge, and a third glucose challenge, when the first glucose challenge, the second glucose challenge, and the third glucose challenge are applied sequentially.
60. The method of any one of aspects 47-59, wherein the at least one non-native pancreatic β cell is genetically modified.
61. The method of any one of aspects 47-60, wherein secretion of insulin by the at least one non-native pancreatic β cell in response to a glucose challenge is proportional to glucose concentration in the glucose challenge.
62. The method of any one of aspects 43-61, wherein the reaggregation culture medium does not comprise triiodothyronine.
63. The method of any one of aspects 43-62, wherein the reaggregation culture medium does not comprise an exogenous thyroid hormone signaling pathway activator.
64. The method of any one of aspects 43-63, wherein the reaggregation culture medium does not comprise an activing receptor-like kinse-5 (Alk5) inhibitor.
65. The method of any one of aspects 43-64, wherein the reaggregation culture medium does not comprise an exogenous transforming growth factor β signaling pathway inhibitor.
66. The method of any one of aspects 43-65, wherein the reaggregation culture medium does not comprise an exogenous inhibitor of Rho-associated, coiled-coil containing protein kinase (ROCK).
67. The method of any one of aspects 43-66, wherein the reaggregation culture medium does not comprise an exogenous extracellular matrix molecule.
68. The method of any one of aspects 43-67, wherein the first cell cluster is recovered from cryopreservation.
69. The method of any one of aspects 43-68, further comprising recovering the first cell cluster from cryopreservation.
70. The method of any one of aspects 43-69, further comprising cryopreserving the plurality of cells for a period of time before the culturing.
71. The method of aspect 70, further comprising recovering the plurality of cells from the cryopreservation before the culturing.
72. The method of any one of aspects 43-71, wherein the second *in vitro* cell cluster has a diameter that is at most 50% of the first cell cluster.
73. The method of any one of aspects 43-72, wherein the second *in vitro* cell cluster has a diameter that is at most 30% of the first cell cluster.
74. The method of any one of aspects 43-73, wherein the second *in vitro* cell cluster has a diameter that is about 150 µm.
75. The method of any one of aspects 43-73, wherein the second *in vitro* cell cluster has a diameter that is from about 50 µm to about 250 µm.
76. The method of any one of aspects 43-75, wherein the second *in vitro* cell cluster has a diameter that is from about 100 µm to about 200 µm.
77. The method of any one of aspects 43-76, wherein a percentage of cells in the second *in vitro* cell cluster that express chromogranin A is at least 1.2, at least 1.3, at least 1.4, or at least 1.5 times more than a percentage of cells in the first cell cluster that express chromogranin A.
78. The method of any one of aspects 43-77, wherein the second *in vitro* cell cluster comprises at least about 70%, at least about 80%, at least about 90%, at least about 95%, or 100% cells that express chromogranin A.
79. The method of any one of aspects 43-78, wherein a percentage of cells in the second *in vitro* cell cluster that express both NKX6.1 and C-peptide is at least 1.5, at least 1.75, or at least 2 times more than a percentage of cells in the first cell cluster that express both NKX6.1 and C-peptide.
80. The method of any one of aspects 43-79, wherein the second *in vitro* cell cluster comprises at least about 35%, at least about 40%, or at least about 60% cells that express both NKX6.1 and C-peptide.
81. The method of any one of aspects 43-80, wherein a percentage of cells in the second *in vitro* cell cluster that express SOX2 is at least 2, at least 3, at least 5, or at least 10 times lower than a percentage of cells in the first cell cluster that express SOX2.
82. The method of any one of aspects 43-81, wherein the second *in vitro* cell cluster comprises at most about 2%, at most about 1.5%, at most about 1%, or at most about 0.5% cells that express SOX2.
83. The method of any one of aspects 43-82, wherein a percentage of cells in the second *in vitro* cell cluster that express SOX9 is at least 2, at least 3, at least 5, or at least 10 times lower than a percentage of cells in the first cell cluster that express SOX9.
84. The method of any one of aspects 43-83, wherein the second *in vitro* cell cluster comprises at most about 10%, at most about 5%, at most about 3%, or at most about 2 % cells that express SOX9.
85. The method of any one of aspects 43-84, wherein the second *in vitro* cell cluster has a lower percentage cells expressing Ki67 as compared to the first cell cluster.
86. The method of any one of aspects 43-85, wherein the second *in vitro* cell cluster comprises at most about 2%, at most about 1%, at most about 0.5%, or at most about 0.1% cells that express Ki67.
87. The method of any one of aspects 43-86, wherein the second *in vitro* cell cluster has a higher insulin content as compared to the first cell cluster.
88. The method of any one of aspects 43-87, wherein the second *in vitro* cell cluster has at least 1.1, at least 1.25 or at least 1.5 times higher insulin content as compared to the first cell cluster.
89. The method of any one of aspects 43-88, wherein the second *in vitro* cell cluster exhibits a higher stimulation index compared to the first cell cluster, wherein the stimulation index equals to a ratio of insulin secreted in response to a first glucose concentration as compared to a second glucose concentration, wherein the first glucose concentration is higher than the second glucose concentration.
90. The method of any one of aspects 43-89, wherein the second *in vitro* cell cluster exhibits insulin secretion in response to a first concentration of K⁺.
91. The method of any one of aspects 43-90, wherein the second *in vitro* cell cluster exhibits biphasic insulin secretion in response to a high glucose concentration that is larger than 10 mM.
92. The method of any one of aspects 43-91, wherein the second *in vitro* cell cluster further comprises at least one cell expressing glucagon or at least one cell expressing somatostatin.
93. The method of any one of aspects 43-92, wherein at least about 95%, at least about 98%, or at least about 99% of cells that express both NKX6.1 and C-peptide in the first cell cluster are retained in the second *in vitro* cell cluster.
94. The method of any one of aspects 43-93, wherein the second *in vitro* cell cluster has a higher oxygen consumption rate as compared to the first cell cluster.
95. The method of any one of aspects 43-94, wherein the second *in vitro* cell cluster has at least 1.1, at least 1.25 or at least 1.5 times higher oxygen consumption rate as compared to the first cell cluster.
96. The method of any one of aspects 43-95, wherein, when transplanted into a subject, the second *in vitro* cell cluster exhibits an *in vivo* glucose-stimulated insulin secretion response to a glucose challenge in the subject within 28 days after transplantation.
97. The method of any one of aspects 43-96, wherein, when transplanted into a subject, the second *in vitro* cell cluster exhibits an *in vivo* glucose-stimulated insulin secretion response to a glucose challenge in the subject within 14 days after transplantation.
98. An *in vitro* cell cluster that is generated by the method of any one of aspects 43-97.
99. A device comprising the *in vitro* cell cluster of any one of aspects 1-27 or 98, wherein the device is configured to produce and release insulin when implanted into a subject.
100. The device of aspect 99, further comprising a semipermeable membrane, wherein the semipermeable membrane is configured to retain the cell cluster in the device and permit passage of insulin secreted by the cell cluster.
101. The device of aspect 100, wherein the cell cluster is encapsulated by the semipermeable membrane.
102. The device of aspect 100 or 101, wherein the semipermeable membrane is made of polysaccharide or polycation.
103. The device of any one of aspects 100-102, wherein the semipermeable membrane is made of a material selected from the group consisting of: poly(lactide) (PLA), poly(glycolic acid) (PGA), poly(lactide-co-glycolide) (PLGA), other polyhydroxyacids, poly(caprolactone), polycarbonates, polyamides, polyanhydrides, polyphosphazene, polyamino acids, polyortho esters, polyacetals, polycyanoacrylates, polytetrafluoroethylene (PTFE), biodegradable polyurethanes, albumin, collagen, fibrin, polyamino acids, prolamines, alginate, agarose, agarose with gelatin, dextran, polyacrylates, ethylene- vinyl acetate polymers and other acyl-substituted cellulose acetates and derivatives thereof, polyurethanes, polystyrenes, polyvinyl chloride, polyvinyl fluoride, poly(vinyl imidazole), chlorosulphonated polyolefins, polyethylene oxide, and any combinations thereof.
104. The device of any one of aspects 100-103, wherein the semipermeable membrane comprises alginate.
105. The device of any one of aspects 100-104, wherein the cell cluster is encapsulated in a microcapsule that comprises an alginate core surrounded by the semipermeable membrane.
106. A method of treating or preventing a disease in a subject in need thereof, the method comprising administering the cell cluster of any one of aspects any one of aspects 1-27 or 98 or a portion thereof to the subject.
107. A method of treating or preventing a disease in a subject in need thereof, the method comprising implanting the device of any one of aspects 99-105 to the subj ect.
108. The method of aspect 106 or 107, wherein the subject has, or has an increased risk of developing a metabolic disorder.
109. The method of any one of aspects 106-108, wherein the subject has diabetes selected from the group consisting of: Type I diabetes, Type II diabetes, and Type 1.5 diabetes.
110. A cell cluster comprising at least one non-native pancreatic β cell, wherein at least about 70% of cells in the cell cluster express chromogranin A as measured by flow cytometry, and wherein the cell cluster exhibits an *in vitro* glucose-stimulated insulin secretion response when exposed to a glucose challenge.
111. A cell cluster comprising at least one non-native pancreatic β cell, wherein at least about 25% of cells in the cell cluster express NKX6.1 and C-peptide as measured by flow cytometry, and wherein the cell cluster exhibits an *in vitro* glucose-stimulated insulin secretion response when exposed to a glucose challenge.
112. A cell cluster comprising at least one non-native pancreatic β cell, wherein at least about 70% of cells in the cell cluster express chromogranin A as measured by flow cytometry, and wherein, when transplanted into a subject, the cell cluster exhibits an *in vivo* glucose-stimulated insulin secretion response to a glucose challenge in the subject within 28 days after the transplantation.
113. A cell cluster comprising at least one non-native pancreatic β cell, wherein at least about 25% of cells in the cell cluster express NKX6.1 and C-peptide as measured by flow cytometry, and, wherein, when transplanted into a subject, the cell cluster exhibits an *in vivo* glucose-stimulated insulin secretion response to a glucose challenge in the subject within about 28 days after the transplantation.
114. The cell cluster of aspect 110 or 112, wherein at least about 90% of cells in the cell cluster express chromogranin A as measured by flow cytometry.
115. The cell cluster of aspect 111 or 113, wherein at least about 70% of cells in the cell cluster express NKX6.1 and C-peptide as measured by flow cytometry.
116. The cell cluster of aspect 110 or 112, wherein at least about 25% of cells in the cell cluster express NKX6.1 and C-peptide as measured by flow cytometry.
117. The cell cluster of aspect 111 or 113, wherein the cell cluster exhibits an *in vitro* glucose-stimulated insulin secretion response to a first glucose challenge and a second glucose challenge when the first and second glucose challenges are applied sequentially.
118. The cell cluster of aspect 112 or 113, wherein, when transplanted into the subject, the cell cluster exhibits an *in vivo* glucose-stimulated insulin secretion response to a glucose challenge in the subject within 14 days after the transplantation.
119. The cell cluster as in one of aspects 110-118, wherein the cell cluster has a diameter less than about 300 µm.
120. The cell cluster of aspect 119, wherein the diameter is less than about 100 µm.
121. The cell cluster as in one of aspects 110-120, wherein less than about 10% of cells in the cell cluster are not viable.
122. The cell cluster as in one of aspects 110-121, wherein the cell cluster exhibits a glucose-stimulated calcium flux response when exposed to a glucose challenge.
123. A cell cluster comprising at least one non-native pancreatic β cell, wherein at least about 70% of cells in the cell cluster express chromogranin A, and at least about 25% of cells in the cell cluster express NKX6.1 and C-peptide, as measured by flow cytometry, and wherein the cell cluster exhibits an *in vitro* glucose-stimulated insulin secretion response when exposed to a glucose challenge, and wherein, when transplanted into a subject, the cell cluster exhibits an *in vivo* glucose-stimulated insulin secretion response to a glucose challenge in the subject within about 28 days after the transplantation.
124. A composition comprising the cell cluster as in on of aspects 110-118 and a scaffold.
125. The composition of aspect 124, wherein the scaffold is a biodegradable scaffold.
126. A method comprising:
   (a) obtaining a first cell cluster comprising at least one non-native pancreatic β cell;
   (b) dissociating a plurality of cells from the first cell cluster;
   (c) culturing the plurality of cells from (b) in a medium comprising a thyroid hormone signaling pathway activator and a transforming growth factor β signaling pathway inhibitor, thereby forming a second cell cluster *in vitro* using at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry.
127. A method comprising:
   (a) obtaining a first cell cluster comprising at least one non-native pancreatic β cell;
   (b) dissociating a plurality of cells from the first cell cluster;
   (c) culturing the plurality of cells from (a) in a medium comprising
      (i)serum; and
      (ii)one of a thyroid hormone signaling pathway activator and a transforming growth factor β signaling pathway inhibitor,
   thereby forming a second cell cluster *in vitro* using at least a portion of the plurality of cells, wherein the second cell cluster comprises a non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry.
128. The method of aspect 127, wherein the medium further comprises a thyroid hormone signaling pathway activator.
129. The method of aspect 128, wherein the medium further comprises a transforming growth factor β signaling pathway inhibitor.
130. The method according to aspects 126 or 128, wherein the thyroid hormone signaling pathway activator is triiodothyronine.
131. The method of aspect 130, wherein a concentration of the triiodothyronine in the medium is from about 0.1 µM to about 10 µM.
132. The method according to aspects 126 or 129, wherein the transforming growth factor β signaling pathway inhibitor is an activin receptor-like kinase-5 inhibitor.
133. The method of aspect 132, wherein a concentration of the activin receptor-like kinase-5 inhibitor in the medium is from about 1 µM to about 50 µM.
134. The method according to aspect 126, wherein the medium further comprises a serum selected from the group consisting of human serum, human platelet lysate, fetal bovine serum, and serum replacement.
135. The method according to aspect 127 or 134, wherein the medium comprises from about 5% to about 15% serum.
136. The method of aspect 135, wherein the medium comprises about 10% serum.
137. The method according to aspect 126 or 127, wherein the medium further comprises Connought Medical Research Laboratories 1066 supplemented islet media (CMRLS) or MCDB131 basal medium.
138. The method according to aspect 126 or 127, wherein the medium further comprises an extracellular matrix molecule.
139. The method of aspect 138, wherein the extracellular matrix molecule is heparin.
140. The method of aspect 138, wherein the extracellular matrix molecule is laminin.
141. The method according to aspect 126 or 127, wherein the second cell cluster comprises a higher percentage of cells expressing chromogranin A as compared to the first cell cluster as measured by flow cytometry.
142. The method according to aspect 126 or 127, wherein the second cell cluster comprises a higher percentage of cells expressing NKX6.1 and C-peptide compared to the first cell cluster as measured by flow cytometry.
143. The method according to aspect 126 or 127, wherein the second cell cluster comprises less inviable cells compared to the first cell cluster.
144. The method according to aspect 126 or 127, wherein a portion of the plurality of cells fail to form the second cell cluster, and wherein less than about 10% of the portion of the plurality of cells that fail to form the second cell cluster express NKX6.1 and C-peptide as measured by flow cytometry.
145. The method according to aspect 126 or 127, wherein a diameter of the second cell cluster is less than a diameter of the first cell cluster.
146. The method according to aspect 126 or 127, wherein, when exposed to a glucose challenge *in vitro,* the second cell cluster exhibits a higher stimulation index compared to the first cell cluster, wherein the stimulation index equals a ratio of insulin secreted in response to a first glucose concentration as compared to a second glucose concentration, wherein the first glucose concentration is higher than the second glucose concentration.
147. The method according to aspect 126 or 127, wherein, when exposed to a glucose challenge *in vivo,* the second cell cluster exhibits a higher stimulation index compared to the first cell cluster, wherein the stimulation index equals a ratio of insulin secreted in response to a first glucose concentration compared to a second glucose concentration, wherein the first glucose concentration is higher than the second glucose concentration.
148. The method according to aspect 126 or 127, wherein the plurality of cells from (b) are cultured in a spinner flask.
149. The method according to aspect 126 or 127, wherein the plurality of cells from (b) are generated from one or more stem cells *in vitro.*
150. The method of aspect 149, wherein the plurality of cells from (b) are generated from one or more induced pluripotent stem cells.
151. The method according to aspect 126 or 127, wherein the plurality of cells from (b) are cultured in the medium for at least 1 day.
152. The method of aspect 151, wherein the plurality of cells are cultured in the medium for at least 4 days.
153. A method for treating or preventing a disease in a subject in need thereof, the method comprising administering a composition comprising the cell cluster in any one of aspects 1, 2, 3, 4 or a portion thereof to the subject.
154. The method of aspect 153, wherein the subject is a human.
155. The method according to aspects 153 or 154, wherein the subject has, or has an increased risk of developing, diabetes.
156. The method of aspect 155, wherein the diabetes is Type I diabetes, Type II diabetes, Type 1.5 diabetes, or pre-diabetes.
157. The method of aspect 153, wherein the administering comprises administering the composition under a kidney capsule, in a liver, or in a pancreas.
158. The method according to aspects 153 or 154, further comprising cryopreserving the second cell cluster, thereby producing a cryopreserved second cell cluster.
159. The method according to aspect 158, further comprising thawing the cryopreserved second cell cluster, thereby producing a thawed second cell cluster comprising at least one non-native pancreatic β cell.
160. The method according to aspect 159, wherein (i) at least about 70% of cells in the thawed second cell cluster express chromogranin A as measured by flow cytometry, and/or (ii) at least about 25% of cells in the thawed second cell cluster express NKX6.1 and C-peptide as measured by flow cytometry.
161. A system for use in a method as in any of aspects 126-160, the system comprising a magnetic stir plate, a spinner flask comprising a plurality of cells from the first cell cluster, wherein the magnetic stir plate and the spinner flask are configured to stir the plurality of cells from the first cluster thereby forming the second cell cluster *in vitro* using at least a portion of the plurality of cells.
162. A method for treating or preventing a disease in a subject in need thereof, the method comprising:
   (a)obtaining a first cell cluster comprising at least one non-native pancreatic β cell;
   (b)dissociating a plurality of cells from the first cell cluster;
   (c)culturing the plurality of cells from (a) in a medium comprising a thyroid hormone signaling pathway activator and a transforming growth factor β signaling pathway inhibitor, thereby forming a second cell cluster *in vitro* using at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 25% of cells in the second cell cluster express NKX6.1 and C-peptide as measured by flow cytometry; and
   (d)administering a composition comprising the second cell cluster or a portion thereof to the subject.
163. A method for treating or preventing a disease in a subject in need thereof, the method comprising:
   (a) obtaining a first cell cluster comprising at least one non-native pancreatic β cell;
   (b)dissociating a plurality of cells from the first cell cluster;
   (c) culturing the plurality of cells from (b) in a medium comprising
      (i)serum; and
      (ii)a thyroid hormone signaling pathway activator or a transforming growth factor β signaling pathway inhibitor,
      thereby forming a second cell cluster *in vitro* from at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 25% of cells in the second cell cluster express NKX6.1 and C-peptide as measured by flow cytometry; and
   (d)administering a composition comprising the second cell cluster or a portion thereof to the subject.
164. A pharmaceutical composition comprising the cell cluster in any one of previous cell cluster aspect or a portion thereof and at least one pharmaceutically acceptable excipient.
165. The pharmaceutical composition of aspect 164, wherein the pharmaceutical composition is a liquid composition.
166. The pharmaceutical composition of aspect 165, wherein the pharmaceutical composition is a capsule.
167. The pharmaceutical composition of aspect 166, wherein the capsule is a gel capsule.
168. The pharmaceutical composition of aspect 166, wherein the capsule is a liposome.
169. A cell cluster comprising at least one non-native pancreatic β cell, wherein the cell cluster is prepared by a process comprising:
   (a) obtaining a first cell cluster comprising at least one non-native pancreatic β cell;
   (b)dissociating a plurality of cells from the first cell cluster ;
   (c) culturing the plurality of cells from (b) in a medium comprising a thyroid hormone signaling pathway activator and a transforming growth factor β signaling pathway inhibitor, thereby forming a second cell cluster *in vitro* from at least a portion of the plurality of cells, wherein the second cell cluster comprises at least one non-native pancreatic β cell, and wherein (i) at least about 70% of cells in the second cell cluster express chromogranin A as measured by flow cytometry, and/or (ii) at least about 40% of cells in the second cell cluster express NKX6.1 and C-peptide as measured by flow cytometry.
170. A kit comprising (i) a cell cluster comprising at least one non-native pancreatic β cell, wherein at least about 70% of cells in the cell cluster express chromogranin A as measured by flow cytometry, and wherein the cell cluster exhibits an *in vitro* glucose-stimulated insulin secretion response when exposed to a glucose challenge, and (ii) a buffer.
171. A kit comprising (i) a cell cluster comprising at least one non-native pancreatic β cell, wherein at least about 25% of cells in the cell cluster express NKX6.1 and C-peptide as measured by flow cytometry, and wherein the cell cluster exhibits an *in vitro* glucose-stimulated insulin secretion response when exposed to a glucose challenge, and (ii) a buffer.

## Claims

1. An *in vitro* cell cluster comprising at least one non-native pancreatic β cell that exhibits an *in vitro* glucose-stimulated insulin secretion response when exposed to a glucose challenge, wherein the cell cluster is an unsorted cell cluster, and wherein the cell cluster comprises:
(a) at least about 35% cells that express NKX6.1 and C-peptide;
(b) at least about 95% cells that express chromogranin A; and
(c) at most about 2% cells that express SOX9.

2. An *in vitro* cell cluster comprising at least one NKX6.1⁺ and C-peptide⁺ cell that comprises no fluorescent protein, wherein the cell cluster exhibits an *in vitro* glucose-stimulated insulin secretion response when exposed to a glucose challenge, and wherein the cell cluster comprises:
(a) at least about 35% cells that express NKX6.1 and C-peptide;
(b) at least about 95% cells that express chromogranin A; and
(c) at most about 2% cells that express SOX9.

3. The cell cluster of claim 1 or 2, comprising at least about 35% cells that express NKX6.1 and C-peptide.

4. The cell cluster of any one of claims 1-3, comprising at least about 40% or at least about 60% cells that express both NKX6.1 and C-peptide.

5. The cell cluster of any one of claims 1-4, comprising at most about 2% cells in the cell cluster that express SOX2.

6. The cell cluster of any one of claims 1-5, comprising at most about 1% cells in the cell cluster that express SOX2.

7. The cell cluster of any one of claims 1-6, comprising at most about 2%, at most about 1%, at most about 0.5%, or at most about 0.1% cells that express Ki67.

8. The cell cluster of any one of claims 1-7, wherein the *in vitro* cell cluster has a diameter that is from about 50 µm to about 250 µm.

9. The cell cluster of any one of claims 1-8, wherein the *in vitro* cell cluster has a diameter that is from about 100 µm to about 200 µm.

10. The cell cluster of any one of claims 1-9, wherein the *in vitro* cell cluster further comprises at least one cell expressing glucagon or at least one cell expressing somatostatin.

11. The cell cluster of any one of claims 1-10, wherein, when transplanted into a subject, the *in vitro* cell cluster exhibits an *in vivo* glucose-stimulated insulin secretion response to a glucose challenge in the subject within 14 days after transplantation.

12. The cell cluster of any one of claims 1 or 3-11, wherein the at least one non-native pancreatic β cell is genetically modified.

13. A composition comprising at least one cell cluster of any one of claims 1-12 in a serum-free culture medium, wherein the cell cluster is in suspension in the culture medium.

14. The composition of claim 13, wherein the culture medium does not comprise an exogenous extracellular matrix molecule.

15. The composition of claim 13 or 14, further comprising a bioreactor containing the at least one cell cluster and the culture medium.
